# EUROPEAN PATENT SPECIFICATION

(11) **EP 3 618 579 B1**
(45) Date of publication and mention of the grant of the patent: **15.03.2023**
(21) Application number: 18791887.5
(22) Date of filing: 20.04.2018
(51) Int. Cl.: C09K 11/06, H01L 51/50, C07D 209/86, C07D 333/76, C07D 409/14, C07F 15/00, H01L 51/00

(54) **COMPOSITION AND LIGHT-EMITTING ELEMENT IN WHICH SAME IS USED**
ZUSAMMENSETZUNG UND LICHTEMITTIERENDES ELEMENT DAMIT
COMPOSITION ET ÉLÉMENT ÉLECTROLUMINESCENT FAISANT APPEL À CETTE DERNIÈRE

(30) Priority: 27.04.2017 JP 2017088005
(43) Date of publication of application: 04.03.2020
(73) Proprietor: Sumitomo Chemical Company Limited, Tokyo 104-8260 (JP)
(72) Inventor: ISHIKAWA, Rui, Tsukuba-shi Ibaraki 300-3294 (JP); TARRAN, William, Godmanchester Cambridgeshire PE29 2XG (GB); KAMTEKAR, Timothy, Kiran, Godmanchester Cambridgeshire PE29 2XG (GB)
(74) Representative: J A Kemp LLP
(86) International application number: PCT/JP2018/016306
(87) International publication number: WO 2018/198971

(56) References cited:
- WO-A1-2016/185183
- CN-A- 104 250 242
- CN-A- 105 524 114
- CN-A- 105 524 114
- JP-A- 2012 503 889
- JP-A- 2012 516 831
- JP-A- 2016 540 381
- US-A1- 2011 284 799
- US-A1- 2016 087 224
- US-A1- 2016 111 665
- US-A1- 2016 190 500
- US-A1- 2016 285 003

## Description

### Technical Field

The present invention relates to a composition and a light emitting device using the same.

### Background Art

Light emitting devices such as organic electroluminescent devices and the like can be suitably used for display and lighting applications. As a light emitting material used for a light emitting layer of a light emitting device, for example, Patent Document 1 suggests a composition containing a compound (H0-1) and FIrpic which is a phosphorescent compound. Patent Document 2 suggests a composition containing a compound (HO-2) and a phosphorescent compound (B0). Patent Document 3 describes a multilayer structure comprising at least two adjacent layers L1 and L2 wherein both layers L1 and L2 comprise at least 50 wt % of a spirobifluorene derivative or an open spirobifluorene derivative. Patent Document 4 describes a series of deep blue metal iridium phosphorescence OLED materials.

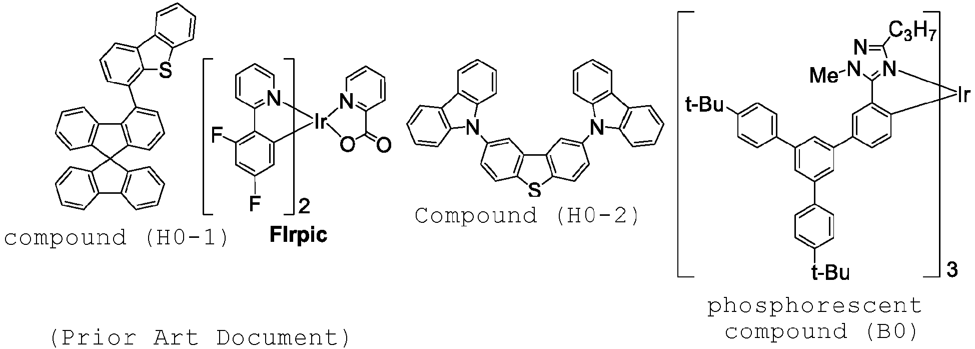

### (Patent Document)

(Patent Document 1)
   Chinese Patent Application Publication No. 102911145
(Patent Document 2)
   International Publication WO 2015/156235
(Patent Document 3)
   US Patent Application Publication No. 2016/285003
(Patent Document 4)
   Chinese Patent Application Publication No. 105524114

### Summary of the Invention

### Problem to be Solved by the Invention

However, the light emitting device produced using the above-described composition was not necessarily sufficient in light emission efficiency. Then, an object of the present invention is to provide a composition which is useful for production of a light emitting device excellent in light emission efficiency.

### Means for Solving the Problem

The present invention provides a composition as defined in claim 1. Some preferred embodiments of the composition of the present invention are defined in claims 2 to 8. The present invention also provides a light emitting device as defined in claim 9.

### Effect of the Invention

According to the present invention, a composition which is useful for production of a light emitting device excellent in light emission efficiency can be provided. Further, according to the present invention, a light emitting device comprising the composition can be provided.

### Modes for Carrying Out the Invention

Suitable embodiments of the present invention will be illustrated in detail below.

### <Explanation of common terms>

Terms commonly used in the present specification have the following meanings unless otherwise stated.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

In the formula representing a metal complex, the solid line representing the bond to the central metal means a covalent bond or a coordinate bond.

"The polymer compound" means a polymer having molecular weight distribution and having a polystyrene-equivalent number-average molecular weight of 1×10³ to 1×10³.

"The low molecular compound" means a compound having no molecular weight distribution and having a molecular weight of 1×10⁴ or less.

"The constitutional unit" means a unit which is present one or more times in the polymer compound.

"The alkyl group" may be any of linear or branched. The number of carbon atoms of the linear alkyl group is, not including the number of carbon atoms of the substituent, usually 1 to 50, preferably 3 to 30, more preferably 4 to 20. The number of carbon atoms of the branched alkyl group is, not including the number of carbon atoms of the substituent, usually 3 to 50, preferably 3 to 30, more preferably 4 to 20.

The alkyl group optionally has a substituent, and examples thereof include a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group, and these groups in which its hydrogen atom is substituted with a cycloalkyl group, an alkoxy group, a cycloalkoxy group, an aryl group, a fluorine atom or the like (for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group and a 6-ethyloxyhexyl group).

The number of carbon atoms of the "cycloalkyl group" is, not including the number of carbon atoms of the substituent, usually 3 to 50, preferably 3 to 30, more preferably 4 to 20.

The cycloalkyl group optionally has a substituent, and examples thereof include a cyclohexyl group, a cyclohexylmethyl group and a cyclohexylethyl group.

"The aryl group" means an atomic group remaining after removing from an aromatic hydrocarbon one hydrogen atom directly bonding to a carbon atom constituting the ring. The number of carbon atoms of the aryl group is, not including the number of carbon atoms of the substituent, usually 6 to 60, preferably 6 to 20, more preferably 6 to 10.

The aryl group optionally has a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group and a 4-phenylphenyl group, and these groups in which its hydrogen atom is substituted with an alkyl group, a cycloalkyl group, an alkoxy group, a cycloalkoxy group, an aryl group, a fluorine atom or the like.

"The alkoxy group" may be any of linear or branched. The number of carbon atoms of the linear alkoxy group is, not including the number of carbon atoms of the substituent, usually 1 to 40, preferably 4 to 10. The number of carbon atoms of the branched alkoxy group is, not including the number of carbon atoms of the substituent, usually 3 to 40, preferably 4 to 10.

The alkoxy group optionally has a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group and a lauryloxy group, and these groups in which its hydrogen atom is substituted with a cycloalkyl group, an alkoxy group, a cycloalkoxy group, an aryl group, a fluorine atom or the like.

The number of carbon atoms of the "cycloalkoxy group" is, not including the number of carbon atoms of the substituent, usually 3 to 40, preferably 4 to 10.

The cycloalkoxy group optionally has a substituent, and examples thereof include a cyclohexyloxy group.

The number of carbon atoms of the "aryloxy group" is, not including the number of carbon atoms of the substituent, usually 6 to 60, preferably 6 to 48.

The aryloxy group optionally has a substituent, and examples thereof include a phenoxy group, a 1-naphthyloxy group, a 2-naphthyloxy group, a 1-anthracenyloxy group, a 9-anthracenyloxy group and a 1-pyrenyloxy group, and these groups in which its hydrogen atom is substituted with an alkyl group, a cycloalkyl group, an alkoxy group, a cycloalkoxy group, a fluorine atom or the like.

"The p-valent heterocyclic group" (p represents an integer of 1 or more.) means an atomic group remaining after removing from a heterocyclic compound p hydrogen atoms among hydrogen atoms directly bonding to carbon atoms or hetero atoms constituting the ring. Of the p-valent heterocyclic groups, preferable are "p-valent aromatic heterocyclic groups" which are atomic groups remaining after removing from an aromatic heterocyclic compound p hydrogen atoms among hydrogen atoms directly bonding to carbon atoms or hetero atoms constituting the ring.

"The aromatic heterocyclic compound" means a compound in which the hetero ring itself shows aromaticity such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzophosphole and the like and a compound in which an aromatic ring is condensed to the hetero ring even if the hetero ring itself shows no aromaticity such as phenoxazine, phenothiazine, dibenzoborole, dibenzosilole, benzopyran and the like.

The number of carbon atoms of the monovalent heterocyclic group is, not including the number of carbon atoms of the substituent, usually 2 to 60, preferably 4 to 20.

The monovalent heterocyclic group optionally has a substituent, and examples thereof include a thienyl group, a pyrrolyl group, a furyl group, a pyridyl group, a piperidinyl group, a quinolinyl group, an isoquinolinyl group, a pyrimidinyl group and a triazinyl group, and these groups in which its hydrogen atom is substituted with an alkyl group, a cycloalkyl group, an alkoxy group, a cycloalkoxy group or the like.

"The halogen atom" denotes a fluorine atom, a chlorine atom, a bromine atom or an iodine atom.

"The amino group" optionally has a substituent, and a substituted amino group is preferable. The substituent which the amino group has is preferably an alkyl group, a cycloalkyl group, an aryl group or a monovalent heterocyclic group.

The substituted amino group includes, for example, a dialkylamino group, a dicycloalkylamino group and a diarylamino group.

The amino group includes, for example, a dimethylamino group, a diethylamino group, a diphenylamino group, a bis(4-methylphenyl)amino group, a bis(4-tert-butylphenyl)amino group and a bis(3,5-di-tert-butylphenyl)amino group.

"The alkenyl group" may be any of linear or branched. The number of carbon atoms of the linear alkenyl group is, not including the number of carbon atoms of the substituent, usually 2 to 30, preferably 3 to 20. The number of carbon atoms of the branched alkenyl group is, not including the number of carbon atoms of the substituent, usually 3 to 30, preferably 4 to 20.

The number of carbon atoms of "the cycloalkenyl group" is, not including the number of carbon atoms of the substituent, usually 3 to 30, preferably 4 to 20.

The alkenyl group and the cycloalkenyl group optionally have a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group and a 7-octenyl group, and these groups having a substituent.

"The alkynyl group" may be any of linear or branched. The number of carbon atoms of the alkynyl group is, not including the number of carbon atoms of the substituent, usually 2 to 20, preferably 3 to 20. The number of carbon atoms of the branched alkynyl group is, not including the number of carbon atoms of the substituent, usually 4 to 30, preferably 4 to 20.

The number of carbon atoms of "the cycloalkynyl group" is, not including the number of carbon atoms of the substituent, usually 4 to 30, preferably 4 to 20.

The alkynyl group and the cycloalkynyl group optionally have a substituent, and examples thereof include an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and these groups having a substituent.

"The arylene group" means an atomic group remaining after removing from an aromatic hydrocarbon two hydrogen atoms directly bonding to carbon atoms constituting the ring. The number of carbon atoms of the arylene group is, not including the number of carbon atoms of the substituent, usually 6 to 60, preferably 6 to 30, more preferably 6 to 18.

The arylene group optionally has a substituent, and examples thereof include a phenylene group, a naphthalenediyl group, an anthracenediyl group, a phenanthrenediyl group, a dihydrophenanthrenediyl group, a naphthacenediyl group, a fluorenediyl group, a pyrenediyl group, a perylenediyl group and a chrysenediyl group, and these groups having a substituent, and preferable are groups represented by the formula (A-1) to the formula (A-20). The arylene group includes groups in which a plurality of these groups are connected. 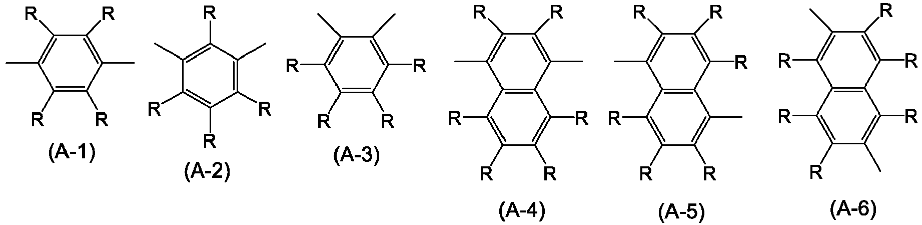 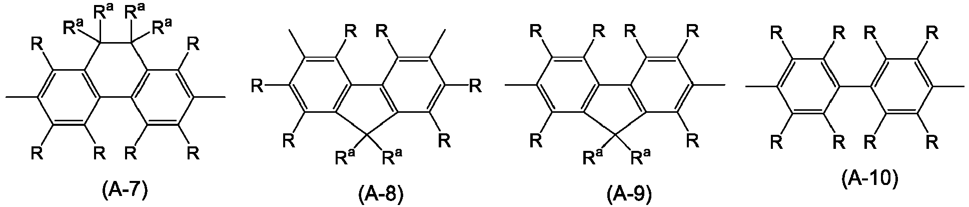 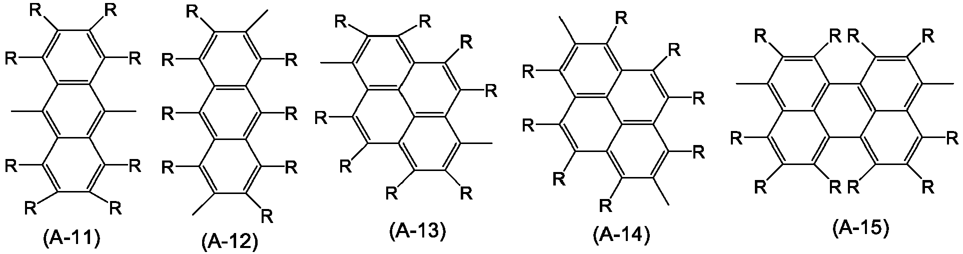 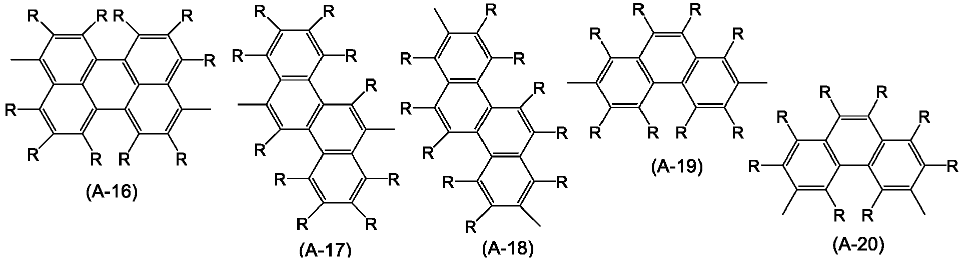 [wherein, R and R^{a} each independently represent a hydrogen atom, an alkyl group, a cycloalkyl group, an aryl group or a monovalent heterocyclic group. A plurality of R and R^{a} each may be the same or different, and the groups of R^{a} may be combined together to form a ring together with the atoms to which they are attached.]

The number of carbon atoms of the divalent heterocyclic group is, not including the number of carbon atoms of the substituent, usually 2 to 60, preferably 3 to 20, more preferably 4 to 15.

The divalent heterocyclic group optionally has a substituent, and examples thereof include divalent groups obtained by removing from pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole or triazole two hydrogen atoms among hydrogen atoms directly bonding to carbon atoms or hetero atoms constituting the ring, and preferable are groups represented by the formula (AA-1) to the formula (AA-34). The divalent heterocyclic group includes groups in which a plurality of these groups are connected. 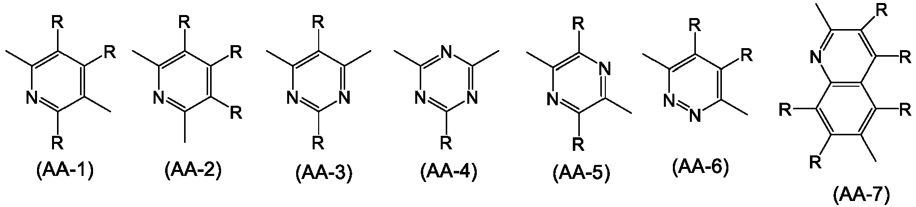 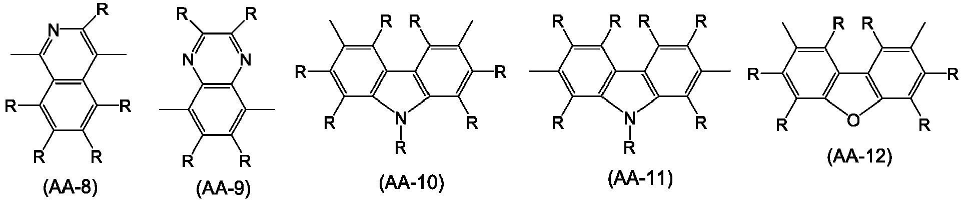 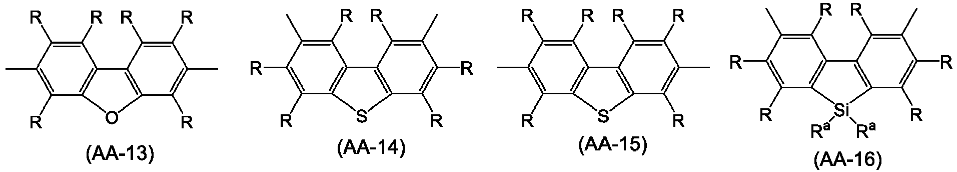 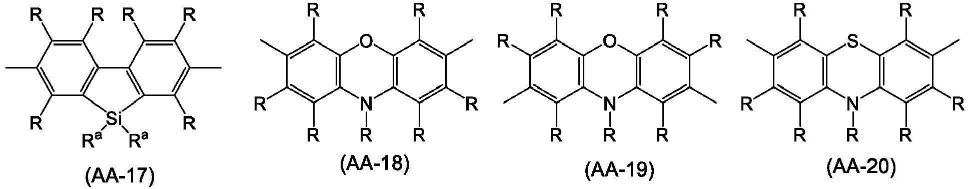 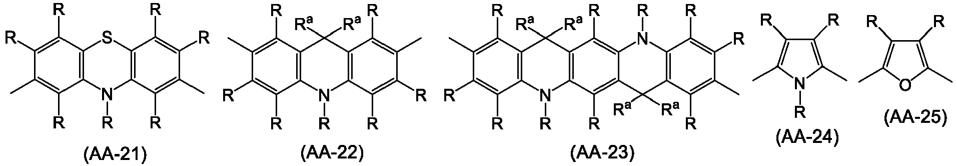 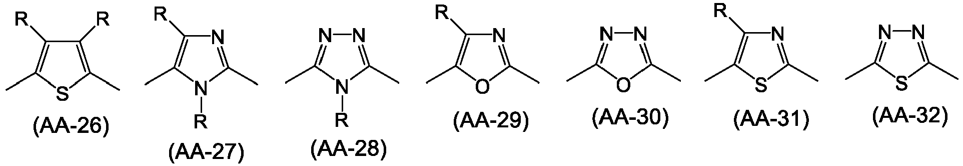 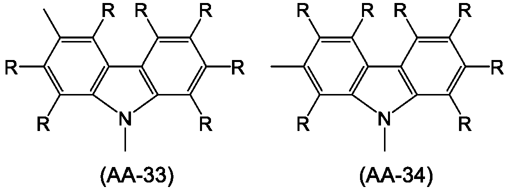 [wherein, R and R^{a} represent the same meaning as described above.]

"The crosslinking group" is a group capable of generating a new bond by being subjected to heating, ultraviolet irradiation, near ultraviolet irradiation, visible light irradiation, infrared irradiation, radical reaction and the like, and preferable are crosslinking groups represented by the formula (XL-1) to the formula (XL-17) in Group A of crosslinking group.

### (Group A of crosslinking group)

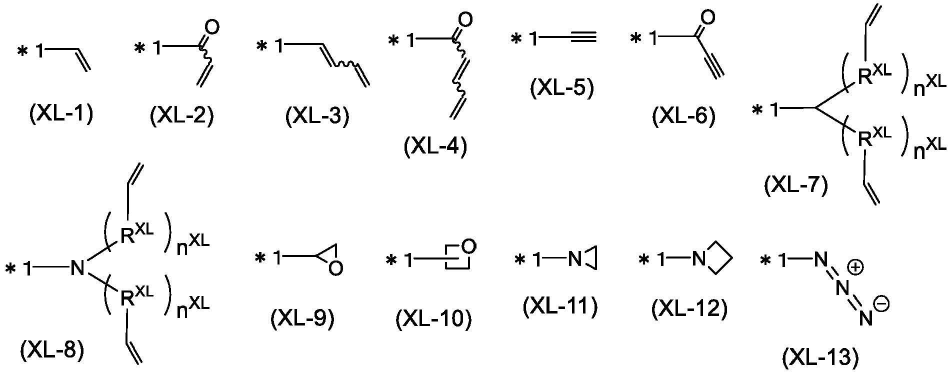

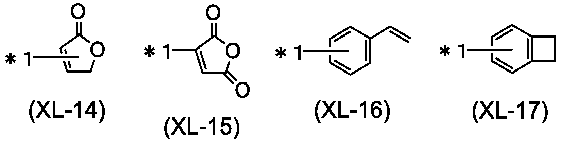

[wherein, R^{XL} represents a methylene group, an oxygen atom or a sulfur atom, and n^{XL} represents an integer of 0 to 5. When a plurality of R^{XL} are present, they may be the same or different, and when a plurality of n^{XL} are present, they may be the same or different. *1 represents the binding position. These crosslinking groups optionally have a substituent.]

"The substituent" represents a halogen atom, a cyano group, an alkyl group, a cycloalkyl group, an aryl group, a monovalent heterocyclic group, an alkoxy group, a cycloalkoxy group, an aryloxy group, amino group, a substituted amino group, an alkenyl group, a cycloalkenyl group, an alkynyl group or a cycloalkynyl group. The substituent may be a crosslinking group.

### <Composition>

The composition of the present invention is a composition containing a compound represented by the formula (C-1) and a phosphorescent compound represented by the formula (1).

In the composition of the present invention, the compound represented by the formula (C-1) and the phosphorescent compound represented by the formula (1) may each be contained only singly or in combination of two or more.

### [Compound represented by the formula (C-1)]

The compound represented by the formula (C-1) has a molecular weight of preferably 2×10² to 5×10⁴, more preferably 2×10² to 5×10³, further preferably 3×10² to 3×10³, particularly preferably 4×10² to 1×10³.

The number of carbon atoms of the aromatic hydrocarbon ring represented by Ring R^{1C}, Ring R^{2C}, Ring R^{3C} and Ring R^{4C} is, not including the number of carbon atoms of the substituent, usually 6 to 60, preferably 6 to 30, more preferably 6 to 18.

The aromatic hydrocarbon ring represented by Ring R^{1C}, Ring R^{2C}, Ring R^{3C} and Ring R^{4C} includes, for example, a benzene ring, a naphthalene ring, an anthracene ring, an indene ring, a fluorene ring, a spirobifluorene ring, a phenanthrene ring, a dihydrophenanthrene ring, a pyrene ring, a chrysene ring and a triphenylene ring, and is preferably a benzene ring, a naphthalene ring, an anthracene ring, a fluorene ring, a spirobifluorene ring, a phenanthrene ring or a dihydrophenanthrene ring, more preferably a benzene ring, a naphthalene ring, a fluorene ring or a spirobifluorene ring, further preferably a benzene ring, and the foregoing rings optionally have a substituent.

The number of carbon atoms of the aromatic hetero ring represented by Ring R^{1C}, Ring R^{2C}, Ring R^{3C} and Ring R^{4C} is, not including the number of carbon atoms of the substituent, usually 2 to 60, preferably 3 to 30, more preferably 4 to 15.

The aromatic hetero ring represented by Ring R^{1C}, Ring R^{2C}, Ring R^{3C} and Ring R^{4C} includes, for example, a pyrrole ring, a diazole ring, a triazole ring, a furan ring, a thiophene ring, an oxadiazole ring, a thiadiazole ring, a pyridine ring, a diazabenzene ring, a triazine ring, an azanaphthalene ring, a diazanaphthalene ring, a triazanaphthalene ring, an azaanthracene ring, a diazaanthracene ring, a triazaanthracene ring, an azaphenanthrene ring, a diazaphenanthrene ring, a triazaphenanthrene ring, a dibenzofuran ring, a dibenzothiophene ring, a dibenzosilole ring, a dibenzophosphole ring, a carbazole ring, an azacarbazole ring, a diazacarbazole ring, a phenoxazine ring, a phenothiazine ring, a dihydroacridine ring and a dihydrophenazine ring, and is preferably a pyridine ring, a diazabenzene ring, an azanaphthalene ring, a diazanaphthalene ring, an azaanthracene ring, a diazaphenanthrene ring, a dibenzofuran ring, a dibenzothiophene ring, a carbazole ring, a phenoxazine ring, a phenothiazine ring, a dihydroacridine ring or a dihydrophenazine ring, more preferably a pyridine ring, a diazabenzene ring, an azanaphthalene ring, a diazanaphthalene ring, a dibenzofuran ring, a dibenzothiophene ring or a carbazole ring, further preferably a pyridine ring or a diazabenzene ring, and the foregoing rings optionally have a substituent.

Since the light emitting device comprising the composition of the present invention (hereinafter, referred to as "light emitting device of the present invention") is more excellent in light emission efficiency, it is preferable that at least one of Ring R^{1C}, Ring R^{2C}, Ring R^{3C} and Ring R^{4C} represents an aromatic hydrocarbon ring, it is more preferable that at least two of Ring R^{1C}, Ring R^{2C}, Ring R^{3C} and Ring R^{4C} represent an aromatic hydrocarbon ring, it is further preferable that all Ring R^{1C}, Ring R^{2C}, Ring R^{3C} and Ring R^{4C} represent an aromatic hydrocarbon ring, it is particularly preferable that all Ring R^{1C}, Ring R^{2C}, Ring R^{3C} and Ring R^{4C} represent a benzene ring, and the foregoing rings optionally have a substituent.

The substituent which Ring R^{1C}, Ring R^{2C}, Ring R^{3C} and Ring R^{4C} optionally have is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an aryl group, a monovalent heterocyclic group or a substituted amino group, further preferably an aryl group or a monovalent heterocyclic group, particularly preferably a monovalent heterocyclic group, and the foregoing groups optionally further have a substituent.

The number of carbon atoms of the aryl group as the substituent which Ring R^{1C}, Ring R^{2C}, Ring R^{3C} and Ring R^{4C} optionally have is, not including the number of carbon atoms of the substituent, usually 6 to 60, preferably 6 to 40, more preferably 6 to 25.

The aryl group as the substituent which Ring R^{1C}, Ring R^{2C}, Ring R^{3C} and Ring R^{4C} optionally have includes, for example, groups obtained by removing from a benzene ring, a naphthalene ring, an anthracene ring, an indene ring, a fluorene ring, a spirobifluorene ring, a phenanthrene ring, a dihydrophenanthrene ring, a pyrene ring, a chrysene ring, a triphenylene ring or a ring obtained by condensing these rings one hydrogen atom directly bonding to a carbon atom constituting the ring, and is preferably a group obtained by removing from a benzene ring, a naphthalene ring, a fluorene ring, a spirobifluorene ring, a phenanthrene ring, a dihydrophenanthrene ring or a triphenylene ring one hydrogen atom directly bonding to a carbon atom constituting the ring, more preferably a group obtained by removing from a benzene ring, a fluorene ring or a spirobifluorene ring one hydrogen atom directly bonding to a carbon atom constituting the ring, further preferably a group obtained by removing from a fluorene ring or a spirobifluorene ring one hydrogen atom directly bonding to a carbon atom constituting the ring, and the foregoing groups optionally further have a substituent.

The number of carbon atoms of the monovalent heterocyclic group as the substituent which Ring R^{1C}, Ring R^{2C}, Ring R^{3C} and Ring R^{4C} optionally have is, not including the number of carbon atoms of the substituent, usually 2 to 60, preferably 3 to 30, more preferably 3 to 15.

The monovalent heterocyclic group as the substituent which Ring R^{1C}, Ring R^{2C}, Ring R^{3C} and Ring R^{4C} optionally have includes, for example, groups obtained by removing from a pyrrole ring, a diazole ring, a triazole ring, a furan ring, a thiophene ring, an oxadiazole ring, a thiadiazole ring, a pyridine ring, a diazabenzene ring, a triazine ring, an azanaphthalene ring, a diazanaphthalene ring, a triazanaphthalene ring, an azaanthracene ring, a diazaanthracene ring, a triazaanthracene ring, an azaphenanthrene ring, a diazaphenanthrene ring, a triazaphenanthrene ring, a dibenzofuran ring, a dibenzothiophene ring, a dibenzosilole ring, a dibenzophosphole ring, a carbazole ring, an azacarbazole ring, a diazacarbazole ring, a phenoxazine ring, a phenothiazine ring, a dihydroacridine ring, a dihydrophenazine ring or a ring obtained by condensing an aromatic ring to these rings one hydrogen atom bonding directly to a carbon atom or a hetero atom constituting the ring, and is preferably a group obtained by removing from a pyridine ring, a diazabenzene ring, a triazine ring, an azanaphthalene ring, a diazanaphthalene ring, a dibenzofuran ring, a dibenzothiophene ring, a carbazole ring, an azacarbazole ring, a diazacarbazole ring, a phenoxazine ring, a phenothiazine ring, a dihydroacridine ring or a dihydrophenazine ring one hydrogen atom directly bonding to a carbon atom or a hetero atom constituting the ring, more preferably a group obtained by removing from a pyridine ring, a diazabenzene ring, a triazine ring, a dibenzofuran ring, a dibenzothiophene ring, a carbazole ring, an azacarbazole ring, a diazacarbazole ring, a phenoxazine ring, a phenothiazine ring, a dihydroacridine ring or a dihydrophenazine ring one hydrogen atom directly bonding to a carbon atom or a hetero atom constituting the ring, further preferably a group obtained by removing from a dibenzofuran ring, a dibenzothiophene ring, a carbazole ring, a phenoxazine ring, a phenothiazine ring, a dihydroacridine ring or a dihydrophenazine ring one hydrogen atom directly bonding to a carbon atom or a hetero atom constituting the ring, particularly preferably a group obtained by removing from a dibenzofuran ring, a dibenzothiophene ring, a carbazole ring or a dihydroacridine ring one hydrogen atom directly bonding to a carbon atom or a hetero atom constituting the ring, especially preferably a ring obtained by removing from a dibenzofuran ring or a dibenzothiophene ring one hydrogen atom directly bonding to a carbon atom constituting the ring, and the foregoing rings optionally have a substituent.

In the substituted amino group as the substituent which Ring R^{1C}, Ring R^{2C}, Ring R^{3C} and Ring R^{4C} optionally have, the substituent which the amino group has is preferably an aryl group or a monovalent heterocyclic group, more preferably an aryl group, and the foregoing groups optionally further have a substituent. The examples and preferable ranges of the aryl group as the substituent which the amino group has are the same as the examples and preferable ranges of the aryl group as the substituent which Ring R^{1C}, Ring R^{2C}, Ring R^{3C} and Ring R^{4C} optionally have. The examples and preferable ranges of the monovalent heterocyclic group as the substituent which the amino group has are the same as the examples and preferable ranges of the monovalent heterocyclic group as the substituent which Ring R^{1C}, Ring R^{2C}, Ring R^{3C} and Ring R^{4C} optionally have.

The substituent which the substituent which Ring R^{1C}, Ring R^{2C}, Ring R^{3C} and Ring R^{4C} optionally have optionally further has is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an aryl group or a monovalent heterocyclic group, further preferably an alkyl group or an aryl group, particularly preferably an alkyl group, and the foregoing groups optionally further have a substituent, but it is preferable that the foregoing groups do not further have a substituent.

The examples and preferable ranges of the aryl group, the monovalent heterocyclic group and the substituted amino group as the substituent which the substituent which Ring R^{1C}, Ring R^{2C}, Ring R^{3C} and Ring R^{4C} optionally have optionally further has are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have.

R^{C} is preferably a carbon atom or a silicon atom, further preferably a carbon atom, since the light emitting device of the present invention is more excellent in light emission efficiency.

Since the light emitting device of the present invention is more excellent in light emission efficiency, at least one of Ring R^{1C}, Ring R^{2C}, Ring R^{3C} and Ring R^{4C} has an aryl group or a monovalent heterocyclic group, and it is more preferable that at least one of Ring R^{1C}, Ring R^{2C}, Ring R^{3C} and Ring R^{4C} has a group represented by the formula (D-1), and the foregoing groups optionally have a substituent.

In the compound of formula (C-1) in which at least one of Ring R^{1C}, Ring R^{2C}, Ring R^{3C} and Ring R^{4C} has an aryl group or a monovalent heterocyclic group, the total number of the aryl group and the monovalent heterocyclic group which Ring R^{1C}, Ring R^{2C}, Ring R^{3C} and Ring R^{4C} have is preferably 1 to 5, more preferably 1 to 3, further preferably 1 or 2, particularly preferably 1.

When at least one of Ring R^{1C}, Ring R^{2C}, Ring R^{3C} and Ring R^{4C} has a group represented by the formula (D-1), the total number of the group represented by the formula (D-1) which Ring R^{1C}, Ring R^{2C}, Ring R^{3C} and Ring R^{4C} have is preferably 1 to 5, more preferably 1 to 3, further preferably 1 or 2, particularly preferably 1.

### Group represented by the formula (D-1)

The examples and preferable ranges of the aromatic hydrocarbon ring and the aromatic hetero ring represented by Ring R^{D} are the same as the examples and preferable ranges of the aromatic hydrocarbon ring and the aromatic hetero ring represented by Ring R^{1C}, Ring R^{2C}, Ring R^{3C} and Ring R^{4C}, respectively.

The examples and preferable ranges of the substituent which Ring R^{D} optionally has are the same as the examples and preferable ranges of the substituent which the substituent which Ring R^{1C}, Ring R^{2C}, Ring R^{3C} and Ring R^{4C} optionally have optionally further has.

Ring R^{D} is preferably an aromatic hydrocarbon ring, more preferably a benzene ring, since the light emitting device of the present invention is more excellent in light emission efficiency.

X^{D1} and X^{D2} are each preferably a single bond, an oxygen atom, a sulfur atom or a group represented by - C(R^{XD2})₂-, more preferably a single bond, an oxygen atom or a sulfur atom, further preferably a single bond or a sulfur atom, since the light emitting device of the present invention is more excellent in light emission efficiency.

It is preferable that at least one of X^{D1} and X^{D2} is a single bond, and it is more preferable that X^{D2} is a single bond.

R^{XD1} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

R^{XD2} is preferably an alkyl group, a cycloalkyl group, an aryl group or a monovalent heterocyclic group, more preferably an alkyl group or an aryl group, and the foregoing groups optionally have a substituent.

The examples and preferable ranges of the aryl group, the monovalent heterocyclic group and the substituted amino group represented by R^{XD1} and R^{XD2} are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have, respectively.

Regarding the combination of two groups R^{XD2} in the group represented by -C(R^{XD2})₂- represented by X^{D1} and X^{D2}, it is preferable that both are alkyl groups or cycloalkyl groups, both are aryl groups, both are monovalent heterocyclic groups, or one is an alkyl group or a cycloalkyl group and the other is an aryl group or a monovalent heterocyclic group, it is more preferable that both are aryl groups, or one is an alkyl group or a cycloalkyl group and the other is an aryl group, it is further preferable that both are aryl groups, and the foregoing groups optionally have a substituent. It is preferable that two groups R^{XD2} are combined together to form a ring together with a carbon atom to which they are attached. When R^{XD2} forms a ring, the group represented by -C(R^{XD2})₂- is preferably a group represented by the formula (Y-A1) to the formula (Y-A5), more preferably a group represented by the formula (Y-A4), and the foregoing groups optionally have a substituent.

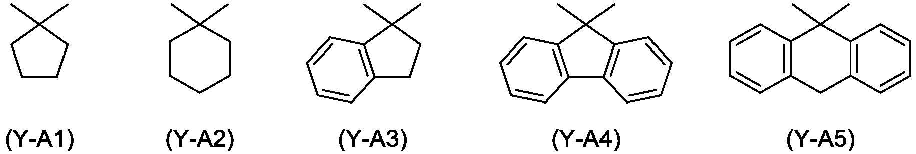

The examples and preferable ranges of the substituent which R^{XD1} and R^{XD2} optionally have are the same as the examples and preferable ranges of the substituent which the substituent which Ring R^{1C}, Ring R^{2C}, Ring R^{3C} and Ring R^{4C} optionally have optionally further has.

E^{1D}, E^{2D} and E^{3D} are each preferably a carbon atom.

R^{1D}, R^{2D} and R^{3D} are each preferably a hydrogen atom, an alkyl group, a cycloalkyl group, an aryl group, a monovalent heterocyclic group or a substituted amino group, more preferably a hydrogen atom, an alkyl group or an aryl group, further preferably a hydrogen atom, and the foregoing groups optionally further have a substituent.

The examples and preferable ranges of the aryl group, the monovalent heterocyclic group and the substituted amino group represented by R^{1D}, R^{2D} and R^{3D} are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have, respectively.

The examples and preferable ranges of the substituent which R^{1D}, R^{2D} and R^{3D} optionally have are the same as the examples and preferable ranges of the substituent which R^{XD1} and R^{XD2} optionally have.

R^{1D} and R^{2D}, R^{2D} and R^{3D}, R^{1D} and R^{XD1}, R^{1D} and R^{XD2}, R^{XD1} and a substituent which Ring R^{D} optionally has, and R^{XD2} and a substituent which Ring R^{D} optionally has each may be combined together to form a ring together with the carbon atoms to which they are attached, but it is preferable that they do not form a ring.

The group represented by the formula (D-1) is preferably a group represented by the formula (D-2), since the light emitting device of the present invention is more excellent in light emission efficiency.

E^{4D}, E^{5D}, E^{6D} and E^{7D} are each preferably a carbon atom.

The examples and preferable ranges of R^{4D}, R^{5D}, R^{6D} and R^{7D} are the same as the examples and preferable ranges of R^{1D}, R^{2D} and R^{3D}.

The examples and preferable ranges of the substituent which R^{4D}, R^{5D}, R^{6D} and R^{7D} optionally have are the same as the examples and preferable ranges of the substituent which R^{1D}, R^{2D} and R^{3D} optionally have.

R^{4D} and R^{5D}, R^{5D} and R^{6D}, and R^{6D} and R^{7D} each may be combined together to form a ring together with the carbon atoms to which they are attached, but it is preferable that they do not form a ring.

### [Compound represented by the formula (C-2)]

The compound represented by the formula (C-1) is preferably a compound represented by the formula (C-2), since the light emitting device of the present invention is more excellent in light emission efficiency.

E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} and E^{44C} are each preferably a carbon atom.

Ring R^{1C}', Ring R^{2C}', Ring R^{3C}' and Ring R^{4C}' are each preferably a benzene ring.

R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} are each preferably a hydrogen atom, an alkyl group, a cycloalkyl group, an aryl group, a monovalent heterocyclic group or a substituted amino group, more preferably a hydrogen atom, an aryl group or a monovalent heterocyclic group, further preferably a hydrogen atom or a group represented by the formula (D-1), and the foregoing groups optionally further have a substituent.

At least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, more preferably a group represented by the formula (D-1), and the foregoing groups optionally further have a substituent.

The examples and preferable ranges of the aryl group, the monovalent heterocyclic group and the substituted amino group represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have.

The examples and preferable ranges of the substituent R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} optionally have are the same as the examples and preferable ranges of the substituent which the substituent which Ring R^{1C}, Ring R^{2C}, Ring R^{3C} and Ring R^{4C} optionally have optionally further has.

In the compound of formula (C-2) in which at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, the total number of the aryl group or the monovalent heterocyclic group represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is preferably 1 to 5, more preferably 1 to 3, further preferably 1 or 2, particularly preferably 1.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is a group represented by the formula (D-1), the total number of the group represented by the formula (D-1) represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is preferably 1 to 5, more preferably 1 to 3, further preferably 1 or 2, particularly preferably 1.

In the compound of formula (C-2) in which at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, it is preferable that at least one of R^{11C}, R^{12C}, R^{14C}, R^{21C}, R^{22C}, R^{24C}, R^{31C}, R^{32C}, R^{34C}, R^{41C}, R^{42C} and R^{44C} is an aryl group or a monovalent heterocyclic group, it is more preferable that at least one of R^{11C}, R^{12C}, R^{21C}, R^{22C}, R^{31C}, R^{32C}, R^{41C} and R^{42C} is an aryl group or a monovalent heterocyclic group, it is further preferable that at least one of R^{11C}, R^{12C}, R^{21C} and R^{22C} is an aryl group or a monovalent heterocyclic group, it is particularly preferable that at least one of R^{12C} and R^{22C} is an aryl group or a monovalent heterocyclic group, and the foregoing groups optionally have a substituent.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is a group represented by the formula (D-1), it is preferable that at least one of R^{11C}, R^{12C}, R^{14C}, R^{21C}, R^{22C}, R^{24C}, R^{31C}, R^{32C}, R^{34C}, R^{41C}, R^{42C} and R^{44C} is a group represented by the formula (D-1), it is more preferable that at least one of R^{11C}, R^{12C}, R^{21C}, R^{22C}, R^{31C}, R^{32C}, R^{41C} and R^{42C} is a group represented by the formula (D-1), it is further preferable that at least one of R^{11C}, R^{12C}, R^{21C} and R^{22C} is a group represented by the formula (D-1), it is particularly preferable that at least one of R^{11C} and R^{12C} is a group represented by the formula (D-1), it is especially preferable that R^{12C} is a group represented by the formula (D-1).

R^{11C} and R^{12C}, R^{12C} and R^{13C}, R^{13C} and R^{14C}, R^{14C} and R^{34C}, R^{34C} and R^{33C}, R^{33C} and R^{32C}, R^{32C} and R^{31C}, R^{31C} and R^{41C}, R^{41C} and R^{42C}, R^{42C} and R^{43C}, R^{43C} and R^{44C}, R^{44C} and R^{24C}, R^{24C} and R^{23C}, R^{23C} and R^{22C}, R^{22C} and R^{21C}, and R^{21C} and R^{11C} each may be combined together to form a ring together with the carbon atoms to which they are attached, but it is preferable that they do not form a ring.

### [Compound represented by the formula (C-3)]

The compound represented by the formula (C-2) is preferably a compound represented by the formula (C-3), since the light emitting device of the present invention is more excellent in light emission efficiency.

The compound represented by the formula (C-1) includes, for example, compounds represented by the formula (C-101) to the formula (C-137). 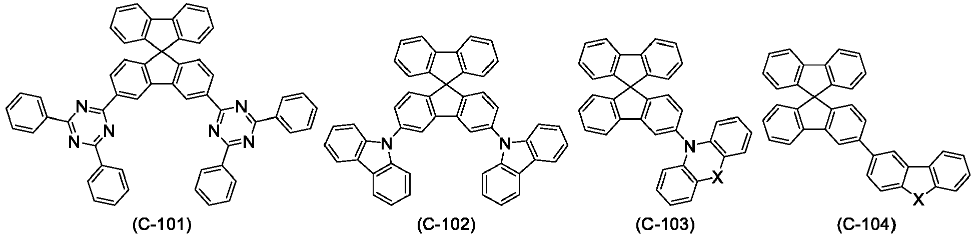 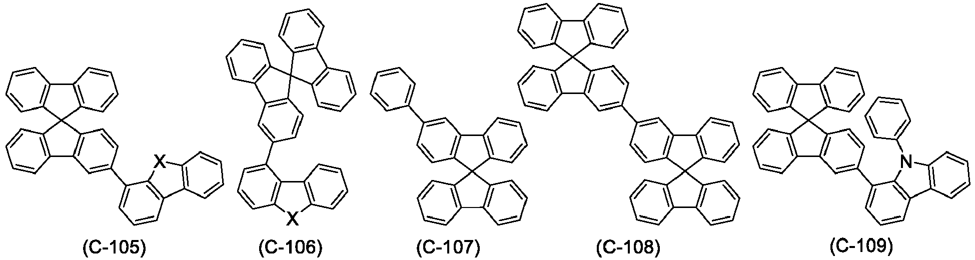 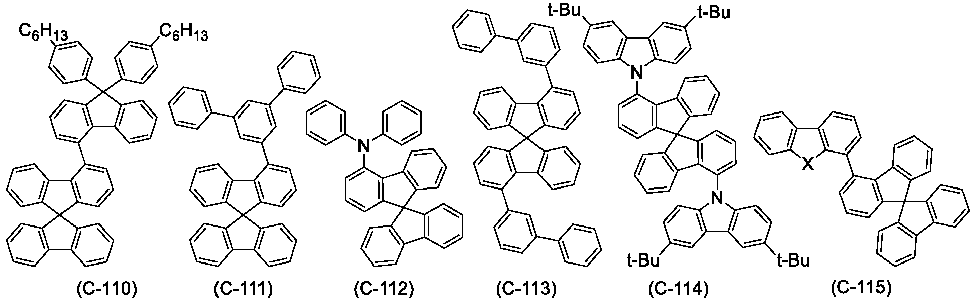 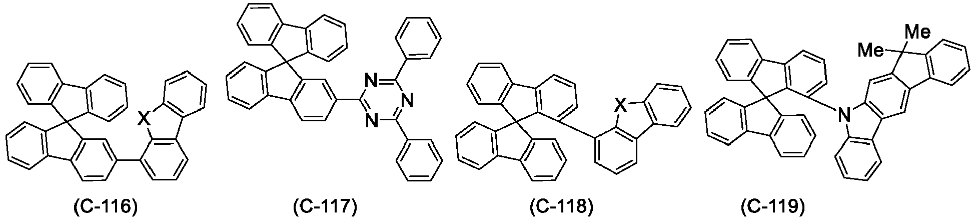 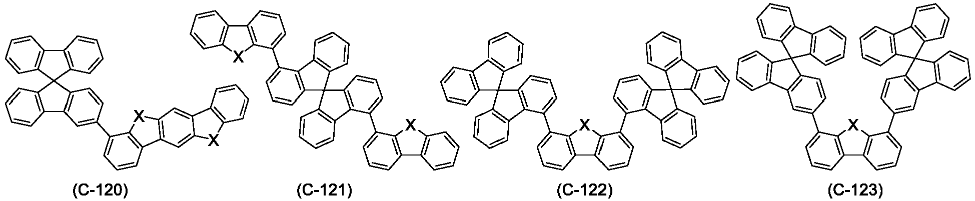 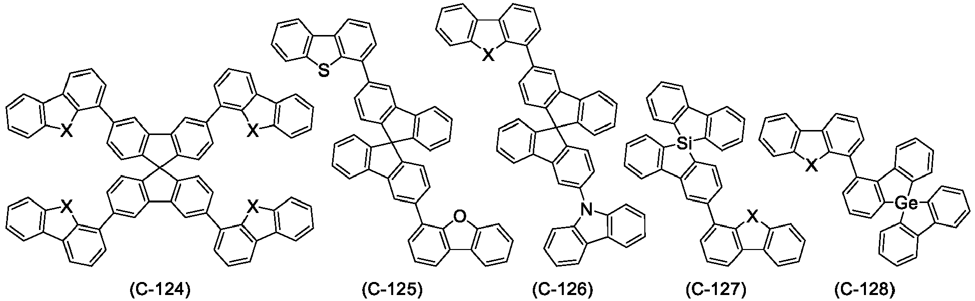 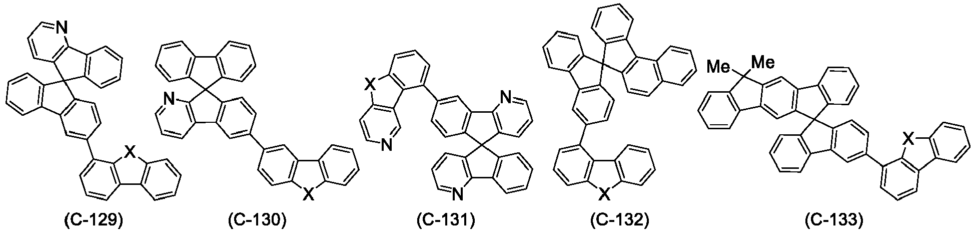 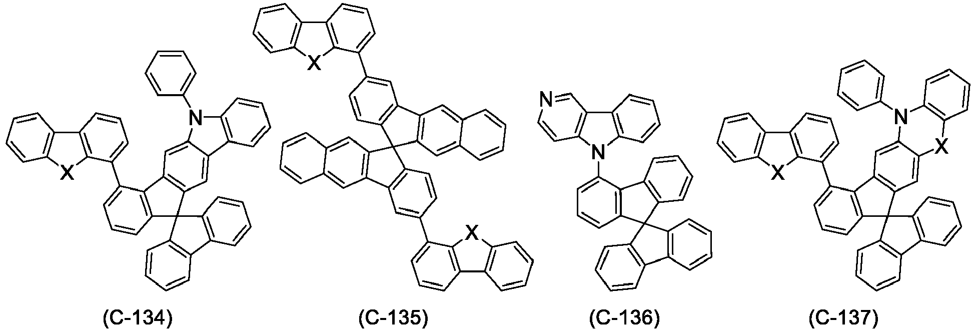 [wherein, X represents an oxygen atom or a sulfur atom. When a plurality of X are present, they may be the same or different.]

X is preferably a sulfur atom.

The compound represented by the formula (C-1) is available from, for example, Aldrich, Luminescence Technology Corp. The compound represented by the formula (C-1) can be synthesized according to methods described in, for example, International Publication WO 2014/023388, International Publication WO 2013/045408, International Publication WO 2013/045410, International Publication WO 2013/045411, International Publication WO 2012/048820, International Publication WO 2012/048819, International Publication WO 2011/006574 and "Organic Electronics vol. 14, 902-908 (2013)", as other means.

In the composition of the present invention, the compound represented by the formula (C-1) is preferably a host material having at least on function selected from the group consisting of hole injectability, hole transportability, electron injectability and electron transportability, since the light emitting device of the present invention is more excellent in light emission efficiency.

In the composition of the present invention, the lowest excited triplet state (T₁) of the compound represented by the formula (C-1) is preferably at energy level corresponding to that of the phosphorescent compound represented by the formula (1) or higher energy level than it, more preferably at higher energy level than it, since the light emitting device of the present invention is more excellent in light emission efficiency.

In the composition of the present invention, the compound represented by the formula (C-1) is preferably one that shows solubility in a solvent which is capable of dissolving a phosphorescent compound represented by the formula (1), since the light emitting device of the present invention can be fabricated by a solution application process.

### [Phosphorescent compound represented by the formula (1) ]

The phosphorescent compound represented by the formula (1) is usually a metal complex showing phosphorescence at room temperature (25°C), preferably a metal complex showing light emission from triplet excited state at room temperature.

M¹ is preferably an iridium atom or a platinum atom, more preferably an iridium atom, since the light emitting device of the present invention is more excellent in light emission efficiency.

When M¹ is a rhodium atom or an iridium atom, n¹ is preferably 2 or 3, more preferably 3.

When M¹ is a palladium atom or a platinum atom, n¹ is preferably 2.

E¹ and E² are each preferably a carbon atom.

Ring R^{1A} is preferably a triazole ring in which E^{11A} and E^{12A} are each a nitrogen atom or a triazole ring in which E^{11A} and E^{13A} are each a nitrogen atom, more preferably a triazole ring in which E^{11A} and E^{13A} are each a nitrogen atom.

When E^{11A} is a nitrogen atom and R^{11A} is present, R^{11A} is preferably an alkyl group, a cycloalkyl group, an aryl group or a monovalent heterocyclic group, more preferably an alkyl group or an aryl group, further preferably an aryl group, and the foregoing groups optionally have a substituent.

When E^{11A} is a carbon atom, R^{11A} is preferably an alkyl group, a cycloalkyl group, an aryl group, a monovalent heterocyclic group or a substituted amino group, more preferably an alkyl group, a cycloalkyl group or an aryl group, further preferably an alkyl group or an aryl group, and the foregoing groups optionally have a substituent.

When E^{12A} is a nitrogen atom and R^{12A} is present, R^{12A} is preferably an alkyl group, a cycloalkyl group, an aryl group or a monovalent heterocyclic group, more preferably an alkyl group or an aryl group, further preferably an aryl group, and the foregoing groups optionally have a substituent.

When E^{12A} is a carbon atom, R^{12A} is preferably an alkyl group, a cycloalkyl group, an aryl group, a monovalent heterocyclic group or a substituted amino group, more preferably an alkyl group, a cycloalkyl group or an aryl group, further preferably an alkyl group or an aryl group, and the foregoing groups optionally have a substituent.

When E^{13A} is a nitrogen atom and R^{13A} is present, R^{13A} is preferably an alkyl group, a cycloalkyl group, an aryl group or a monovalent heterocyclic group, more preferably an alkyl group or an aryl group, further preferably an aryl group, and the foregoing groups optionally have a substituent.

When E^{13A} is a carbon atom, R^{13A} is preferably an alkyl group, a cycloalkyl group, an aryl group, a monovalent heterocyclic group or a substituted amino group, more preferably an alkyl group, a cycloalkyl group or an aryl group, further preferably an alkyl group, a cycloalkyl group or an aryl group, particularly preferably an alkyl group, and the foregoing groups optionally have a substituent.

Ring R^{1A} is preferably a triazole ring in which E^{11A} and E^{12A} are each a nitrogen atom and R^{11A} is present and R^{12A} is absent or a triazole ring in which E^{11A} and E^{13A} are each a nitrogen atom and R^{11A} is present and R^{13A} is absent, more preferably a triazole ring in which E^{11A} and E^{13A} are each a nitrogen atom and R^{11A} is present and R^{13A} absent.

Two of R^{11A}, R^{12A} and R^{13A} are each preferably an alkyl group, a cycloalkyl group, an aryl group or a monovalent heterocyclic group, more preferably an alkyl group or an aryl group, and the foregoing groups optionally have a substituent.

The aryl group represented by R^{11A}, R^{12A} and R^{13A} is preferably a phenyl group, a naphthyl group, an anthracenyl group, a phenanthrenyl group, a dihydrophenanthrenyl group, a fluorenyl group or a pyrenyl group, more preferably a phenyl group, a naphthyl group or a fluorenyl group, further preferably a phenyl group, and the foregoing groups optionally have a substituent.

The monovalent heterocyclic group represented by R^{11A}, R^{12A} and R^{13A} is preferably a pyridyl group, a pyrimidinyl group, a triazinyl group, a quinolinyl group, an isoquinolinyl group, a dibenzofuranyl group, a dibenzothienyl group, a carbazolyl group, an azacarbazolyl group, a diazacarbazolyl group, a phenoxazinyl group or a phenothiazinyl group, more preferably a pyridyl group, a pyrimidinyl group, a triazinyl group, a dibenzofuranyl group, a carbazolyl group, an azacarbazolyl group or a diazacarbazolyl group, further preferably a pyridyl group, a pyrimidinyl group or a triazinyl group, and the foregoing groups optionally have a substituent.

In the substituted amino group represented by R^{11A}, R^{12A} and R^{13A}, the substituent which the amino group has is preferably an aryl group or a monovalent heterocyclic group, more preferably an aryl group, and the foregoing groups optionally further have a substituent. The examples and preferable ranges of the aryl group as the substituent which the amino group has are the same as the examples and preferable ranges of the aryl group represented by R^{11A}, R^{12A} and R^{13A}. The examples and preferable ranges of the monovalent heterocyclic group as the substituent which the amino group has are the same as the examples and preferable ranges of the monovalent heterocyclic group represented by R^{11A}, R^{12A} and R^{13A}.

The substituent which R^{11A}, R^{12A} and R^{13A} optionally have is preferably an alkyl group, a cycloalkyl group, an aryl group, a monovalent heterocyclic group, an alkoxy group, a cycloalkoxy group or a substituted amino group, more preferably an alkyl group, a cycloalkyl group, an aryl group, a monovalent heterocyclic group or a substituted amino group, further preferably an alkyl group, a cycloalkyl group or an aryl group, particularly preferably an alkyl group, and the foregoing groups optionally further have a substituent, but it is preferable that these groups do not further have a substituent.

The examples and preferable ranges of the aryl group, the monovalent heterocyclic group and the substituted amino group as the substituent which R^{11A}, R^{12A} and R^{13A} optionally have are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have, respectively.

The aryl group, the monovalent heterocyclic group or the substituted amino group represented by R^{11A}, R^{12A} and R^{13A} is preferably a group represented by the formula (D-A), the formula (D-B) or the formula (D-C), more preferably a group represented by the formula (D-A) or the formula (D-C), further preferably a group represented by the formula (D-C), since the light emitting device of the present invention is more excellent in light emission efficiency. 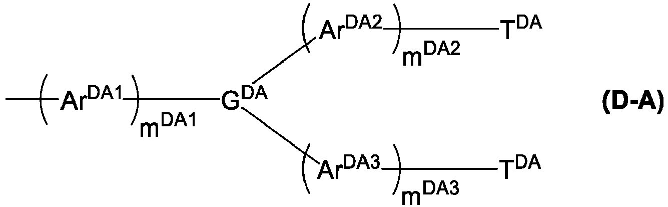 [wherein,
m^{DA1}, m^{DA2} and m^{DA3} each independently represent an integer of 0 or more.
G^{DA} represents a nitrogen atom, an aromatic hydrocarbon group or a heterocyclic group, and the foregoing groups optionally have a substituent.
Ar^{DA1}, Ar^{DA2} and Ar^{DA3} each independently represent an arylene group or a divalent heterocyclic group, and the foregoing groups optionally have a substituent. When a plurality of Ar^{DA1}, Ar^{DA2} and Ar^{DA3} are present, they each may be the same or different.
T^{DA} represents an aryl group or a monovalent heterocyclic group, and the foregoing groups optionally have a substituent. A plurality of T^{DA} may be the same or different.] 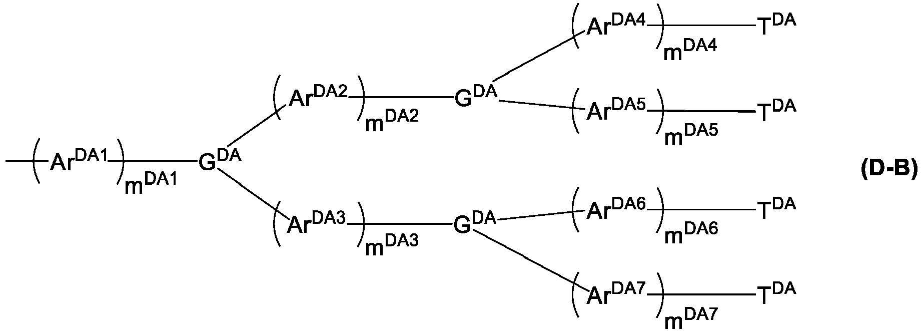 [wherein,
m^{DA1}, m^{DA2}, m^{DA3} , m^{DA4} , m^{DA5}, m^{DA6} and m^{DA7} each independently represent an integer of 0 or more.
G^{DA} represents a nitrogen atom, an aromatic hydrocarbon group or a heterocyclic group, and the foregoing groups optionally have a substituent. A plurality of G^{DA} may be the same or different.
Ar^{DA1}, Ar^{DA2}, Ar^{DA3}, Ar^{DA4}, Ar^{DA5}, Ar^{DA6} and Ar^{DA7} each independently represent an arylene group or a divalent heterocyclic group, and the foregoing groups optionally have a substituent. When a plurality of Ar^{DA1}, Ar^{DA2}, Ar^{DA3}, Ar^{DA4}, Ar^{DA5}, Ar^{DA6} and Ar^{DA7} are present, they each may be the same or different.
T^{DA} represents an aryl group or a monovalent heterocyclic group, and the foregoing groups optionally have a substituent. A plurality of T^{DA} may be the same or different.] 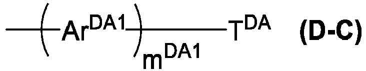 [wherein,
m^{DA1} represents an integer of 0 or more.
Ar^{DA1} represents an arylene group or a divalent heterocyclic group, and the foregoing groups optionally have a substituent. When a plurality of Ar^{DA1} are present, they may be the same or different.
T^{DA} represents an aryl group or a monovalent heterocyclic group, and the foregoing groups optionally have a substituent.]
m^{DA1}, m^{DA2}, m^{DA3}, m^{DA4} , m^{DA5}, m^{DA6} and m^{DA7} are each usually an integer of 10 or less, preferably an integer of 5 or less, more preferably an integer of 2 or less, further preferably 0 or 1. It is preferable that m^{DA2}, m^{DA3}, m^{DA4} , m^{DA5}, m^{DA6} and m^{DA7} are the same integer, and it is more preferable that m^{DA1}, m^{DA2}, m^{DA3}, m^{DA4} , m^{DA5}, m^{DA6} and m^{DA7} are the same integer.
G^{DA} is preferably an aromatic hydrocarbon group or a heterocyclic group, more preferably a group obtained by removing from a benzene ring, a pyridine ring, a pyrimidine ring, a triazine ring or a carbazole ring three hydrogen atoms directly bonding to carbon atoms or nitrogen atoms constituting the ring, and the foregoing groups optionally have a substituent.

The substituent which G^{DA} optionally has is preferably an alkyl group, a cycloalkyl group, an alkoxy group, a cycloalkoxy group, an aryl group or a monovalent heterocyclic group, more preferably an alkyl group, a cycloalkyl group, an alkoxy group or a cycloalkoxy group, further preferably an alkyl group or a cycloalkyl group, and the foregoing groups optionally further have a substituent, but it is preferable that these groups do not further have a substituent.

G^{DA} is preferably a group represented by the formula (GDA-11) to the formula (GDA-15), more preferably a group represented by the formula (GDA-11) to the formula (GDA-14), further preferably a group represented by the formula (GDA-11) or the formula (GDA-14), particularly a group represented by the formula (GDA-11). 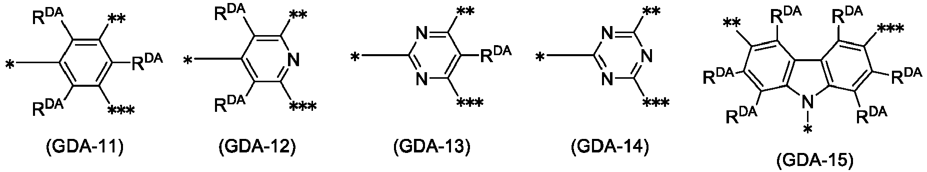 [wherein,
* represents a bond to Ar^{DA1} in the formula (D-A), to Ar^{DA1} in the formula (D-B), to Ar^{DA2} in the formula (D-B) or to Ar^{DA3} in the formula (D-B).
** represents a bond to Ar^{DA2} in the formula (D-A), to Ar^{DA2} in the formula (D-B), to Ar^{DA4} in the formula (D-B) or to Ar^{DA6} in the formula (D-B).
*** represents a bond to Ar^{DA3} in the formula (D-A), to Ar^{DA3} in the formula (D-B), to Ar^{DA5} in the formula (D-B) or to Ar^{DA7} in the formula (D-B).

R^{DA} represents a hydrogen atom, an alkyl group, a cycloalkyl group, an alkoxy group, a cycloalkoxy group, an aryl group or a monovalent heterocyclic group, and the foregoing groups optionally further have a substituent. When a plurality of R^{DA} are present, they may be the same or different.]

R^{DA} is preferably a hydrogen atom, an alkyl group, a cycloalkyl group, an alkoxy group or a cycloalkoxy group, more preferably a hydrogen atom, an alkyl group or a cycloalkyl group, and the foregoing groups optionally have a substituent.

Ar^{DA1}, Ar^{DA2}, Ar^{DA3}, Ar^{DA4}, Ar^{DA5}, Ar^{DA6} and Ar^{DA7} are each preferably a phenylene group, a fluorenediyl group or a carbazolediyl group, more preferably a group represented by the formula (ArDA-1) to (ArDA-5), further preferably a group represented by the formula (ArDA-1) to the formula (ArDA-3), particularly preferably a group represented by the formula (ArDA-1), and the foregoing groups optionally have a substituent. 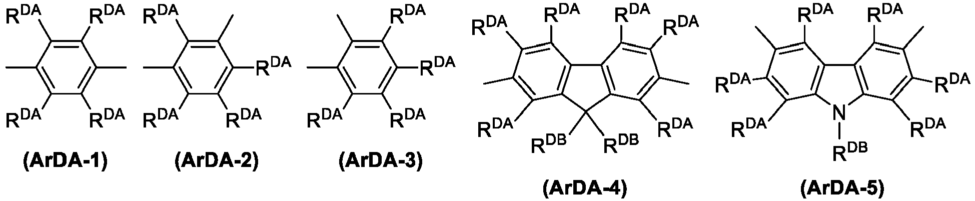 [wherein,
R^{DA} represents the same meaning as described above.
R^{DB} represents a hydrogen atom, an alkyl group, a cycloalkyl group, an aryl group or a monovalent heterocyclic group, and the foregoing groups optionally have a substituent. When a plurality of R^{DB} are present, they may be the same or different.]

R^{DB} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

The examples and preferable ranges of the substituent which Ar^{DA1}, Ar^{DA2}, Ar^{DA3}, Ar^{DA4}, Ar^{DA5}, Ar^{DA6}, Ar^{DA7} and R^{DB} optionally have are the same as the examples and preferable ranges of the substituent which G^{DA} optionally has.

T^{DA} is preferably a group represented by the formula (TDA-1) to the formula (TDA-3), more preferably a group represented by the formula (TDA-1). 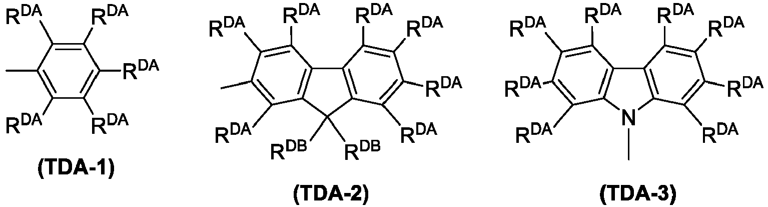 [wherein, R^{DA} and R^{DB} represent the same meaning as described above.]

The group represented by the formula (D-A) is preferably a group represented by the formula (D-A1) to the formula (D-A5), more preferably a group represented by the formula (D-A1), the formula (D-A4) or the formula (D-A5), further preferably a group represented by the formula (D-A1). 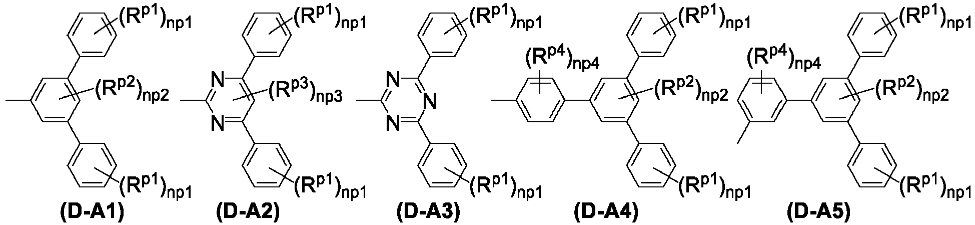 [wherein,
R^{p1}, R^{p2}, R^{p3} and R^{p4} each independently represent an alkyl group, a cycloalkyl group, an alkoxy group, a cycloalkoxy group or a fluorine atom. When a plurality of R^{p1}, R^{p2} and R^{p4} are present, they each may be the same or different.
np1 represents an integer of 0 to 5, np2 represents an integer of 0 to 3, np3 represents 0 or 1, and np4 represents an integer of 0 to 4. A plurality of np1 may be the same or different.]

The group represented by the formula (D-B) is preferably a group represented by the formula (D-B1) to the formula (D-B6), more preferably a group represented by the formula (D-B1) to the formula (D-B3) or the formula (D-B5), further preferably a group represented by the formula (D-B1). 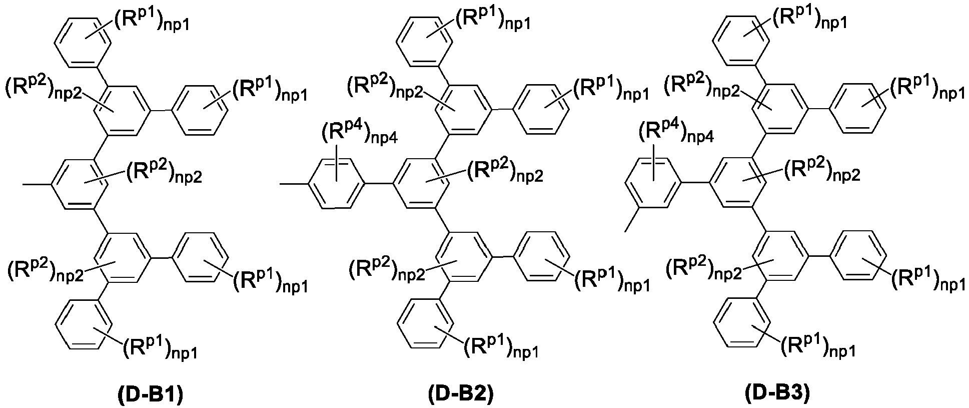 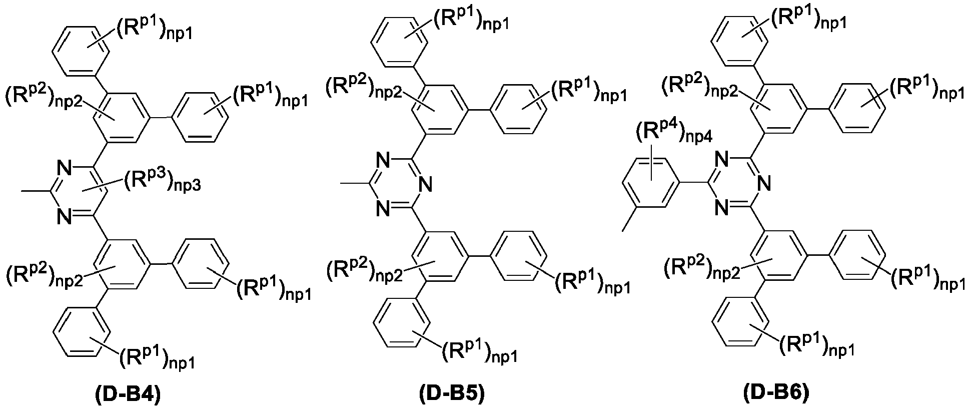 [wherein,
R^{p1}, R^{p2}, R^{p3} and R^{p4} each independently represent an alkyl group, a cycloalkyl group, an alkoxy group, a cycloalkoxy group or a fluorine atom. When a plurality of R^{p1}, R^{p2} and R^{p4} are present, they each may be the same or different.
np1 represents an integer of 0 to 5, np2 represents an integer of 0 to 3, np3 represents 0 or 1, and np4 represents an integer of 0 to 4. A plurality of np1 and np2 each may be the same or different.]

The group represented by the formula (D-C) is preferably a group represented by the formula (D-C1) to the formula (D-C4), more preferably a group represented by the formula (D-C1) or the formula (D-C2), further preferably a group represented by the formula (D-C1). 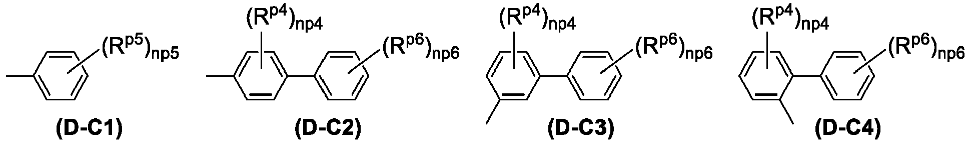 [wherein,
R^{p4}, R^{p5} and R^{p6} each independently represent an alkyl group, a cycloalkyl group, an alkoxy group, a cycloalkoxy group or a fluorine atom. When a plurality of R^{p4}, R^{p5} and R^{p6} are present, they each may be the same or different.
np4 represents an integer of 0 to 4, np5 represents an integer of 0 to 5, and np6 represents an integer of 0 to 5.]
np1 is preferably an integer of 0 to 2, more preferably 0 or 1. np2 is preferably 0 or 1, more preferably 0. np3 is preferably 0. np4 is preferably an integer of 0 to 2, more preferably 0. np5 is preferably an integer of 0 to 3, more preferably 0 or 1. np6 is preferably an integer of 0 to 2, more preferably 0 or 1.

The alkyl group or the cycloalkyl group represented by R^{p1}, R^{p2}, R^{p3}, R^{p4}, R^{p5} and R^{p6} is preferably a methyl group, an ethyl group, an isopropyl group, a tert-butyl group, a hexyl group, a 2-ethylhexyl group, a cyclohexyl group or a tert-octyl group.

The alkoxy group or the cycloalkoxy group represented by R^{p1}, R^{p2}, R^{p3}, R^{p4}, R^{p5} and R^{p6} is preferably a methoxy group, a 2-ethylhexyloxy group or a cyclohexyloxy group.

R^{p1}, R^{p2}, R^{p3}, R^{p4}, R^{p5} and R^{p6} are each preferably an alkyl group optionally having a substituent or a cycloalkyl group optionally having a substituent, more preferably an alkyl group optionally having a substituent, further preferably a methyl group, an ethyl group, an isopropyl group, a tert-butyl group, a hexyl group, a 2-ethylhexyl group or a tert-octyl group.

At least one selected from the group consisting of R^{11A}, R^{12A} and R^{13A} is preferably an alkyl group optionally having a substituent, an aryl group optionally having a substituent or a monovalent heterocyclic group optionally having a substituent, more preferably an alkyl group optionally having a substituent or an aryl group optionally having a substituent, further preferably an alkyl group optionally having a substituent or a group represented by the formula (D-A1), the formula (D-A4), the formula (D-A5), the formula (D-B1) to the formula (D-B3) or the formula (D-C1) to the formula (D-C4), particularly preferably an alkyl group optionally having a substituent or a group represented by the formula (D-C1) or the formula (D-C2), since the light emitting device of the present invention is more excellent in light emission efficiency.

When at least one of R^{11A}, R^{12A} and R^{13A} is an alkyl group optionally having a substituent, an aryl group optionally having a substituent or a monovalent heterocyclic group optionally having a substituent, it is preferable that R^{11A} is an alkyl group optionally having a substituent, an aryl group optionally having a substituent or a monovalent heterocyclic group optionally having a substituent, it is more preferable that R^{11A} is an alkyl group optionally having a substituent or an aryl group optionally having a substituent, it is further preferable that R^{11A} is an aryl group optionally having a substituent.

It is preferable that R^{11A} and R^{12A}, R^{12A} and R^{13A}, and the substituent which Ring R² optionally has and R^{11A} are each not combined together to form a ring together with the atoms to which they are attached, since the maximum peak wavelength of the emission spectrum of the phosphorescent compound represented by the formula (1) is a short wavelength.

Ring R² is preferably a 5-membered or 6-membered aromatic hydrocarbon ring or a 5-membered or 6-membered aromatic hetero ring, more preferably a 6-membered aromatic hydrocarbon ring or a 6-membered aromatic hetero ring, further preferably a 6-membered aromatic hydrocarbon ring, and the foregoing rings optionally have a substituent. When Ring R² is a 6-membered aromatic hetero ring, E² is preferably a carbon atom.

Ring R² includes, for example, a benzene ring, a naphthalene ring, a fluorene ring, a phenanthrene ring, an indene ring, a pyridine ring, a diazabenzene ring and a triazine ring, and is preferably a benzene ring, a naphthalene ring, a fluorene ring, a pyridine ring or a diazabenzene ring, more preferably a benzene ring, a pyridine ring or a diazabenzene ring, further preferably a benzene ring, and the foregoing rings optionally have a substituent.

The substituent which Ring R² optionally has is preferably an alkyl group, a cycloalkyl group, an alkoxy group, a cycloalkoxy group, an aryl group, a monovalent heterocyclic group, a substituted amino group or a fluorine atom, more preferably an alkyl group, a cycloalkyl group, an aryl group, a monovalent heterocyclic group or a substituted amino group, further preferably an alkyl group, or a group represented by the formula (D-A), (D-B) or (D-C), particularly preferably an alkyl group, or a group represented by the formula (D-A), and the foregoing groups optionally further have a substituent.

The examples and preferable ranges of the aryl group, the monovalent heterocyclic group and the substituted amino group as the substituent which Ring R² optionally has are the same as the examples and preferable ranges of the aryl group, the monovalent heterocyclic group and the substituted amino group represented by R^{11A}, R^{12A} and R^{13A}, respectively.

The examples and preferable ranges of the substituent which the substituent which Ring R² optionally has optionally further has are the same as the examples and preferable ranges of the substituent which R^{11A}, R^{12A} and R^{13A} optionally have.

### Anionic bidentate ligand

The anionic bidentate ligand represented by A¹-G¹-A² includes, for example, ligands represented by the following formulae. However, the anionic bidentate ligand represented by A¹-G¹-A² is different from a ligand of which number is defined by subscript n¹. 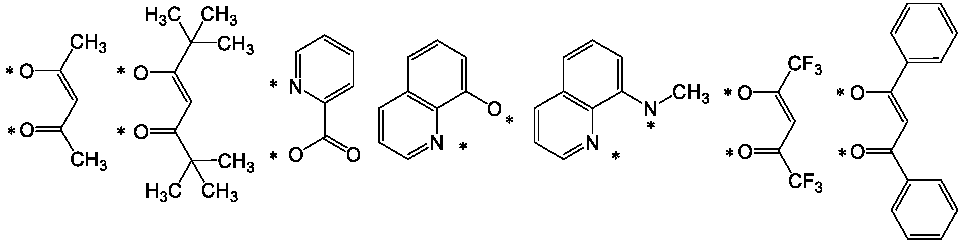 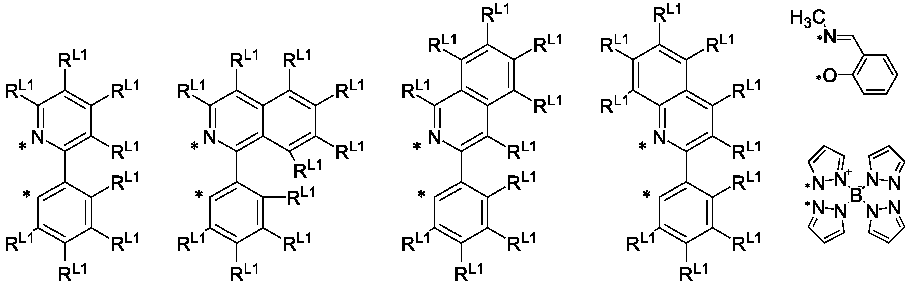 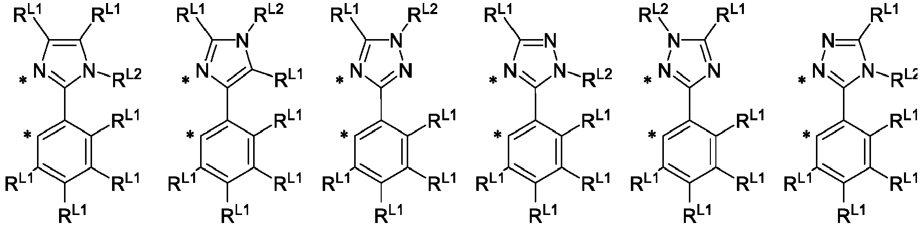 [wherein,
* represents a site binding to M¹.
R^{L1} represents a hydrogen atom, an alkyl group, a cycloalkyl group or a halogen atom, and the foregoing groups optionally have a substituent. A plurality of R^{L1} may be the same or different.
R^{L2} represents an alkyl group, a cycloalkyl group, an aryl group, a monovalent heterocyclic group or a halogen atom, and the foregoing groups optionally have a substituent.]
R^{L1} is preferably a hydrogen atom, an alkyl group, a cycloalkyl group or a fluorine atom, more preferably a hydrogen atom or an alkyl group, and the foregoing groups optionally have a substituent.
R^{L2} is preferably an alkyl group, a cycloalkyl group, an aryl group or a monovalent heterocyclic group, more preferably an aryl group, and the foregoing groups optionally have a substituent.

### [Phosphorescent compound represented by the formula (1-A)]

The phosphorescent compound represented by the formula (1) is preferably a phosphorescent compound represented by the formula (1-A), since the light emitting device of the present invention is more excellent in light emission efficiency.

When Ring R^{2A} is a pyridine ring, a pyridine ring in which E^{21A} is a nitrogen atom, a pyridine ring in which E^{22A} is a nitrogen atom or a pyridine ring in which E^{23A} is a nitrogen atom is preferable, a pyridine ring in which E^{22A} is a nitrogen atom is more preferable.

When Ring R^{2A} is a diazabenzene ring, a pyrimidine ring in which E^{21A} and E^{23A} are each a nitrogen atom or a pyrimidine ring in which E^{22A} and E^{24A} are each a nitrogen atom is preferable, a pyrimidine ring in which E^{22A} and E^{24A} are each a nitrogen atom is more preferable.

Ring R^{2A} is preferably a benzene ring.

R^{21A}, R^{22A}, R^{23A} and R^{24A} are each a hydrogen atom, an alkyl group, a cycloalkyl group, an alkoxy group, a cycloalkoxy group, an aryl group, a monovalent heterocyclic group, a substituted amino group or a fluorine atom, more preferably a hydrogen atom, an alkyl group, a cycloalkyl group, an aryl group, a monovalent heterocyclic group or a substituted amino group, further preferably a hydrogen atom, an alkyl group, or a group represented by the formula (D-A), (D-B) or (D-C), particularly preferably a hydrogen atom, an alkyl group, or a group represented by the formula (D-A), especially preferably a hydrogen atom or a group represented by the formula (D-A), and the foregoing groups optionally have a substituent.

The examples and preferable ranges of the aryl group, the monovalent heterocyclic group and the substituted amino group represented by R^{21A}, R^{22A}, R^{23A} and R^{24A} are the same as the examples and preferable ranges of the aryl group, the monovalent heterocyclic group and the substituted amino group as the substituent which Ring R² optionally has.

The examples and preferable ranges of the substituent which R^{21A}, R^{22A}, R^{23A} and R^{24A} optionally have are the same as the examples and preferable ranges of the substituent which the substituent which Ring R² optionally has optionally further has.

It is preferable that R^{21A} and R^{22A}, R^{22A} and R^{23A}, R^{23A} and R^{24A}, and R^{11A} and R^{21A} are each not combined together to form a ring together with the atoms to which they are attached.

The phosphorescent compound represented by the formula (1-A) is preferably a phosphorescent compound represented by the formula (1-A1) to the formula (1-A3), more preferably a phosphorescent compound represented by the formula (1-A1) or the formula (1-A3), further preferably a phosphorescent compound represented by the formula (1-A3), since the light emitting device of the present invention is more excellent in light emission efficiency.

The phosphorescent compound represented by the formula (1) includes, for example, phosphorescent compounds represented by the following formulae.

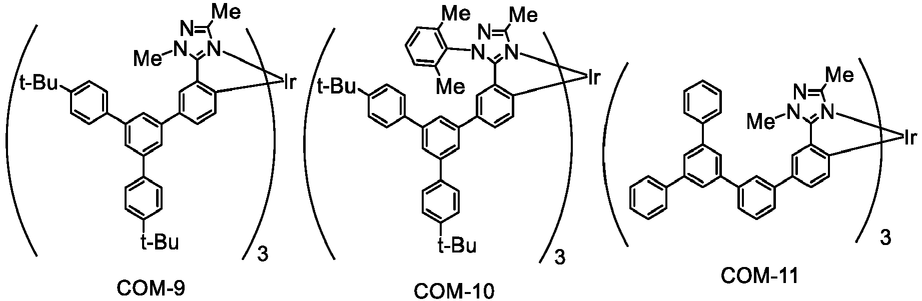

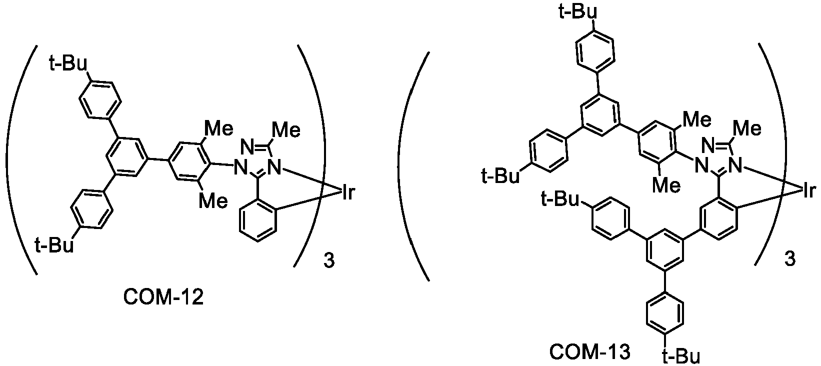

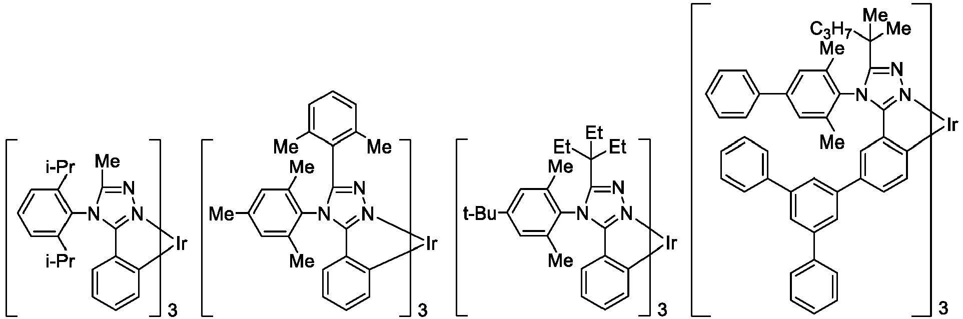

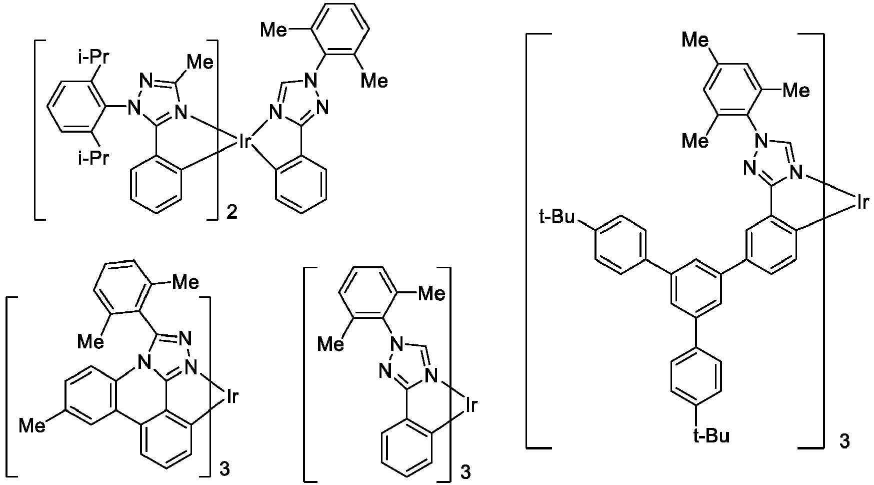

The phosphorescent compound represented by the formula (1) is available, for example, from Aldrich, Luminescence Technology Corp. or American Dye Source. The phosphorescent compound represented by the formula (1) can be synthesized according to methods described in, for example, International Publication WO 2006/121811, International Publication WO 2007/097153, JP-A No. 2013-048190 and JP-A No. 2015-174824, as other means.

In the composition of the present invention, the content of the phosphorescent compound represented by the formula (1) is usually 0.01 to 99 parts by mass when the sum of a compound represented by the formula (C-1) and a phosphorescent compound represented by the formula (1) is taken as 100 parts by mass, and since the light emitting device of the present invention is more excellent in light emission efficiency, it is preferably 0.1 to 80 parts by mass, more preferably 1 to 65 parts by mass, further preferably 10 to 50 parts by mass, particularly preferably 20 to 40 parts by mass.

### [Other components]

The composition of the present invention may further contain at least one material selected from the group consisting of a hole transporting material, a hole injection material, an electron transporting material, an electron injection material, a light emitting material, an antioxidant and a solvent. However, the hole transporting material, the hole injection material, the electron transporting material and the electron injection material are different from a compound represented by the formula (C-1), and the light emitting material is different from a compound represented by the formula (C-1) and a phosphorescent compound represented by the formula (1).

### [Ink]

The composition (hereinafter, referred to as "ink") containing a compound represented by the formula (C-1), a phosphorescent compound represented by the formula (1) and a solvent is suitable for fabrication of a light emitting device using a printing method such as an ink jet print method, a nozzle print method and the like. The viscosity of the ink may be adjusted according to the type of the printing method, and it is preferably 1 to 20 mPa•s at 25°C.

The solvent contained in the ink is preferably a solvent that can dissolve or uniformly disperse the solid content in the ink. The solvent includes, for example, chlorine-based solvents, ether type solvents, aromatic hydrocarbon type solvents, aliphatic hydrocarbon type solvents, ketone type solvents, ester type solvents, poly-hydric alcohol type solvents, alcohol type solvents, sulfoxide type solvents and amide type solvents.

In the ink, the compounding amount of the solvent is usually 1000 to 100000 parts by mass, when the sum of a compound represented by the formula (C-1) and a phosphorescent compound represented by the formula (1) is taken as 100 parts by mass.

The solvents may be used each singly or in combination of two or more.

### [Hole transporting material]

The hole transporting materials are classified into low molecular compounds and polymer compounds, and preferable are polymer compounds having a crosslinking group.

The polymer compound includes, for example, polyvinylcarbazole, and derivatives thereof; polyarylenes having an aromatic amine structure in the side chain or main chain, and derivatives thereof. The polymer compound may also be a compound to which an electron accepting site is bound, such as fullerene, tetrafluorotetracyanoquinodimethane, tetracyanoethylene, trinitrofluorenone and the like.

In the composition of the present invention, the compounding amount of the hole transporting material is usually 1 to 400 parts by mass, when the sum of a compound represented by the formula (C-1) and a phosphorescent compound represented by the formula (1) is taken as 100 parts by mass.

The hole transporting materials may be used each singly or in combination of two or more.

### [Electron transporting material]

The electron transporting material is classified into low molecular compounds and polymer compounds. The electron transporting material may have a crosslinking group.

The low molecular compound includes, for example, metal complexes having 8-hydroxyquinoline as a ligand; oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the composition of the present invention, the compounding amount of the electron transporting material is usually 1 to 400 parts by mass, when the sum of a compound represented by the formula (C-1) and a phosphorescent compound represented by the formula (1) is taken as 100 parts by mass.

The electron transporting materials may be used each singly or in combination of two or more.

### [Hole injection material and electron injection material]

The hole injection material and the electron injection material are each classified into low molecular compounds and polymer compounds. The hole injection material and the electron injection material optionally have a crosslinking group.

The low molecular compound includes, for example, metal phthalocyanines such as copper phthalocyanine and the like; carbon; oxides of metals such as molybdenum, tungsten and the like; and metal fluorides such as lithium fluoride, sodium fluoride, cesium fluoride, potassium fluoride and the like.

The polymer compound includes electrically conductive polymers such as, for example, polyaniline, polythiophene, polypyrrole, polyphenylenevinylene, polythienylenevinylene, polyquinoline and polyquinoxaline, and derivatives thereof; electrically conductive polymers containing an aromatic amine structure in the main chain or side chain, and the like.

In the composition of the present invention, the compounding amounts of the hole injection material and the electron injection material are each usually 1 to 400 parts by mass, when the sum of a compound represented by the formula (C-1) and a phosphorescent compound represented by the formula (1) is taken as 100 parts by mass.

The hole injection materials and the electron injection materials each may be used singly or in combination of two or more.

### [Ion doping]

When the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions. The kind of the ion for doping is an anion for the hole injection material and a cation for the electron injection material. The anion includes, for example, a polystyrenesulfonate ion, an alkylbenzenesulfonate ion and a camphor sulfonate ion. The cation includes, for example, a lithium ion, a sodium ion, a potassium ion and a tetrabutylammonium ion.

The ions for doping may be used each singly or in combination of two or more.

### [Light emitting material]

The light emitting material is classified into low molecular compounds and polymer compounds. The light emitting material may have a crosslinking group.

The low molecular compound includes, for example, naphthalene and derivatives thereof, anthracene and derivatives thereof, perylene and derivatives thereof, and triplet light emitting complexes containing iridium, platinum or europium as the central metal.

The polymer compound includes, for example, polymer compounds containing an arylene group such as a phenylene group, a naphthalenediyl group, a fluorenediyl group, a phenanthrenediyl group, a dihydrophenanthrenediyl group, an anthracenediyl group, a pyrenediyl group and the like; an aromatic amine residue such as a group obtained by removing two hydrogen atoms from an aromatic amine, and the like; and a divalent heterocyclic group such as a carbazolediyl group, a phenoxazinediyl group, a phenothiazinediyl group and the like.

The triplet light emitting complex includes, for example, metal complexes shown below.

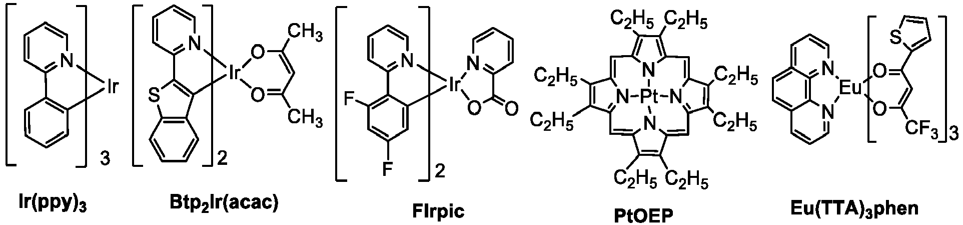

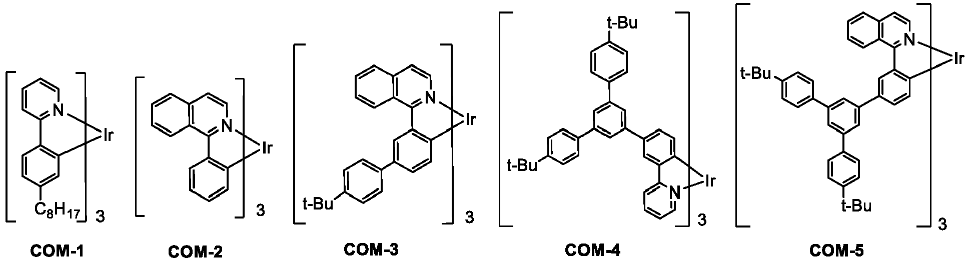

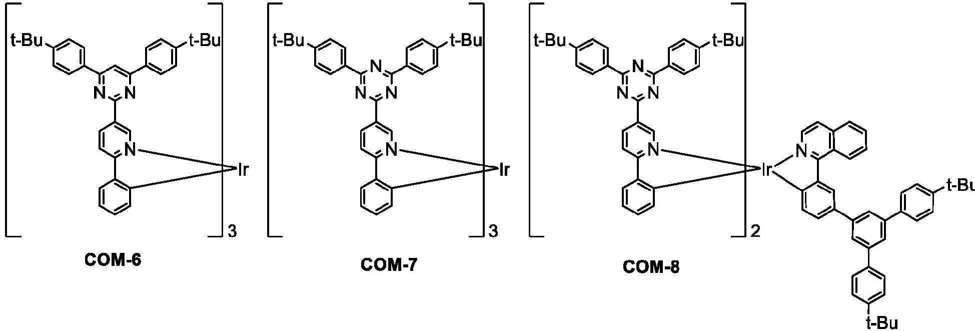

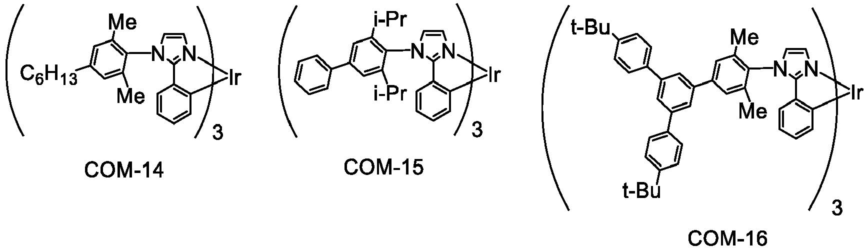

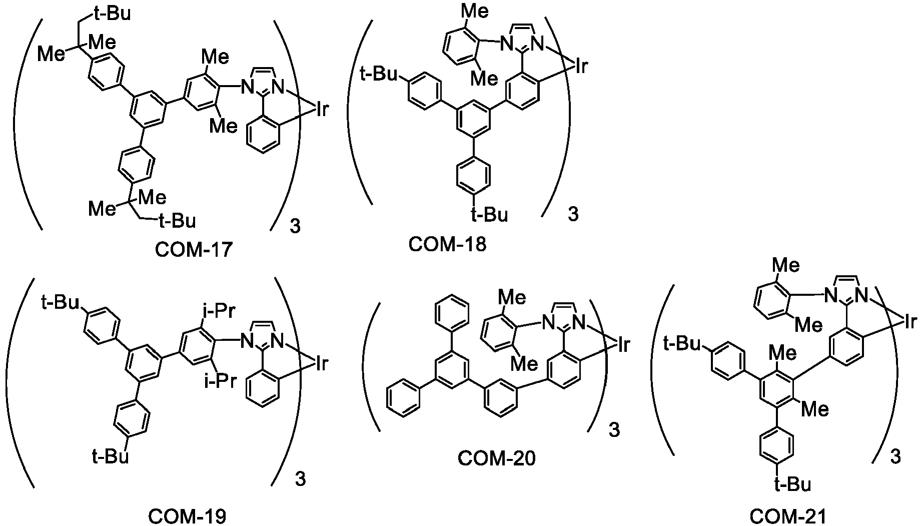

In the composition of the present invention, the compounding amount of the light emitting material is usually 0.1 to 400 parts by mass, when the sum of a compound represented by the formula (C-1) and a phosphorescent compound represented by the formula (1) is taken as 100 parts by mass.

The light emitting materials may be used each singly or in combination of two or more.

### [Antioxidant]

The antioxidant may be a compound which is soluble in the same solvent as for a compound represented by the formula (C-1) and a phosphorescent compound represented by the formula (1) and which does not disturb light emission and charge transportation, and includes, for example, phenol type antioxidants and phosphorus-based antioxidants.

In the composition of the present invention, the compounding amount of the antioxidant is usually 0.001 to 10 parts by mass, when the sum of a compound represented by the formula (C-1) and a phosphorescent compound represented by the formula (1) is taken as 100 parts by mass,.

The antioxidants may be used each singly or in combination of two or more.

### <Film>

The film contains the composition of the present invention.

The film is suitable as a light emitting layer in a light emitting device.

The film can be fabricated by, for example, a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an ink jet printing method, a capillary coat method or a nozzle coat method, using an ink.

The thickness of the film is usually 1 nm to 10 um.

### <Light emitting device>

The light emitting device of the present invention is a light emitting device containing the composition of the present invention.

The constitution of the light emitting device of the present invention includes, for example, electrodes consisting of an anode and a cathode, and a layer containing the composition of the present invention disposed between the electrodes.

### [Layer constitution]

The layer containing the composition of the present invention is usually at least one layer selected from the group consisting of a light emitting layer, a hole transporting layer, a hole injection layer, an electron transporting layer and an electron injection layer, and is preferably a light emitting layer. These layers contain a light emitting material, a hole transporting material, a hole injection material, an electron transporting material and an electron injection material, respectively. These layers can be formed by dissolving a light emitting material, a hole transporting material, a hole injection material, an electron transporting material and an electron injection material in the solvent described above to prepare an ink and by using the same method as for fabrication of the film described above, using the prepared ink.

The light emitting device has a light emitting layer between an anode and a cathode. The light emitting device of the present invention preferably has at least one of a hole injection layer and a hole transporting layer between an anode and a light emitting layer from the standpoint of hole injectability and hole transportability, and preferably has at least one of an electron injection layer and an electron transporting layer between a cathode and a light emitting layer from the standpoint of electron injectability and electron transportability.

As the materials of the hole transporting layer, the electron transporting layer, the light emitting layer, the hole injection layer and the electron injection layer, hole transporting materials, electron transporting materials, light emitting materials, hole injection materials and electron injection materials described above and the like are mentioned, respectively, in addition to the materials in the composition of the present invention.

When the material of the hole transporting layer, the material of the electron transporting layer and the material of the light emitting layer are dissolved in a solvent used in forming a layer adjacent to the hole transporting layer, the electron transporting layer and the light emitting layer in fabrication of a light emitting device, it is preferable that the materials have a crosslinking group to avoid dissolving of the materials in the solvent. After forming each layer using the material having a crosslinking group, the crosslinking group can be cross-linked to insolubilize the layer.

The method for forming each of the light emitting layer, the hole transporting layer, the electron transporting layer, the hole injection layer, the electron injection layer and the like in the light emitting device of the present invention includes, when a low molecular compound is used, for example, a method of vacuum vapor deposition from a powder and a method of forming a film from a solution or melted state, and when a polymer compound is used, for example, a method of forming a film from a solution or melted state.

The order, the number and the thickness of layers to be laminated may be adjusted in consideration of the light emission efficiency and luminance life.

### [Substrate/electrode]

The substrate in the light emitting device may advantageously be a substrate on which an electrode can be formed and which does not change chemically in forming an organic layer, and is, for example, a substrate made of a material such as glass, plastic, silicon and the like. When an opaque substrate is used, it is preferable that the electrode farthest from the substrate is transparent or semi-transparent.

The material of the anode includes, for example, electrically conductive metal oxides and semi-transparent metals, preferably includes indium oxide, zinc oxide, tin oxide; electrically conductive compounds such as indium-tin-oxide (ITO), indium-zinc-oxide and the like; silver-palladium-copper (APC) complex; NESA, gold, platinum, silver and copper.

The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of at least one of them and at least one of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

The anode and the cathode may each have a laminated structure of two or more layers.

### [Application]

In order to obtain planar light emission using a light emitting device, the planar anode and the planar cathode may be arranged so as to overlap each other. In order to obtain patterned light emission, there are a method of installing a mask having a patterned window on the surface of a planar light emitting device, a method in which a layer to be formed as a non-light emitting part is formed extremely thick so as to cause substantially non light emission and a method of forming an anode or a cathode, or both electrodes in a pattern. A segment type display capable of displaying numerals, letters and the like can be obtained by forming a pattern by any one of these methods and disposing several electrodes so that several electrodes can be turned on and off independently. In order to obtain a dot matrix display, both the anode and the cathode may be formed in a stripe shape and arranged so as to be orthogonal to each other. Partial color display and multicolor display become possible by a method of separately coating plural kinds of polymer compounds having different emission colors or a method using a color filter or a fluorescence conversion filter. The dot matrix display can be driven passively or can be driven actively in combination with a TFT and the like. These displays can be used for displays of computers, televisions, portable terminals, and the like. The planar light emitting device can be suitably used as a planar light source for backlight of a liquid crystal display, or as a planar light source for illumination. If a flexible substrate is used, it can be used as a curved light source and a curved display.

### EXAMPLES

The present invention will be illustrated further in detail by examples below, but the present invention is not limited to these examples.

In examples, the polystyrene-equivalent number-average molecular weight (Mn) and the polystyrene-equivalent weight-average molecular weight (Mw) of polymer compounds were determined by the following size exclusion chromatography (SEC) using tetrahydrofuran as a mobile phase.

A polymer compound to be measured was dissolved in tetrahydrofuran at a concentration of about 0.05% by mass, and 10 µL of the solution was injected into SEC. The mobile phase was flowed at a flow rate of 1.0 mL/min. As a column, PLgel MIXED-B (manufactured by Polymer Laboratories Ltd.) was used. As a detector, a UV-VIS detector (trade name: UV-8320GPC manufactured by Tosoh Corp.) was used.

NMR was measured by the following method.

Five to ten mg of a measurement sample was dissolved in about 0.5 mL of deutero-chloroform (CDCl₃), deutero-tetrahydrofuran, deutero-dimethyl sulfoxide, deutero-acetone, deutero-N,N-dimethylformamide, deutero-toluene , deutero-methanol, deutero-ethanol, deutero-2-propanol or deutero-methylene chloride, and NMR thereof was measured using an NMR apparatus (trade name: INOVA300 manufactured by Agilent, trade name: JNM-ECZ400S/L1 manufactured by JEOL RESONANCE, or trade name: AVANCE600 manufactured by Bruker).

As an indicator of the purity of the compound, high performance liquid chromatography (HPLC) area percentage value was used. This value is a value by HPLC (trade name: LC-20A manufactured by Shimadzu Corp.) at UV = 254 nm unless otherwise stated. In this operation, the compound to be measured was dissolved in tetrahydrofuran or chloroform so as to give a concentration of 0.01 to 0.2% by mass, and 1 to 10 µL of the solution was poured into HPLC depending on the concentration. As a mobile phase of HPLC, acetonitrile/tetrahydrofuran were used while changing the ratio thereof from 100/0 to 0/100 (volume ratio), and flowed at a flow rate of 1.0 mL/min. As a column, SUMIPAX CDS Z-CLUE (manufactured by Sumika Chemical Analysis Service, Ltd., internal diameter: 4.6 mm, length: 250 mm, particle size: 3 µm) or an ODS column having the equivalent performance was used. As a detector, a photodiode array detector (trade name: SPD-M20A manufactured by Shimadzu Corp.) was used.

### <Synthesis Example M1> Synthesis of compounds M1 to M5 and metal complex RM1

Compounds M1, M2 and M3 were synthesized according to a method described in International Publication WO 2013/146806.

A compound M4 was synthesized according to a method described in JP-A No. 2012-33845.

A compound M5 was synthesized according to a method described in JP-A No. 2010-189630.

A metal complex RM1 was synthesized according to a method described in International Publication WO 2009/157424.

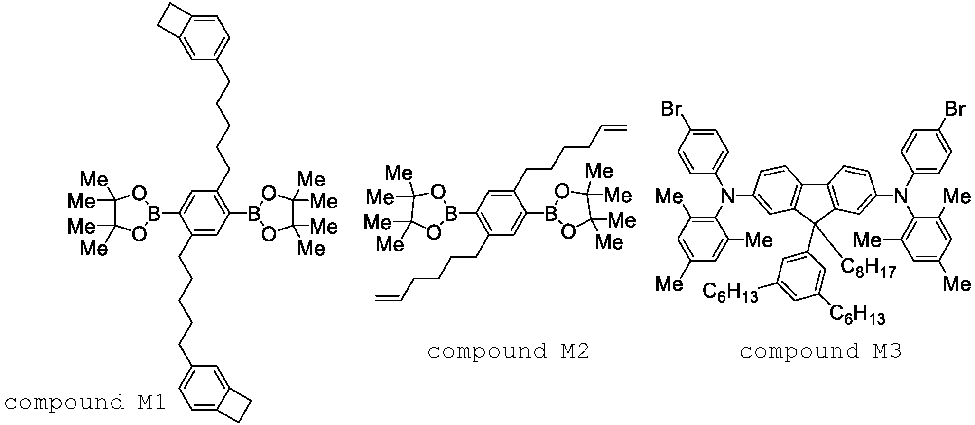

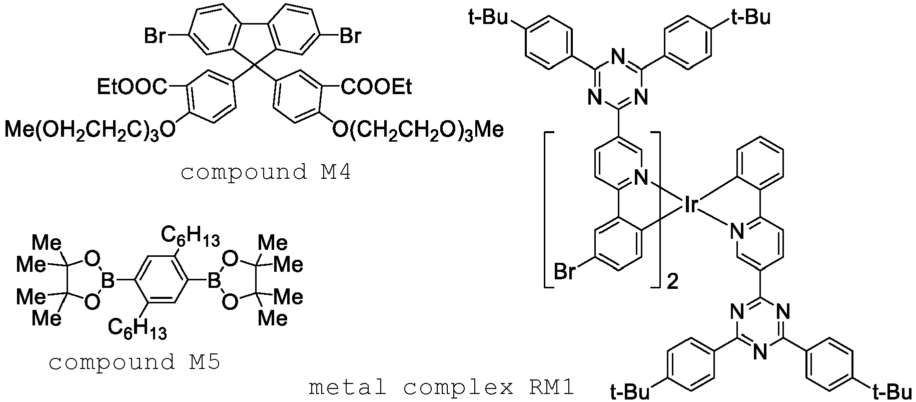

### <Synthesis Example HTL1> Synthesis of polymer compound HTL-1

An inert gas atmosphere was prepared in a reaction vessel, then, the compound M1 (0.800 g), the compound M2 (0.149 g), the compound M3 (1.66 g), dichlorobis(tris-o-methoxyphenylphosphine)palladium (1.4 mg) and toluene (45 mL) were added, and the mixture was heated at 100°C. Thereafter, a 20% by mass tetraethylammonium hydroxide aqueous solution (16 mL) was dropped into this, and the solution was refluxed for 7 hours. Thereafter, to this were added 2-ethylphenylboronic acid (90 mg) and dichlorobis(tris-o-methoxyphenylphosphine)palladium (1.3 mg), and the solution was refluxed for 17.5 hours. Thereafter, to this was added a sodium diethyldithiacarbamate aqueous solution, and the mixture was stirred at 85°C for 2 hours. The resultant reaction liquid was cooled, then, washed with 3.6% by mass hydrochloric acid, 2.5% by mass ammonia water and water, respectively. The resultant solution was dropped into methanol, to generate a precipitate. The resultant precipitate was dissolved in toluene, and purified by passing through an alumina column and a silica gel column in this order. The resultant solution was dropped into methanol, and the mixture was stirred, then, the resultant precipitate was collected by filtration, and dried, to obtain 1.64 g of a polymer compound HTL-1. The polymer compound HTL-1 had an Mn of 3.5×10⁴ and an Mw of 2.2×10⁵.

The polymer compound HTL-1 is a copolymer constituted of a constitutional unit derived from the compound M1, a constitutional unit derived from the compound M2 and a constitutional unit derived from the compound M3 at a molar ratio of 40:10:50, according to the theoretical values calculated from the amounts of the charged raw materials.

### <Synthesis Example HTL2> Synthesis of polymer compound HTL-2

An inert gas atmosphere was prepared in a reaction vessel, then, the compound M1 (2.52 g), the compound M2 (0.470 g), the compound M3 (4.90 g), the metal complex RM1 (0.530 g), dichlorobis(tris-o-methoxyphenylphosphine)palladium (4.2 mg) and toluene (158 mL) were added, and the mixture was heated at 100°C. Thereafter, a 20% by mass tetraethylammonium hydroxide aqueous solution (16 mL) was dropped into this, and the solution was refluxed for 8 hours. Thereafter, to this were added phenylboronic acid (116 mg) and dichlorobis(tris-o-methoxyphenylphosphine)palladium (4.2 mg), and the solution was refluxed for 15 hours. Thereafter, to this was added a sodium diethyldithiacarbamate aqueous solution, and the mixture was stirred at 85°C for 2 hours. The resultant reaction liquid was cooled, then, washed with 3.6% by mass hydrochloric acid, 2.5% by mass ammonia water and water, respectively. The resultant solution was dropped into methanol, to generate a precipitate. The resultant precipitate was dissolved in toluene, and purified by passing through an alumina column and a silica gel column in this order. The resultant solution was dropped into methanol, and the mixture was stirred, then, the resultant precipitate was collected by filtration, and dried, to obtain 6.02 g of a polymer compound HTL-2. The polymer compound HTL-2 had an Mn of 3.8×10⁴ and an Mw of 4.5×10⁵.

The polymer compound HTL-2 is a copolymer constituted of a constitutional unit derived from the compound M1, a constitutional unit derived from the compound M2, a constitutional unit derived from the compound M3 and a constitutional unit derived from the metal complex RM1 at a molar ratio of 40:10:47:3, according to the theoretical values calculated from the amounts of the charged raw materials.

### <Synthesis Examples B1 to B3 and B8> Synthesis and acquisition of phosphorescent compounds B1 to B3 and B8

A phosphorescent compound B1 (FIrpic) was purchased from Aldrich.

A phosphorescent compound B2 was synthesized according to a method described in JP-A No. 2013-147551.

A phosphorescent compound B3 was synthesized with reference to a method described in International Publication WO 2016/185183.

A phosphorescent compound B8 was synthesized with reference to a method described in International Publication WO 2006/121811.

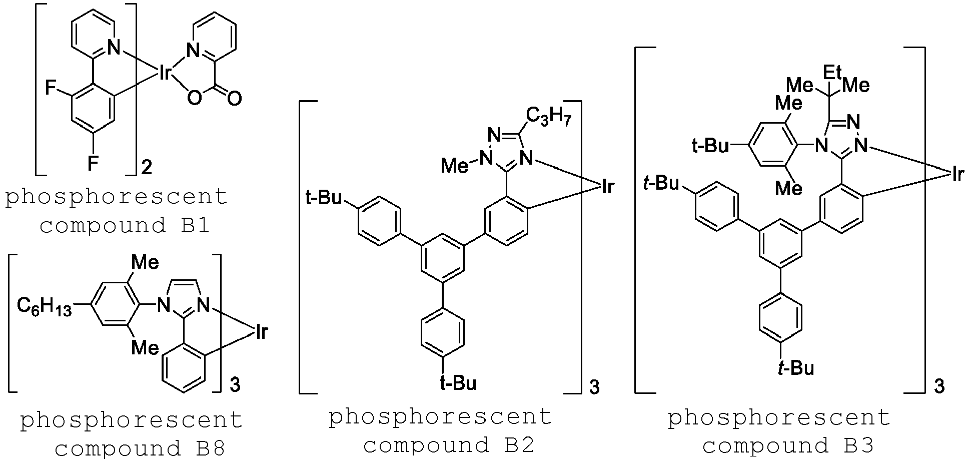

### <Synthesis Example B4> Synthesis of phosphorescent compound B4

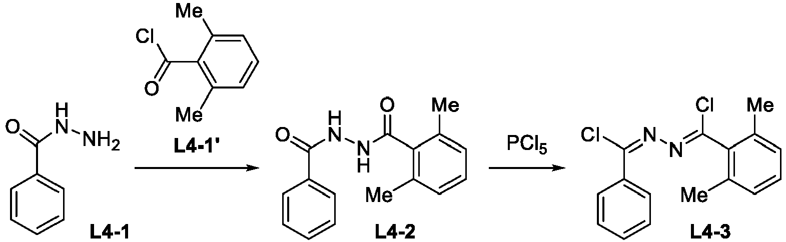

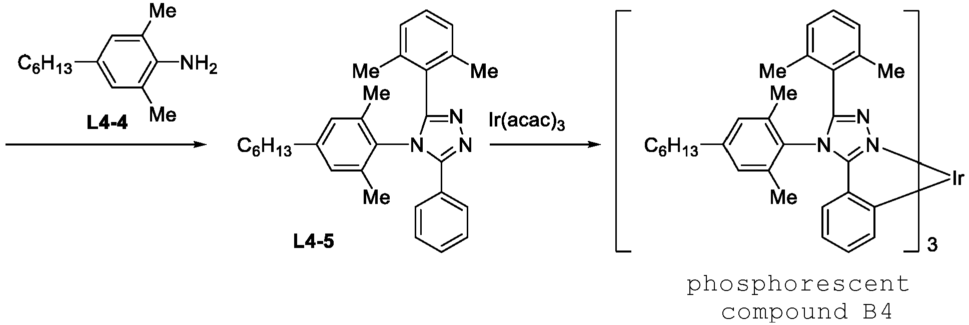

### (Synthesis of compound L4-2)

A nitrogen gas atmosphere was prepared in a reaction vessel, then, a compound L4-1 (50 g) and N-methyl-2-pyrrolidone (200 mL) were added, and the mixture was stirred at 0°C. Thereafter, a compound L4-1' (40 g) dissolved in N-methyl-2-pyrrolidone (40 mL) was dropped into this, and the solution was stirred at room temperature for 18 hours. The resultant reaction liquid was poured into ion exchanged water (1.2 L), to generate a precipitate. The resultant precipitate was collected by filtration, then, washed with a 1 M hydrochloric acid aqueous solution, ion exchanged water and heptane in series. The resultant solid was dried under reduced pressure, to obtain a compound L4-2 (43 g, white solid).

The analysis results of the compound L4-2 were as described below.

¹H-NMR (600 MHz, CDCl₃): δ (ppm) = 9.64 (br, 1H), 8.90 (br, 1H), 7.86 (d, 2H), 7.56 (t, 1H), 7.45 (t, 2H), 7.02-7.08 (m, 3H), 2.41 (s, 6H).

### (Synthesis of compound L4-3)

A nitrogen gas atmosphere was prepared in a reaction vessel, then, the compound L4-2 (43 g) and toluene (740 mL) were added, and the mixture was stirred at room temperature. Thereafter, to this was added phosphorus pentachloride (67 g), then, the mixture was stirred at 110°C for 21 hours. The resultant reaction liquid was cooled down to room temperature, then, poured into ice water (500 mL), the mixture was stirred for 2 hours, then, an aqueous layer was removed. The resultant organic layer was washed with ion exchanged water and a 10% by mass sodium hydrogen carbonate aqueous solution, respectively. The resultant organic layer was dried over magnesium sulfate, then, filtrated. The resultant filtrate was concentrated under reduced pressure, to obtain a compound L4-3 (40 g).

### (Synthesis of compound L4-5)

A nitrogen gas atmosphere was prepared in a reaction vessel, then, the compound L4-3 (40 g), compound L4-4 (32 g) and xylene (800 mL) were added, and the mixture was stirred at room temperature. Thereafter, to this was added p-toluenesulfonic acid (3 g), and the mixture was stirred at 120°C for 116 hours. The resultant reaction liquid was cooled down to room temperature, then, ion exchanged water (800 mL) was added, and the mixture was stirred at room temperature for 1 hour. An aqueous layer was removed from the resultant reaction liquid, then, the resultant organic layer was washed with a 5% by mass sodium hydrogen carbonate aqueous solution. The resultant organic layer was dried over magnesium sulfate, then, filtrated. The resultant filtrate was concentrated under reduced pressure, to obtain a coarse product. The resultant coarse product was purified by silica gel column chromatography (using a mixed solvent of heptane and ethyl acetate) and silica gel column chromatography (using acetonitrile and tetrahydrofuran) in series, to obtain a compound L4-5 (1.3 g, white solid). The HPLC area percentage value of the compound L4-5 was 99.5% or more. The above-described operation was conducted repeatedly, to obtain a necessary amount of the compound L4-5.

The analysis results of the compound L4-5 were as described below.

¹H-NMR (600 MHz, THF-d₈): δ (ppm) = 7.42 (d, 2H), 7.30 (t, 1H), 7.24 (t, 2H), 7.15 (t, 1H), 6.98 (d, 2H), 6.85 (s, 2H), 2.51 (t, 2H), 2.07 (s, 6H), 1.81 (s, 6H), 1.56 (m, 2H), 1.26-1.32 (m, 6H), 0.88 (t, 3H).

### (Synthesis of phosphorescent compound B4)

A nitrogen gas atmosphere was prepared in a reaction vessel, then, tris(acetylacetonato)iridium(III) (0.6 g), the compound L4-5 (2.0 g) and tridecane (2 mL) were added, and the mixture was stirred at 250°C for 120 hours. The resultant reaction liquid was cooled down to room temperature, then, purified by silica gel column chromatography (using a mixed solvent of heptane and ethyl acetate), then, crystallized using a mixed solvent of methylene chloride and acetonitrile. The resultant solid was dried under reduced pressure, to obtain a phosphorescent compound B4 (0.6 g, yellow solid). The HPLC area percentage value of the phosphorescent compound B4 was 99.2%.

The analysis results of the phosphorescent compound B4 were as described below.

¹H-NMR (600 MHz, THF-d₈): δ (ppm) = 7.04-7.08 (m, 6H), 6.93 (s, 3H), 6.92 (s, 3H), 6.88 (d, 3H), 6.84 (d, 3H), 6.61 (t, 3H), 6.43 (t, 3H), 6.29 (d, 3H), 2.57 (t, 6H), 2.12 (s, 9H), 1.95 (s, 9H), 1.82 (s, 9H), 1.70 (s, 9H), 1.62 (m, 6H), 1.28-1.36 (m, 18H), 0.89 (t, 9H).

### <Synthesis Example B5> Synthesis of phosphorescent compound B5

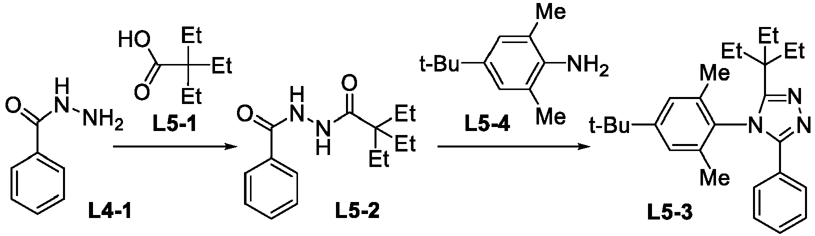

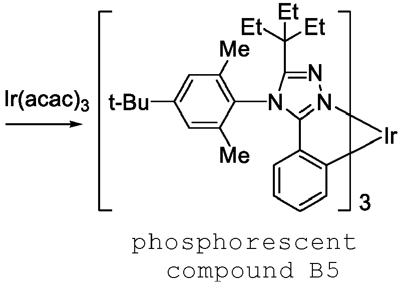

### (Synthesis of reaction mixture L5-1')

A nitrogen gas atmosphere was prepared in a reaction vessel, then, the compound L5-1 (50 g) and thionyl chloride (100 mL) were added, and the mixture was stirred for 3 hours under reflux. The resultant reaction mixture was cooled down to room temperature, then, thionyl chloride was distilled off under reduced pressure, to obtain a reaction mixture L5-1'.

### (Synthesis of compound L5-2)

A nitrogen gas atmosphere was prepared in a reaction vessel, then, the compound L4-1 (47 g) and tetrahydrofuran (1 L) were added, and the mixture was cooled to 0°C. Thereafter, to this was added triethylamine (54 mL), and the mixture was stirred at 0°C for 45 minutes. Thereafter, to this was added the reaction mixture L5-1' (total amount) obtained in (Synthesis of reaction mixture L5-1'), and the mixture was stirred at room temperature for 16 hours. The resultant reaction liquid was filtrated, then, the resultant filtrate was concentrated under reduced pressure, to obtain a coarse product. The resultant coarse product was washed with a mixed solvent of ethyl acetate and hexane, then, dried under reduced pressure, to obtain a compound L5-2 (50 g). The HPLC area percentage value of the compound L5-2 was 95.2%. The above-described operation was conducted repeatedly, to obtain a necessary amount of the compound L5-2.

The analysis results of the compound L5-2 were as described below.

LC-MS (APCI, positive): m/z = 263 [M+H]⁺

¹H-NMR (300 MHz, CDCl₃): δ (ppm) = 0.84 (t, 9H), 1.64 (q, 6H), 7.39-7.54 (m, 3H), 7.81-7.84 (m, 2H), 8.72-8.74 (m, 1H), 9.66-9.68 (m, 1H).

### (Synthesis of compound L5-3)

A nitrogen gas atmosphere was prepared in a reaction vessel, then, the compound L5-2 (58 g) and toluene (600 mL) were added, and the mixture was stirred at room temperature. Thereafter, to this was added phosphorus pentachloride (92 g), then, the mixture was stirred at 110°C for 3 hours. The resultant reaction liquid was cooled down to room temperature, then, a compound L5-4 (78.2 g) and p-toluenesulfonic acid (3 g) were added, and the mixture was stirred at 130°C for 4 days. The resultant reaction liquid was cooled down to room temperature, concentrated under reduced pressure, then, ethyl acetate (2 L) was added, and the liquid was washed with a 10% by mass sodium hydrogen carbonate aqueous solution. The resultant organic layer was dried over magnesium sulfate, then, filtrated, and the resultant filtrate was concentrated under reduced pressure, to obtain a coarse product. The resultant coarse product was purified by silica gel column chromatography (using a mixed solvent of methanol and chloroform), then, crystallized using acetonitrile, then, dried under reduced pressure, to obtain a compound L5-3 (6 g). The HPLC area percentage value of the compound L5-3 was 99.1%.

The analysis results of the compound L5-3 were as described below.

LC-MS (APCI, positive): m/z = 404 [M+H]⁺

¹H-NMR (400 MHz, CDCl₃): δ (ppm) = 0.83 (t, 9H), 1.34 (s, 9H), 1.64 (q, 6H), 1.96 (s, 6H), 7.12 (s, 2H), 7.20-7.23 (m, 2H), 7.28-7.34 (m, 3H).

### (Synthesis of phosphorescent compound B5)

A nitrogen gas atmosphere was prepared in a reaction vessel, then, tris(acetylacetonato)iridium(III) (1.4 g), the compound L5-3 (4.6 g) and pentadecane (2 mL) were added, and the mixture was stirred at 300°C for 18 hours. The resultant reaction liquid was cooled down to room temperature, dissolved in toluene, then, concentrated under reduced pressure, to obtain a coarse product. The resultant coarse product was purified by silica gel column chromatography (using a mixed solvent of heptane and ethyl acetate), then, crystallized using a mixed solvent of acetonitrile and toluene. The resultant solid was dried under reduced pressure, to obtain a phosphorescent compound B5 (2.8 g). The HPLC area percentage value of the phosphorescent compound B5 was 99.5% or more.

The analysis results of the phosphorescent compound B5 were as described below.

¹H-NMR (600 MHz, THF-d₈): δ (ppm) = 7.30 (s, 6H), 6.90 (d, 3H), 6.44-6.48 (m, 3H), 6.22-6.26 (m, 3H), 5.77 (d, 3H), 2.10 (s, 9H), 1.89 (s, 9H), 1.56 (s, 18H), 1.38 (s, 27H), 0.73 (t, 27H).

### <Synthesis Example B6> Synthesis of phosphorescent compound B6

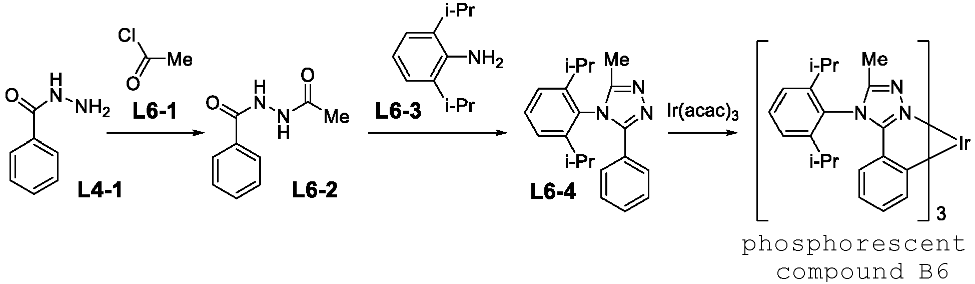

### (Synthesis of compound L6-2)

A nitrogen gas atmosphere was prepared in a reaction vessel, then, the compound L4-1 (100 g), triethylamine (114 mL) and tetrahydrofuran (1.5L) were added, and the mixture was stirred at 0°C. Thereafter, a compound L6-1 (52 mL) was dropped into this, then, the mixture was stirred at room temperature for 16 hours. The resultant reaction liquid was filtrated, then, the resultant filtrate was concentrated, to obtain a coarse product. The resultant coarse product was crystallized using ethyl acetate, then, dried under reduced pressure, to obtain a compound L6-2 (70 g). The HPLC area percentage value of the compound L6-2 was 98.7%.

The analysis results of the compound L6-2 were as described below.

LC-MS (APCI, positive): m/z = 179 [M+H]⁺

¹H-NMR (300 MHz, DMSO-d₆): δ (ppm) = 10.26 (br, 1H), 9.86 (br, 1H), 7.83-7.86 (m, 2H), 7.45-7.56 (m, 3H), 1.90 (s, 3H).

### (Synthesis of compound L6-4)

A nitrogen gas atmosphere was prepared in a reaction vessel, then, the compound L6-2 (70 g) and xylene (700 mL) were added, and the mixture was stirred at room temperature. Thereafter, to this was added phosphorus pentachloride (123 g), and the mixture was stirred at 130°C for 2 hours. The resultant reaction liquid was cooled down to room temperature, a compound L6-3 (70 g) was added, then, the mixture was stirred at 130°C for 8 hours. The resultant reaction liquid was cooled down to room temperature, concentrated under reduced pressure, then, ethyl acetate was added. The resultant organic layer was washed with ion exchanged water, a 10% by mass sodium hydrogen carbonate aqueous solution and saturated saline in series. The resultant organic layer was dried over sodium sulfate, then, filtrated, and the resultant filtrate was concentrated under reduced pressure, to obtain a coarse product. The resultant coarse product was purified by silica gel column chromatography (using a mixed solvent of hexane and ethyl acetate), then, crystallized using a mixed solvent of N,N-dimethylformamide and water. The resultant solid was dried under reduced pressure, to obtain a compound L6-4 (70 g, white solid). The HPLC area percentage value of the compound L6-4 was 99.2%.

The analysis results of the compound L6-4 were as described below.

LC-MS (APCI, positive): m/z = 320 [M+H]⁺

¹H-NMR (400 MHz, CDCl₃): δ (ppm) = 7.53-7.58 (m, 1H), 7.48 (d, 2H), 7.33 (d, 2H), 7.28-7.30 (m, 1H), 7.21-7.25 (m, 2H), 2.39 (q,2H), 2.26 (s, 3H), 1.14 (d,6H), 0.87 (d,6H).

### (Synthesis of phosphorescent compound B6)

A nitrogen gas atmosphere was prepared in a reaction vessel, then, tris(acetylacetonato)iridium(III) (1.2 g), the compound L6-4 (4.0 g) and tridecane (1 mL) were added, and the mixture was stirred at 280°C for 18 hours. The resultant reaction liquid was cooled down to room temperature, then, purified by silica gel column chromatography (using a mixed solvent of ethyl acetate and methanol), then, crystallized using a mixed solvent of toluene and acetonitrile. The resultant solid was dried under reduced pressure, to obtain a phosphorescent compound B6 (1.7g, yellow solid). The HPLC area percentage value of the phosphorescent compound B6 was 99.5% or more.

The analysis results of the phosphorescent compound B6 were as described below.

¹H-NMR (600 MHz, THF-d₈): δ (ppm) = 7.56 (t, 3H), 7.42 (dd, 3H), 7.40 (dd, 3H), 6.87 (dd, 3H), 6.52 (td, 3H), 6.35 (td, 3H), 6.17 (dd, 3H), 2.83 (hept, 3H), 2.34 (hept, 3H), 2.10 (s, 9H), 1.23 (d, 9H), 0.98 (d, 9H), 0.96 (d, 9H), 0.92 (d, 9H).

### <Synthesis Example B7> Synthesis of phosphorescent compound B7

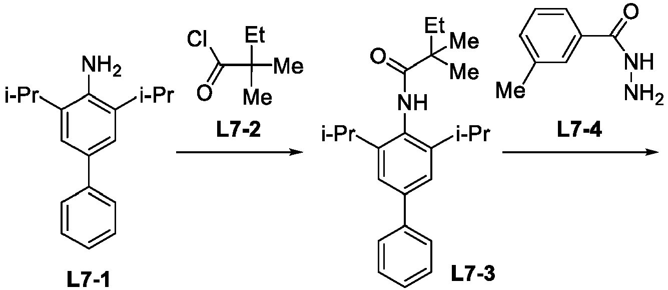

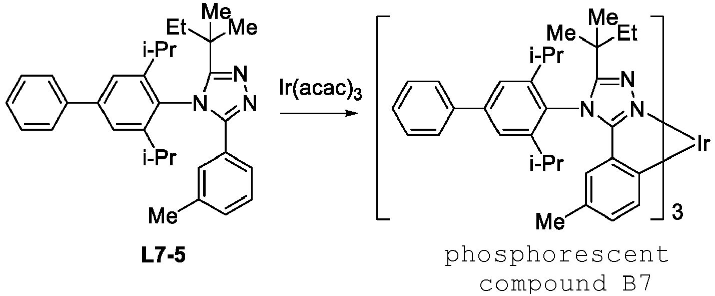

### (Synthesis of compound L7-3)

A nitrogen gas atmosphere was prepared in a reaction vessel, then, a compound L7-1 (21.4 g), triethylamine (13.0 mL) and tetrahydrofuran (300 mL) were added, and the mixture was cooled to 0°C. Thereafter, a compound L7-2 (12.8 mL) was dropped into this, and the mixture was stirred at room temperature for 16 hours. Thereafter, to this was added ion exchanged water (100 mL), to generate a precipitate. The reaction liquid containing the resultant precipitate was filtrated, to obtain a residue L7-3-1 and a filtrate L7-3-2.

The resultant residue L7-3-1 was washed with toluene, then, dried under reduced pressure, to obtain a solid L7-3' (24.5 g).

An aqueous layer was removed from the resultant filtrate L7-3-2, and the resultant organic layer was dried over magnesium sulfate, then, filtrated. The resultant filtrate was concentrated under reduced pressure, then, crystallized with a mixed solvent of toluene and heptane. The resultant solid was dried under reduced pressure, to obtain a solid L7-3" (3.9 g) .

The resultant solid L7-3' and the solid L7-3" were combined, then, crystallized using a mixed solvent of toluene and heptane. The resultant solid was dried under reduced pressure, to obtain a compound L7-3 (27.8 g, white solid). The HPLC area percentage value of the compound L7-3 was 98.9%.

The analysis results of the compound L7-3 were as described below.

¹H-NMR (600 MHz, THF-d₈): δ (ppm) = 7.57 (d, 2H), 7.43 (t, 2H), 7.35 (s, 2H), 7.34 (t, 1H), 6.82 (brs, 1H), 3.08 (septet, 2H), 1.73 (q, 2H), 1.34 (s, 6H), 1.25 (d, 12H), 1.00 (t, 3H).

### (Synthesis of compound L7-5)

A compound L7-5 was synthesized with reference to a method described in Organic Letters, vol. 17, pp. 1184-1187, 2015 using the compound L7-3 (19.1 g), the compound L7-4 (9.0 g), chlorobenzene (150 mL), 2-fluoropyridine (5.15 mL) and trifluoromethanesulfonic anhydride (10.0 mL).

The analysis results of the compound L7-5 were as described below.

¹H-NMR (600 MHz, CDCl₃): δ (ppm) = 7.68 (d, 2H), 7.52 (t, 2H), 7.50 (s, 2H), 7.43 (t, 1H), 7.26 (s, 1H), 7.05-6.98 (m, 3H), 2.53 (septet, 2H), 2.15 (s, 3H), 1.88 (q, 2H), 1.28 (d, 6H), 1.23 (s, 6H), 0.89 (t, 3H), 0.77 (d, 6H).

### (Synthesis of phosphorescent compound B7)

A nitrogen gas atmosphere was prepared in a reaction vessel, then, tris(acetylacetonato)iridium(III)(0.72 g), the compound L7-5 (2.8 g) and pentadecane (2 mL) were added, and the mixture was stirred at 300°C for 24 hours. The resultant reaction liquid was cooled down to room temperature, then, purified by silica gel column chromatography (using a mixed solvent of methylene chloride and ethyl acetate), and subsequently, crystallized in series using a mixed solvent of acetonitrile and toluene, a mixed solvent of toluene and methanol, and a mixed solvent of methylene chloride and acetonitrile. The resultant solid was washed with methylene chloride, then, dried under reduced pressure, to obtain a phosphorescent compound B7 (0.82 g). The HPLC area percentage value of the phosphorescent compound B7 was 98.8%.

The analysis results of the phosphorescent compound B7 were as described below.

¹H-NMR (600 MHz, THF-d₈): δ (ppm) = 7.74 (d, 6H), 7.64 (dd, 6H), 7.48 (t, 6H), 7.38 (t, 3H), 6.68 (d, 3H), 6.30 (d, 3H), 5.62 (s, 3H), 2.94 (septet, 3H), 2.37 (septet, 3H), 1.75 (s, 9H), 1.71-1.64 (m, 6H), 1.34 (d, 9H), 1.22-1.17 (m, 27H), 0.98 (d, 9H), 0.90 (d, 9H), 0.81 (d, 9H).

### <Synthesis Example G1, G2 and R1> Synthesis of phosphorescent compounds G1, G2 and R1

A phosphorescent compound G1 was synthesized with reference to a method described in International Publication WO 2009/131255.

A phosphorescent compound G2 was synthesized according to a method described in JP-A No. 2014-224101.

A phosphorescent compound R1 was synthesized with reference to a method described in JP-A No. 2006-188673.

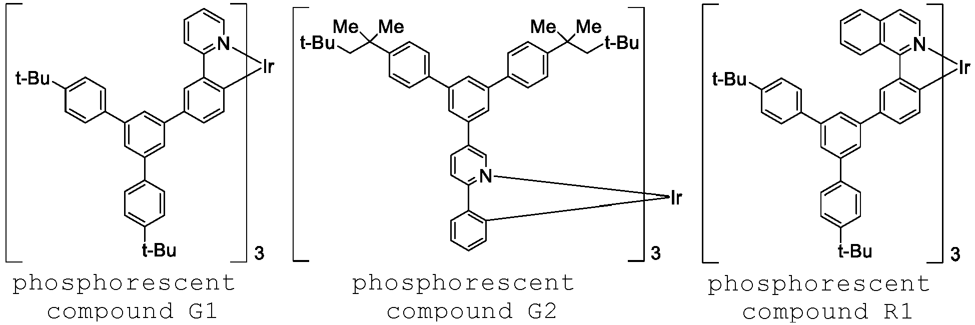

### <Synthesis Example HM-1, HM-5 to HM-7 and HM-9>

Synthesis and acquisition of compounds HM-1, HM-5 to HM-7 and HM-9

A compound HM-1 was purchased from Luminescence Technology Corp.

A compound HM-5 was synthesized with reference to a method described in International Publication WO 2014/023388.

A compound HM-6 and a compound HM-7 were synthesized with reference to a method described in International Publication WO 2012/048820.

A compound HM-9 was synthesized with reference to a method described in International Publication WO 2013/045411.

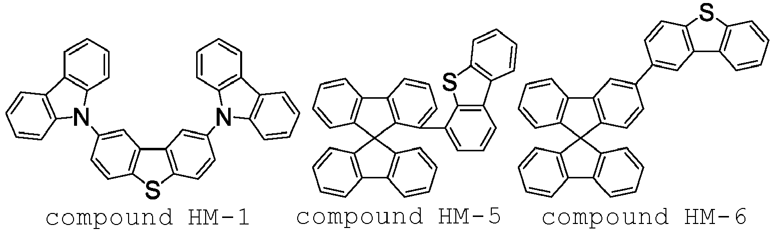

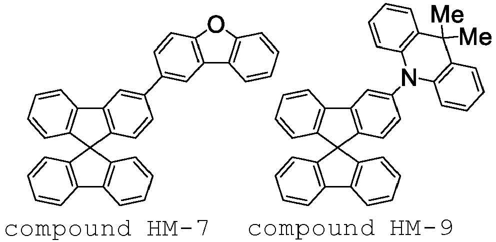

### <Synthesis Example HM-2> Synthesis of compound HM-2

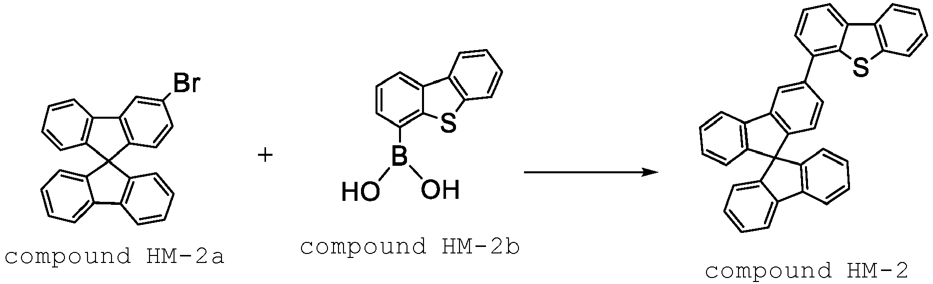

A nitrogen gas atmosphere was prepared in a reaction vessel, then, a compound HM-2a (15.6 g), a compound HM-2b (10.3 g), toluene (390 mL), tetrakis(triphenylphosphine)palladium(0) (2.2 g) and a 20% by mass tetrabutylammonium hydroxide aqueous solution (194 g) were added, and the mixture was stirred at 90°C for 4 hours. The resultant reaction liquid was cooled down to room temperature, then, filtrated through a filter paved with Celite. The resultant filtrate was washed with ion exchanged water, then, the resultant organic layer was dried over anhydrous sodium sulfate, and filtrated. The resultant filtrate was concentrated under reduced pressure, to obtain a solid. The resultant solid was crystallized using a mixed solvent of toluene and 2-propanol, then, dried at 50°C under reduced pressure, to obtain a compound HM-2 (15.2 g). The HPLC area percentage value of the compound HM-2 was 99.5% or more.

The analysis results of the compound HM-2 were as described below.

¹H-NMR (CD₂Cl₂, 400 MHz): δ (ppm) = 6.70-6.83 (4H, m), 7.15 (3H, t), 7.39 (3H, t), 7.48 (3H, t), 7.59 (2H, t), 7.83-7.93 (4H, m), 8.18-8.23 (3H, m).

### <Synthesis Example HM-3> Synthesis of compound HM-3

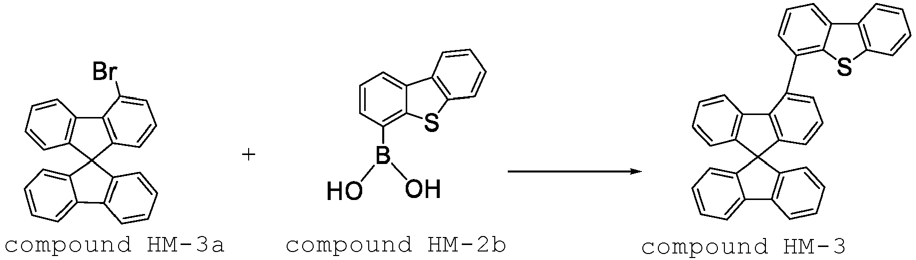

A nitrogen gas atmosphere was prepared in a reaction vessel, then, a compound HM-3a (13.5 g), a compound HM-2b (8.9 g), toluene (404 mL), tetrakis(triphenylphosphine)palladium(0) (2.0 g) and a 20% by mass tetrabutylammonium hydroxide aqueous solution (166 g) were added, and the mixture was stirred at 90°C for 3 hours. The resultant reaction liquid was cooled down to room temperature, then, filtrated through a filter paved with Celite. The resultant filtrate was washed with ion exchanged water, then, the resultant organic layer was dried over anhydrous sodium sulfate, and filtrated. The resultant filtrate was concentrated under reduced pressure, to obtain a solid. The resultant solid was purified by silica gel column chromatography (using a mixed solvent of hexane and chloroform), and further, crystallized using a mixed solvent of toluene and methanol, then, dried at 50°C under reduced pressure, to obtain a compound HM-3 (10.5 g). The HPLC area percentage value of the compound HM-3 was 99.5% or more.

The analysis results of the compound HM-3 were as described below.

¹H-NMR (CD₂Cl₂, 400 MHz): δ (ppm) = 6.51 (1H, d), 6.60 (1H, d), 6.80 (4H, m), 6.92 (1H, t), 7.21 (3H, m), 7.34 (1H, d), 7.39-7.50 (4H, m), 7.65 (1H, d), 7.71 (1H, t), 7.81 (1H, d), 7.88 (2H, d), 8.28-8.35 (2H, m).

### <Synthesis Example HM-4> Synthesis of compound HM-4

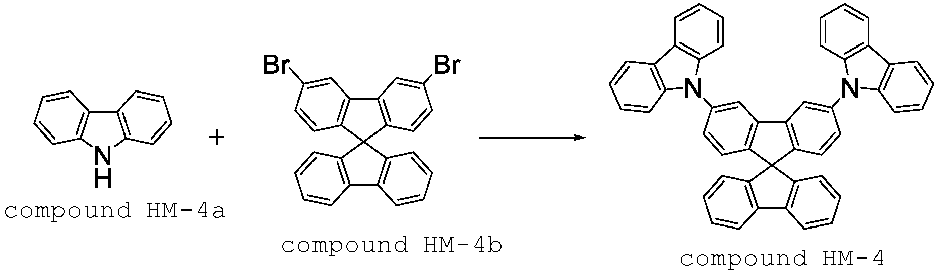

A nitrogen gas atmosphere was prepared in a reaction vessel, then, a compound HM-4a (1.6 g), a compound HM-4b (1.3 g), xylene (63 mL), palladium(II) acetate (22 mg), tri-tert-butylphosphonium tetrafluoroborate (63 mg) and sodium tert-butoxide (1.9 g) were added, and the mixture was stirred for 54 hours under reflux with heat. The resultant reaction liquid was cooled down to room temperature, then, filtrated through a filter paved with silica gel and Celite. The resultant filtrate was washed with ion exchanged water, then, the resultant organic layer was dried over anhydrous sodium sulfate, and filtrated. The resultant filtrate was concentrated under reduced pressure, to obtain a solid. The resultant solid was purified by silica gel column chromatography (using a mixed solvent of hexane and chloroform), and further, crystallized using a mixed solvent of chloroform and 2-propanol, then, dried at 50°C under reduced pressure, to obtain a compound HM-4 (1.0 g). The HPLC area percentage value of the compound HM-4 was 99.5% or more.

The analysis results of the compound HM-4 were as described below.

¹H-NMR (CD₂Cl₂, 400 MHz): δ (ppm) = 7.08 (4H, t), 7.34 (6H, m), 7.47-7.57 (12H, m), 8.02 (2H, d), 8.12 (2H, s), 8.22 (4H, d).

### <Synthesis Example HM-8> Synthesis of compound HM-8

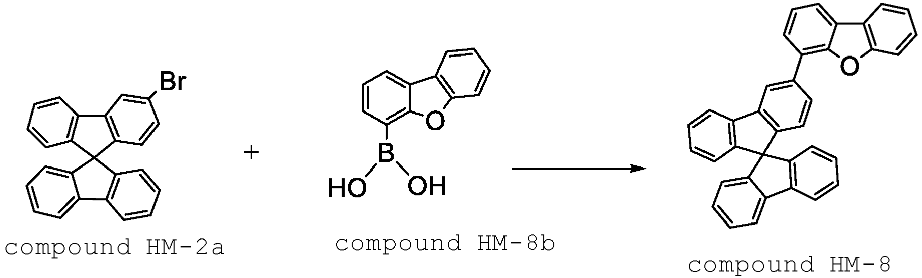

A nitrogen gas atmosphere was prepared in a reaction vessel, then, a compound HM-2a (1.64 g), a compound HM-8b (1.00 g), toluene (40 mL), tetrakis(triphenylphosphine)palladium(0) (0.24 g) and a 20% by mass tetrabutylammonium hydroxide aqueous solution (20 g) were added, and the mixture was stirred at 90°C for 3 hours. The resultant reaction liquid was cooled down to room temperature, then, toluene was added, and the liquid was washed with ion exchanged water. The resultant organic layer was dried over anhydrous magnesium sulfate, then, filtrated through a filter paved with silica gel and Celite. The resultant filtrate was concentrated under reduced pressure, to obtain a solid. The resultant solid was crystallized using a mixed solvent of toluene and 2-propanol, then, dried at 50°C under reduced pressure, to obtain a compound HM-8 (1.7 g). The HPLC area percentage value of the compound HM-8 was 99.5% or more.

The analysis results of the compound HM-8 were as described below.

¹H-NMR (CDCl₃, 400 MHz): δ (ppm) = 8.36 (d, 1H), 8.03-7.99 (m, 1H), 7.98-7.93 (m, 2H), 7.89-7.86 (m, 2H), 7.70-7.60 (m, 3H), 7.51-7.35 (m, 6H), 7.17-7.12 (m, 3H), 6.89 (d, 1H), 6.86-6.82 (m, 2H), 6.78 (d, 1H).

### <Synthesis Example ETL1> Synthesis of polymer compound ETL-1

An inert gas atmosphere was prepared in a reaction vessel, then, the compound M4 (9.23 g), the compound M5 (4.58 g), dichlorobis(tris-o-methoxyphenylphosphine)palladium (8.6 mg), methyltrioctylammonium chloride (trade name: Aliquat336 (registered trademark) manufactured by Sigma Aldrich)(0.098 g) and toluene (175 mL) were added, and the mixture was heated at 105°C. Thereafter, a 12% by mass sodium carbonate aqueous solution (40.3 mL) was dropped into this, and the solution was refluxed for 29 hours. Thereafter, to this were added phenylboronic acid (0.47 g) and dichlorobis(tris-o-methoxyphenylphosphine)palladium (8.7 mg), and the solution was refluxed for 14 hours. Thereafter, to this was added a sodium diethyldithiacarbamate aqueous solution, and the mixture was stirred at 80°C for 2 hours. The resultant reaction liquid was cooled, then, dropped into methanol, to generate a precipitate. The resultant precipitate was collected by filtration, and washed with methanol and water, respectively, then, dried. The resultant solid was dissolved in chloroform, and purified by sequentially passing through an alumina column and a silica gel column through which chloroform had been passed previously. The resultant purified liquid was dropped into methanol, and the liquid was stirred, to generate a precipitate. The resultant precipitate was collected by filtration, and dried, to obtain a polymer compound ETL-1a (7.15 g). The polymer compound ETL-1a had an Mn of 3.2×10⁴ and an Mw of 6.0×10⁴.

The polymer compound ETL-1a is a copolymer constituted of a constitutional unit derived from the compound M4 and a constitutional unit derived from the compound M5 at a molar ratio of 50:50, according to the theoretical values calculated from the amounts of the charged raw materials.

An argon gas atmosphere was prepared in a reaction vessel, then, the polymer compound ETL-1a (3.1 g), tetrahydrofuran (130 mL), methanol (66 mL), cesium hydroxide monohydrate (2.1 g) and water (12.5 mL) were added, and the mixture was stirred at 60°C for 3 hours. Thereafter, to this was added methanol (220 mL), and the mixture was stirred for 2 hours. The resultant reaction mixture was concentrated, then, dropped into isopropyl alcohol, and the liquid was stirred, to generate a precipitate. The resultant precipitate was collected by filtration, and dried, to obtain a polymer compound ETL-1 (3.5 g). By ¹H-NMR analysis of the polymer compound ETL-1, it was confirmed that a signal of an ethyl ester site in the polymer compound ETL-1 disappeared and reaction was completed.

The polymer compound ETL-1 is a copolymer constituted of a constitutional unit represented by the following formula and a constitutional unit derived from the compound M5 at a molar ratio of 50:50, according to the theoretical values calculated from the amounts of the charged raw materials of the polymer compound ETL-1a.

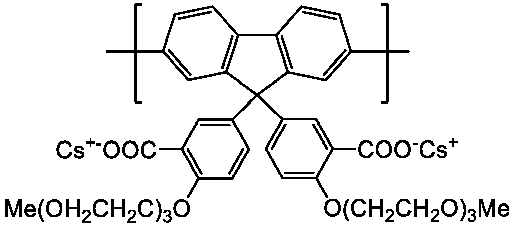

### <Example D1> Fabrication and evaluation of light emitting device D1

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated to form a film with a thickness of 35 nm. In an air atmosphere, the film was heated on a hot plate at 50°C for 3 minutes, further heated at 230°C for 15 minutes, to form a hole injection layer.

### (Formation of hole transporting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer.

### (Formation of light emitting layer)

The compound HM-3 and the phosphorescent compound B4 (compound HM-3/phosphorescent compound B4 = 75% by mass/25% by mass) were dissolved in toluene at a concentration of 2.0% by mass. The resultant toluene solution was spin-coated on the hole transporting layer, to form a film with a thickness of 75 nm, and the film was heated at 130°C for 10 minutes under a nitrogen gas atmosphere, to form a light emitting layer.

### (Formation of electron transporting layer)

The polymer compound ETL-1 was dissolved in 2,2,3,3,4,4,5,5-octafluoro-1-pentanol at a concentration of 0.25% by mass. The resultant 2,2,3,3,4,4,5,5-octafluoro-1-pentanol solution was spin-coated on the light emitting layer, to form a film with a thickness of 10 nm, and the film was heated at 130°C for 10 minutes under a nitrogen gas atmosphere, to form an electron transporting layer.

### (Formation of cathode)

The substrate carrying the electron transporting layer formed thereon was placed in a vapor deposition machine and the inner pressure thereof was reduced to 1.0×10⁻⁴ Pa or less, then, as cathode, sodium fluoride was vapor-deposited with a thickness of about 4 nm on the electron transporting layer, then, aluminum was vapor-deposited with a thickness of about 80 nm on the sodium fluoride layer. After vapor deposition, sealing was performed with a glass substrate, to fabricate a light emitting device D1.

### (Evaluation of light emitting device)

Voltage was applied to the light emitting device D1, to observe EL light emission. The light emission efficiency [cd/A] at 200 cd/m² was measured. The CIE chromaticity coordinate (x, y) at 200 cd/m² was (0.19, 0.41) .

### <Example D2> Fabrication and evaluation of light emitting device D2

A light emitting device D2 was fabricated in the same manner as in Example D1, except that "the phosphorescent compound B2" was used instead of "the phosphorescent compound B4" in (Formation of light emitting layer) in Example D1.

Voltage was applied to the light emitting device D2, to observe EL light emission. The light emission efficiency [cd/A] at 200 cd/m² was measured. The CIE chromaticity coordinate (x, y) at 200 cd/m² was (0.16, 0.35) .

### <Example D3> Fabrication and evaluation of light emitting device D3

A light emitting device D3 was fabricated in the same manner as in Example D1, except that "the phosphorescent compound B6" was used instead of "the phosphorescent compound B4" in (Formation of light emitting layer) in Example D1.

Voltage was applied to the light emitting device D3, to observe EL light emission. The light emission efficiency [cd/A] at 200 cd/m² was measured. The CIE chromaticity coordinate (x, y) at 200 cd/m² was (0.18, 0.36) .

### <Comparative Example CD1> Fabrication and evaluation of light emitting device CD1

A light emitting device CD1 was fabricated in the same manner as in Example D1, except that "the phosphorescent compound B1" was used instead of "the phosphorescent compound B4" in (Formation of light emitting layer) in Example D1.

Voltage was applied to the light emitting device CD1, to observe EL light emission. The light emission efficiency [cd/A] at 200 cd/m² was measured. The CIE chromaticity coordinate (x, y) at 200 cd/m² was (0.16, 0.35) .

The results of Examples D1 to D3 and Comparative Example CD1 are shown in Table 1. The relative values of the light emission efficiency of the light emitting devices D1 to D3 are shown when the light emission efficiency of the light emitting device CD1 is taken as 1.0.

**(Table 1)**

| | light emitting device | hole transporting layer | light emitting layer | | light emission efficiency (relative value) |
|---|---|---|---|---|---|
| | | material | material | composition ratio (% by mass) | |
| Example D1 | D1 | HTL-1 | HM-3/B4 | 75/25 | 64.3 |
| Example D2 | D2 | HTL-1 | HM-3/B2 | 75/25 | 30.3 |
| Example D3 | D3 | HTL-1 | HM-3/B6 | 75/25 | 37.1 |
| Comparative Example CD1 | CD1 | HTL-1 | HM-3/B1 | 75/25 | 1.0 |

### <Example D4> Fabrication and evaluation of light emitting device D4

A light emitting device D4 was fabricated in the same manner as in Example D1, except that "the compound HM-3, the compound HM-2 and the phosphorescent compound B2 (compound HM-3/compound HM-2/phosphorescent compound B2 = 37.5% by mass/37.5% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D4, to observe EL light emission. The light emission efficiency [cd/A] at 200 cd/m² was measured. The CIE chromaticity coordinate (x, y) at 200 cd/m² was (0.16, 0.36) .

### <Example D5> Fabrication and evaluation of light emitting device D5

A light emitting device D5 was fabricated in the same manner as in Example D4, except that "the compound HM-4" was used instead of "the compound HM-2" in (Formation of light emitting layer) in Example D4.

Voltage was applied to the light emitting device D5, to observe EL light emission. The light emission efficiency [cd/A] at 200 cd/m² was measured. The CIE chromaticity coordinate (x, y) at 200 cd/m² was (0.17, 0.37) .

### <Example D6> Fabrication and evaluation of light emitting device D6

A light emitting device D6 was fabricated in the same manner as in Example D4, except that "the phosphorescent compound B4" was used instead of "the phosphorescent compound B2" in (Formation of light emitting layer) in Example D4.

Voltage was applied to the light emitting device D6, to observe EL light emission. The light emission efficiency [cd/A] at 200 cd/m² was measured. The CIE chromaticity coordinate (x, y) at 200 cd/m² was (0.19, 0.39).

### <Example D7> Fabrication and evaluation of light emitting device D7

A light emitting device D7 was fabricated in the same manner as in Example D4, except that "the compound HM-3, the compound HM-1 and the phosphorescent compound B4 (compound HM-3/compound HM-1/phosphorescent compound B4 = 37.5% by mass/37.5% by mass/25% by mass)" were used instead of "the compound HM-3, the compound HM-2 and the phosphorescent compound B2 (compound HM-3/compound HM-2/phosphorescent compound B2 = 37.5% by mass/37.5% by mass/25% by mass)" in (Formation of light emitting layer) in Example D4.

Voltage was applied to the light emitting device D7, to observe EL light emission. The light emission efficiency [cd/A] at 200 cd/m² was measured. The CIE chromaticity coordinate (x, y) at 200 cd/m² was (0.17, 0.34) .

### <Example D8> Fabrication and evaluation of light emitting device D8

A light emitting device D8 was fabricated in the same manner as in Example D4, except that "the phosphorescent compound B6" was used instead of "the phosphorescent compound B2" in (Formation of light emitting layer) in Example D4.

Voltage was applied to the light emitting device D8, to observe EL light emission. The light emission efficiency [cd/A] at 200 cd/m² was measured. The CIE chromaticity coordinate (x, y) at 200 cd/m² was (0.19, 0.38) .

### <Comparative Example CD2> Fabrication and evaluation of light emitting device CD2

A light emitting device CD2 was fabricated in the same manner as in Example D4, except that "the phosphorescent compound B1" was used instead of "the phosphorescent compound B2" in (Formation of light emitting layer) in Example D4.

Voltage was applied to the light emitting device CD2, to observe EL light emission. The light emission efficiency [cd/A] at 200 cd/m² was measured. The CIE chromaticity coordinate (x, y) at 200 cd/m² was (0.16, 0.35) .

The results of Examples D4 to D8 and Comparative Example CD2 are shown in Table 2. The relative values of the light emission efficiency of the light emitting devices D4 to D8 are shown when the light emission efficiency of the light emitting device CD2 is taken as 1.0.

**(Table 2)**

| | light emitting device | hole transporting layer | light emitting layer | | light emission efficiency (relative value) |
|---|---|---|---|---|---|
| | | material | material | composition ratio (% by mass) | |
| Example D4 | D4 | HTL-1 | HM-3/HM-2/B2 | 37.5/37.5/25 | 12.9 |
| Example D5 | D5 | HTL-1 | HM-3/HM-4/B2 | 37.5/37.5/25 | 10.5 |
| Example D6 | D6 | HTL-1 | HM-3/HM-2/B4 | 37.5/37.5/25 | 22.4 |
| Example D7 | D7 | HTL-1 | HM-3/HM-1/B4 | 37.5/37.5/25 | 15.4 |
| Example D8 | D8 | HTL-1 | HM-3/HM-2/B6 | 37.5/37.5/25 | 15.5 |
| Comparative Example CD2 | CD2 | HTL-1 | HM-3/HM-2/B1 | 37.5/37.5/25 | 1.0 |

### <Example D9> Fabrication and evaluation of light emitting device D9

A light emitting device D9 was fabricated in the same manner as in Example D1, except that "the compound HM-4" was used instead of "the compound HM-3" in (Formation of light emitting layer) in Example D1.

Voltage was applied to the light emitting device D9, to observe EL light emission. The light emission efficiency [cd/A] at 1000 cd/m² was measured. The CIE chromaticity coordinate (x, y) at 1000 cd/m² was (0.19, 0.41) .

### <Example D10> Fabrication and evaluation of light emitting device D10

A light emitting device D10 was fabricated in the same manner as in Example D1, except that "the compound HM-4 and the phosphorescent compound B5 (compound HM-4/phosphorescent compound B5 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D10, to observe EL light emission. The light emission efficiency [cd/A] at 1000 cd/m² was measured. The CIE chromaticity coordinate (x, y) at 1000 cd/m² was (0.19, 0.39) .

### <Example D11> Fabrication and evaluation of light emitting device D11

A light emitting device D11 was fabricated in the same manner as in Example D1, except that "the compound HM-4 and the phosphorescent compound B2 (compound HM-4/phosphorescent compound B2 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D11, to observe EL light emission. The light emission efficiency [cd/A] at 1000 cd/m² was measured. The CIE chromaticity coordinate (x, y) at 1000 cd/m² was (0.16, 0.35) .

### <Example D12> Fabrication and evaluation of light emitting device D12

A light emitting device D12 was fabricated in the same manner as in Example D1, except that "the compound HM-9 and the phosphorescent compound B4 (compound HM-9/phosphorescent compound B4 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D12, to observe EL light emission. The light emission efficiency [cd/A] at 1000 cd/m² was measured. The CIE chromaticity coordinate (x, y) at 1000 cd/m² was (0.19, 0.39).

### <Comparative Example CD3> Fabrication and evaluation of light emitting device CD3

A light emitting device CD3 was fabricated in the same manner as in Example D1, except that "the compound HM-1 and the phosphorescent compound B2 (compound HM-1/phosphorescent compound B2 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device CD3, to observe EL light emission. The light emission efficiency [cd/A] at 1000 cd/m² was measured. The CIE chromaticity coordinate (x, y) at 1000 cd/m² was (0.17, 0.38) .

### <Comparative Example CD4> Fabrication and evaluation of light emitting device CD4

A light emitting device CD4 was fabricated in the same manner as in Example D1, except that "the compound HM-4 and the phosphorescent compound B8 (compound HM-4/phosphorescent compound B8 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device CD4, to observe EL light emission. The light emission efficiency [cd/A] at 1000 cd/m² was measured. The CIE chromaticity coordinate (x, y) at 1000 cd/m² was (0.20, 0.45) .

The results of Examples D9 to D12 and Comparative Examples CD3 to CD4 are shown in Table 3. The relative values of the light emission efficiency of the light emitting devices D9 to D12 and CD4 are shown when the light emission efficiency of the light emitting device CD3 is taken as 1.00.

**(Table 3)**

| | light emitting device | hole transporting layer | light emitting layer | | light emission efficiency (relative value) |
|---|---|---|---|---|---|
| | | material | material | composition ratio (% by mass) | |
| Example D9 | D9 | HTL-1 | HM-4/B4 | 75/25 | 4.47 |
| Example D10 | D10 | HTL-1 | HM-4/B5 | 75/25 | 2.90 |
| Example D11 | D11 | HTL-1 | HM-4/B2 | 75/25 | 1.77 |
| Example D12 | D12 | HTL-1 | HM-9/B4 | 75/25 | 1.80 |
| Comparative Example CD3 | CD3 | HTL-1 | HM-1/B2 | 75/25 | 1.00 |
| Comparative Example CD4 | CD4 | HTL-1 | HM-4/B8 | 75/25 | 1.48 |

### <Example D13> Fabrication and evaluation of light emitting device D13

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated, to form a film with a thickness of 35 nm. In an air atmosphere, the film was heated on a hot plate at 50°C for 3 minutes, further heated at 230°C for 15 minutes, to form a hole injection layer.

### (Formation of second light emitting layer)

The polymer compound HTL-2 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a second light emitting layer.

### (Formation of first light emitting layer)

The compound HM-2, the phosphorescent compound B3 and the phosphorescent compound G1 (compound HM-2/phosphorescent compound B3/phosphorescent compound G1 = 74% by mass/25% by mass/1% by mass) were dissolved in toluene at a concentration of 2.0% by mass. The resultant toluene solution was spin-coated on the second light emitting layer, to form a film with a thickness of 75 nm, and the film was heated at 130°C for 10 minutes under a nitrogen gas atmosphere, to form a first light emitting layer.

### (Formation of electron transporting layer)

The polymer compound ETL-1 was dissolved in 2,2,3,3,4,4,5,5-octafluoro-1-pentanol at a concentration of 0.25% by mass. The resultant 2,2,3,3,4,4,5,5-octafluoro-1-pentanol solution was spin-coated on the first light emitting layer, to form a film with a thickness of 10 nm, and the film was heated at 130°C for 10 minutes under a nitrogen gas atmosphere, to form an electron transporting layer.

### (Formation of cathode)

The substrate carrying the electron transporting layer formed thereon was placed in a vapor deposition machine and the inner pressure thereof was reduced to 1.0×10⁻⁴ Pa or less, then, as cathode, sodium fluoride was vapor-deposited with a thickness of about 4 nm on the electron transporting layer, then, aluminum was vapor-deposited with a thickness of about 80 nm on the sodium fluoride layer. After vapor deposition, sealing was performed with a glass substrate, to fabricate a light emitting device D13.

### (Evaluation of light emitting device)

Voltage was applied to the light emitting device D13, to observe EL light emission. The light emission efficiency [cd/A] at 400 cd/m² was measured. The CIE chromaticity coordinate (x, y) at 400 cd/m² was (0.41, 0.46).

### <Comparative Example CD5> Fabrication and evaluation of light emitting device CD5

A light emitting device CD5 was fabricated in the same manner as in Example D13, except that "the compound HM-1" was used instead of "the compound HM-2" in (Formation of first light emitting layer) in Example D13.

Voltage was applied to the light emitting device CD5, to observe EL light emission. The light emission efficiency [cd/A] at 400 cd/m² was measured. The CIE chromaticity coordinate (x, y) at 400 cd/m² was (0.40, 0.44) .

The results of Example D13 and Comparative Example CD5 are shown in Table 4. The relative value of the light emission efficiency of the light emitting device D13 is shown when the light emission efficiency of the light emitting device D15 is taken as 1.00.

**(Table 4)**

| | light emitting device | second light emitting layer | first light emitting layer | | light emission efficiency (relative value) |
|---|---|---|---|---|---|
| | | material | material | composition ratio (% by mass) | |
| Example D13 | D13 | HTL-2 | HM-2/B3/G1 | 74/25/1 | 1.14 |
| Comparative Example CD5 | CD5 | HTL-2 | HM-1/B3/G1 | 74/25/1 | 1.00 |

### <Example D14> Fabrication and evaluation of light emitting device D14

A light emitting device D14 was fabricated in the same manner as in Example D13, except that "the compound HM-3, the phosphorescent compound B7 and the phosphorescent compound G1 (compound HM-3/phosphorescent compound B7/phosphorescent compound G1 = 74% by mass/25% by mass/1% by mass)" were used instead of "the compound HM-2, the phosphorescent compound B3 and the phosphorescent compound G1 (compound HM-2/phosphorescent compound B3/phosphorescent compound G1 = 74% by mass/25% by mass/1% by mass)" in (Formation of first light emitting layer) in Example D13.

Voltage was applied to the light emitting device D14, to observe EL light emission. The light emission efficiency [cd/A] at 10000 cd/m² was measured. The CIE chromaticity coordinate (x, y) at 10000 cd/m² was (0.45,0.46).

### <Example D15> Fabrication and evaluation of light emitting device D15

A light emitting device D15 was fabricated in the same manner as in Example D14, except that "the compound HM-2" was used instead of "the compound HM-3" in (Formation of first light emitting layer) in Example D14.

Voltage was applied to the light emitting device D15, to observe EL light emission. The light emission efficiency [cd/A] at 10000 cd/m² was measured. The CIE chromaticity coordinate (x, y) at 10000 cd/m² was (0.47, 0.45).

### <Example D16> Fabrication and evaluation of light emitting device D16

A light emitting device D16 was fabricated in the same manner as in Example D14, except that "the compound HM-8" was used instead of "the compound HM-3" in (Formation of first light emitting layer) in Example D14.

Voltage was applied to the light emitting device D16, to observe EL light emission. The light emission efficiency [cd/A] at 10000 cd/m² was measured. The CIE chromaticity coordinate (x, y) at 10000 cd/m² was (0.48, 0.45).

### <Example D17> Fabrication and evaluation of light emitting device D17

A light emitting device D17 was fabricated in the same manner as in Example D14, except that "the compound HM-5" was used instead of "the compound HM-3" in (Formation of first light emitting layer) in Example D14.

Voltage was applied to the light emitting device D17, to observe EL light emission. The light emission efficiency [cd/A] at 10000 cd/m² was measured. The CIE chromaticity coordinate (x, y) at 10000 cd/m² was (0.43, 0.46).

### <Comparative Example CD6> Fabrication and evaluation of light emitting device CD6

A light emitting device CD6 was fabricated in the same manner as in Example D14, except that "the compound HM-1" was used instead of "the compound HM-3" in (Formation of first light emitting layer) in Example D14.

Voltage was applied to the light emitting device CD6, to observe EL light emission. The light emission efficiency [cd/A] at 10000 cd/m² was measured. The CIE chromaticity coordinate (x, y) at 10000 cd/m² was (0.48, 0.45).

### <Comparative Example CD7> Fabrication and evaluation of light emitting device CD7

A light emitting device CD7 was fabricated in the same manner as in Example D13, except that "the compound HM-2, the phosphorescent compound B8 and the phosphorescent compound G1 (compound HM-2/phosphorescent compound B8/phosphorescent compound G1 = 74% by mass/25% by mass/1% by mass)" were used instead of "the compound HM-2, the phosphorescent compound B3 and the phosphorescent compound G1 (compound HM-2/phosphorescent compound B3/phosphorescent compound G1 = 74% by mass/25% by mass/1% by mass)" in (Formation of first light emitting layer) in Example D13.

Voltage was applied to the light emitting device CD7, to observe EL light emission. The light emission efficiency [cd/A] at 10000 cd/m² was measured. The CIE chromaticity coordinate (x, y) at 10000 cd/m² was (0.47, 0.46).

The results of Examples D14 to D17 and Comparative Examples CD6 to CD7 are shown in Table 5. The relative values of the light emission efficiency of the light emitting devices D14 to D17 and CD7 are shown when the light emission efficiency of the light emitting device CD6 is taken as 1.00.

**(Table 5)**

| | light emitting device | second light emitting layer | first light emitting layer | | light emission efficiency (relative value) |
|---|---|---|---|---|---|
| | | material | material | composition ratio (% by mass) | |
| Example D14 | D14 | HTL-2 | HM-3/B7/G1 | 74/25/1 | 1.19 |
| Example D15 | D15 | HTL-2 | HM-2/B7/G1 | 74/25/1 | 1.17 |
| Example D16 | D16 | HTL-2 | HM-8/B7/G1 | 74/25/1 | 1.16 |
| Example D17 | D17 | HTL-2 | HM-5/B7/G1 | 74/25/1 | 1.29 |
| Comparative Example CD6 | CD6 | HTL-2 | HM-1/B7/G1 | 74/25/1 | 1.00 |
| Comparative Example CD7 | CD7 | HTL-2 | HM-2/B8/G1 | 74/25/1 | 0.92 |

### <Example D18> Fabrication and evaluation of light emitting device D18

A light emitting device D18 was fabricated in the same manner as in Example D1, except that "the compound HM-2, the phosphorescent compound B2, the phosphorescent compound G2 and the phosphorescent compound R1 (compound HM-2/phosphorescent compound B2/phosphorescent compound G2/phosphorescent compound R1 = 62.05% by mass/37.5% by mass/0.25% by mass/0.20% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D18, to observe EL light emission. The light emission efficiency [cd/A] at 10000 cd/m² was measured. The CIE chromaticity coordinate (x, y) at 10000 cd/m² was (0.34, 0.41).

### <Comparative Example CD8> Fabrication and evaluation of light emitting device CD8

A light emitting device CD8 was fabricated in the same manner as in Example D1, except that "the compound HM-1, the phosphorescent compound B2, the phosphorescent compound G2 and the phosphorescent compound R1 (compound HM-1/phosphorescent compound B2/phosphorescent compound G2/phosphorescent compound R1 = 62.05% by mass/37.5% by mass/0.25% by mass/0.20% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device CD8, to observe EL light emission. The light emission efficiency [cd/A] at 10000 cd/m² was measured. The CIE chromaticity coordinate (x, y) at 10000 cd/m² was (0.31, 0.38).

The results of Example D18 and Comparative Example CD8 are shown in Table 6. The relative value of the light emission efficiency of the light emitting device D18 is shown when the light emission efficiency of the light emitting device CD8 is taken as 1.00.

**(Table 6)**

| | light emitting device | hole transporting layer | light emitting layer | | light emission efficiency (relative value) |
|---|---|---|---|---|---|
| | | material | material | composition ratio (% by mass) | |
| Example D17 | D17 | HTL-1 | HM-2/B2/G2/R 1 | 62.05/37.5/ 0.25/0.20 | 1.18 |
| Comparative Example CD8 | CD8 | HTL-1 | HM-1/B2/G2/R 1 | 62.05/37.5/ 0.25/0.20 | 1.00 |

### <Example D19> Fabrication and evaluation of light emitting device D19

A light emitting device D19 was fabricated in the same manner as in Example D1, except that "the compound HM-3 and the phosphorescent compound B7 (compound HM-3/phosphorescent compound B7 = 75% by mass/25% by mass)" were used instead of "the compound HM-3 and the phosphorescent compound B4 (compound HM-3/phosphorescent compound B4 = 75% by mass/25% by mass)" in (Formation of light emitting layer) in Example D1.

Voltage was applied to the light emitting device D19, to observe EL light emission. The light emission efficiency [cd/A] at 5000 cd/m² was measured. The CIE chromaticity coordinate (x, y) at 5000 cd/m² was (0.20, 0.44) .

### <Example D20> Fabrication and evaluation of light emitting device D20

A light emitting device D20 was fabricated in the same manner as in Example D19, except that "the compound HM-2" was used instead of "the compound HM-3" in (Formation of light emitting layer) in Example D19.

Voltage was applied to the light emitting device D20, to observe EL light emission. The light emission efficiency [cd/A] at 5000 cd/m² was measured. The CIE chromaticity coordinate (x, y) at 5000 cd/m² was (0.21, 0.46) .

### <Example D21> Fabrication and evaluation of light emitting device D21

A light emitting device D21 was fabricated in the same manner as in Example D19, except that "the compound HM-8" was used instead of "the compound HM-3" in (Formation of light emitting layer) in Example D19.

Voltage was applied to the light emitting device D21, to observe EL light emission. The light emission efficiency [cd/A] at 5000 cd/m² was measured. The CIE chromaticity coordinate (x, y) at 5000 cd/m² was (0.20, 0.44) .

### <Example D22> Fabrication and evaluation of light emitting device D22

A light emitting device D22 was fabricated in the same manner as in Example D19, except that "the compound HM-5" was used instead of "the compound HM-3" in (Formation of light emitting layer) in Example D19.

Voltage was applied to the light emitting device D22, to observe EL light emission. The light emission efficiency [cd/A] at 5000 cd/m² was measured. The CIE chromaticity coordinate (x, y) at 5000 cd/m² was (0.19, 0.41) .

### <Example D23> Fabrication and evaluation of light emitting device D23

A light emitting device D23 was fabricated in the same manner as in Example D19, except that "the compound HM-7" was used instead of "the compound HM-3" in (Formation of light emitting layer) in Example D19.

Voltage was applied to the light emitting device D23, to observe EL light emission. The light emission efficiency [cd/A] at 5000 cd/m² was measured. The CIE chromaticity coordinate (x, y) at 5000 cd/m² was (0.21, 0.46) .

### <Example D24> Fabrication and evaluation of light emitting device D24

A light emitting device D24 was fabricated in the same manner as in Example D19, except that "the compound HM-6" was used instead of "the compound HM-3" in (Formation of light emitting layer) in Example D19.

Voltage was applied to the light emitting device D24, to observe EL light emission. The light emission efficiency [cd/A] at 5000 cd/m² was measured. The CIE chromaticity coordinate (x, y) at 5000 cd/m² was (0.22, 0.47) .

The results of Examples D19 to D24 are shown in Table 7. The relative values of the light emission efficiency of the light emitting devices D19 to D23 are shown when the light emission efficiency of the light emitting device D24 is taken as 1.00.

**(Table 7)**

| | light emitting device | hole transporting layer | light emitting layer | | light emission efficiency (relative value) |
|---|---|---|---|---|---|
| | | material | material | composition ratio (% by mass) | |
| Example D19 | D19 | HTL-1 | HM-3/B7 | 75/25 | 2.04 |
| Example D20 | D20 | HTL-1 | HM-2/B7 | 75/25 | 1.43 |
| Example D21 | D21 | HTL-1 | HM-8/B7 | 75/25 | 1.42 |
| Example D22 | D22 | HTL-1 | HM-5/B7 | 75/25 | 1.43 |
| Example D23 | D23 | HTL-1 | HM-7/B7 | 75/25 | 1.27 |
| Example D24 | D24 | HTL-1 | HM-6/B7 | 75/25 | 1.00 |

### Industrial Applicability

According to the present invention, a composition which is useful for production of a light emitting device excellent in light emission efficiency can be provided. Further, according to the present invention, a light emitting device comprising this composition can be provided.

## Claims

1. A composition comprising
a compound represented by the formula (C-1) and
a phosphorescent compound represented by the formula (1), 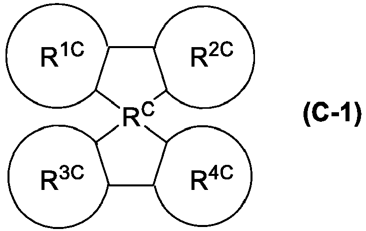 wherein,
Ring R^{1C}, Ring R^{2C}, Ring R^{3C} and Ring R^{4C} each independently represent an aromatic hydrocarbon ring or an aromatic hetero ring, and the foregoing rings optionally have a substituent, when a plurality of the substituents are present, they may be combined together to form a ring together with the atoms to which they are attached, and wherein at least one of Ring R^{1C}, Ring R^{2C}, Ring R^{3C} and Ring R^{4C} has a substituent that is an aryl group optionally having a substituent or a monovalent heterocyclic group optionally having a substituent,
R^{C} represents a carbon atom, a silicon atom, or a germanium atom, 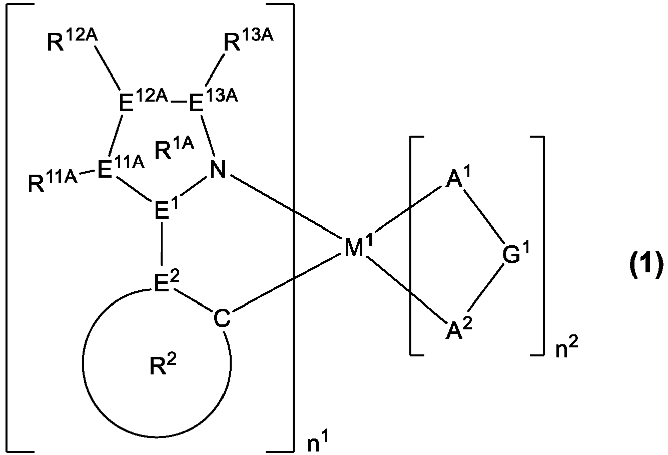 wherein,
M¹ represents a rhodium atom, a palladium atom, an iridium atom or a platinum atom,
n¹ represents an integer of 1 or more, n² represents an integer of 0 or more, and n¹+n² is 3 when M¹ is a rhodium atom or an iridium atom, while n¹+n² is 2 when M¹ is a palladium atom or a platinum atom,
Ring R^{1A} represents a triazole ring,
Ring R² represents an aromatic hydrocarbon ring or an aromatic hetero ring, and the foregoing rings optionally have a substituent, and when a plurality of the substituents are present, they may be combined together to form a ring together with the atoms to which they are attached, and when a plurality of Ring R² are present, they may be the same or different,
E¹, E², E^{11A}, E^{12A} and E^{13A} each independently represent a nitrogen atom or a carbon atom, and when a plurality of E¹, E², E^{11A}, E^{12A} and E^{13A} are present, they may be the same or different at each occurrence, and at least one of E¹ and E² is a carbon atom,
R^{11A}, R^{12A} and R^{13A} each independently represent an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent, and when a plurality of R^{11A}, R^{12A} and R^{13A} are present, they may be the same or different at each occurrence,
R^{11A} and R^{12A} may be combined together to form a ring together with the atoms to which they are attached, R^{12A} and R^{13A} may be combined together to form a ring together with the atoms to which they are attached, and the substituent which Ring R² optionally has and R^{11A} may be combined together to form a ring together with the atoms to which they are attached,
when E^{11A} is a nitrogen atom, R^{11A} may be present or absent, when E^{12A} is a nitrogen atom, R^{12A} may be present or absent, and when E^{13A} is a nitrogen atom, R^{13A} may be present or absent,
A¹-G¹-A² represents an anionic bidentate ligand, A¹ and A² each independently represent a carbon atom, an oxygen atom or a nitrogen atom, and these atoms may be a ring constituent atom, G¹ represents a single bond or an atomic group constituting a bidentate ligand together with A¹ and A², and when a plurality of A¹-G¹-A² are present, they may be the same or different.

2. The composition according to Claim 1, wherein at least one of said Ring R^{1C}, said Ring R^{2C}, said Ring R^{3C} and said Ring R^{4C} has a group represented by the formula (D-1), 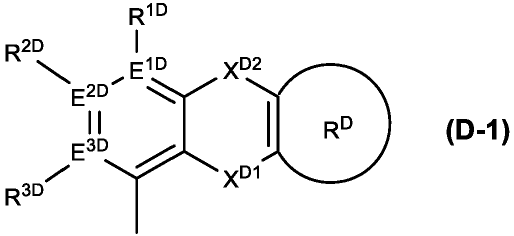 wherein,
Ring R^{D} represents an aromatic hydrocarbon ring or an aromatic hetero ring, and the foregoing rings optionally have a substituent, and when a plurality of the substituents are present, they may be combined together to form a ring together with the atoms to which they are attached,
X^{D1} and X^{D2} each independently represent a single bond, an oxygen atom, a sulfur atom, a group represented by -N(R^{XD1})- or a group represented by - C(R^{XD2})₂-, R^{XD1} and R^{XD2} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent, and a plurality of R^{XD2} may be the same or different and may be combined together to form a ring together with the carbon atom to which they are attached,
E^{1D}, E^{2D} and E^{3D} each independently represent a nitrogen atom or a carbon atom,
R^{1D}, R^{2D} and R^{3D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent,
when E^{1D} is a nitrogen atom, R^{1D} is absent, when E^{2D} is a nitrogen atom, R^{2D} is absent, and when E^{3D} is a nitrogen atom, R^{3D} is absent,
R^{1D} and R^{2D} may be combined together to form a ring together with the carbon atoms to which they are attached, R^{2D} and R^{3D} may be combined together to form a ring together with the carbon atoms to which they are attached, R^{1D} and R^{XD1} may be combined together to form a ring together with the atoms to which they are attached, R^{1D} and R^{XD2} may be combined together to form a ring together with the carbon atoms to which they are attached, the substituent which Ring R^{D} optionally has and R^{XD1} may be combined together to form a ring together with the atoms to which they are attached, and the substituent which Ring R^{D} optionally has and R^{XD2} may be combined together to form a ring together with the carbon atoms to which they are attached.

3. The composition according to Claim 2, wherein the group represented by said formula (D-1) is a group represented by the formula (D-2), 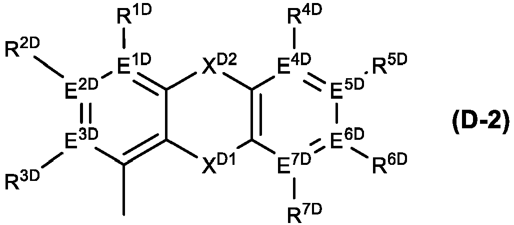 wherein,
X^{D1}, X^{D2}, E^{1D}, E^{2D}, E^{3D}, R^{1D}, R^{2D} and R^{3D} represent the same meaning as described above,
E^{4D}, E^{5D}, E^{6D} and E^{7D} each independently represent a nitrogen atom or a carbon atom,
R^{4D}, R^{5D}, R^{6D} and R^{7D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent,
when E^{4D} is a nitrogen atom, R^{4D} is absent, when E^{5D} is a nitrogen atom, R^{5D} is absent, when E^{6D} is a nitrogen atom, R^{6D} is absent, and when E^{7D} is a nitrogen atom, R^{7D} is absent, and
R^{4D} and R^{5D}, R^{5D} and R^{6D}, R^{6D} and R^{7D}, R^{4D} and R^{XD1}, R^{4D} and R^{XD2}, R^{7D} and R^{XD1}, and R^{7D} and R^{XD2} each may be combined together to form a ring together with the atoms to which they are attached.

4. The composition according to any one of Claims 1 to 3, wherein the compound represented by said formula (C-1) is a compound represented by the formula (C-2), 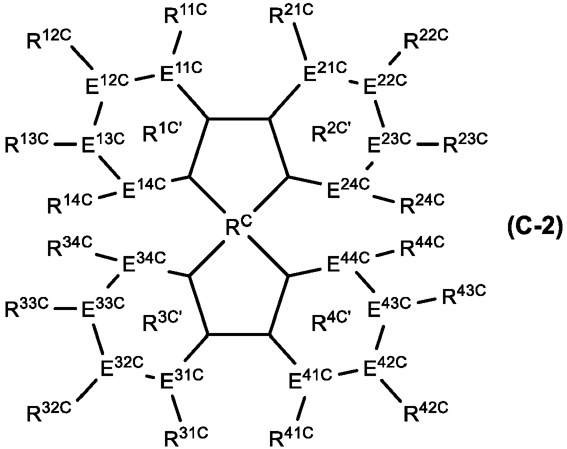 wherein,
R^{C} represents the same meaning as described above,
E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} and E^{44C} each independently represent a nitrogen atom or a carbon atom,
Ring R^{1C}', Ring R^{2C}', Ring R^{3C}' and Ring R^{4C}' each independently represent a benzene ring, a pyridine ring or a diazabenzene ring,
R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent, and wherein at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group optionally having a substituent or a monovalent heterocyclic group optionally having a substituent,
when E^{11C} is a nitrogen atom, R^{11C} is absent, when E^{12C} is a nitrogen atom, R^{12C} is absent, when E^{13C} is a nitrogen atom, R^{13C} is absent, when E^{14C} is a nitrogen atom, R^{14C} is absent, when E^{21C} is a nitrogen atom, R^{21C} is absent, when E^{22C} is a nitrogen atom, R^{22C} is absent, when E^{23C} is a nitrogen atom, R^{23C} is absent, when E^{24C} is a nitrogen atom, R^{24C} is absent, when E^{31C} is a nitrogen atom, R^{31C} is absent, when E^{32C} is a nitrogen atom, R^{32C} is absent, when E^{33C} is a nitrogen atom, R^{33C} is absent, when E^{34C} is a nitrogen atom, R^{34C} is absent, when E^{41C} is a nitrogen atom, R^{41C} is absent, when E^{42C} is a nitrogen atom, R^{42C} is absent, when E^{43C} is a nitrogen atom, R^{43C} is absent, and when E^{44C} is a nitrogen atom, R^{44C} is absent, and
R^{11C} and R^{12C}, R^{12C} and R^{13C}, R^{13C} and R^{14C}, R^{14C} and R^{34C}, R^{34C} and R^{33C}, R^{33C} and R^{32C}, R^{32C} and R^{31C}, R^{31C} and R^{41C}, R^{41C} and R^{42C}, R^{42C} and R^{43C}, R^{43C} and R^{44C}, R^{44C} and R^{24C}, R^{24C} and R^{23C}, R^{23C} and R^{22C}, R^{22C} and R^{21C}, and R^{21C} and R^{11C} each may be combined together to form a ring together with the carbon atoms to which they are attached.

5. The composition according to Claim 4, wherein the compound represented by said formula (C-2) is a compound represented by the formula (C-3), 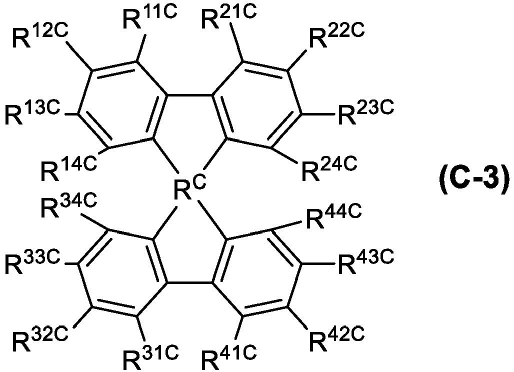 wherein, R^{C}, R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} represent the same meaning as described above.

6. The composition according to Claim 4 or 5, wherein at least one of said R^{11C}, said R^{12C}, said R^{14C}, said R^{21C}, said R^{22C}, said R^{24C}, said R^{31C}, said R^{32C}, said R^{34C}, said R^{41C}, said R^{42C} and said R^{44C} is a group represented by said formula (D-1).

7. The composition according to any one of Claims 1 to 6, wherein the phosphorescent compound represented by said formula (1) is a phosphorescent compound represented by the formula (1-A), 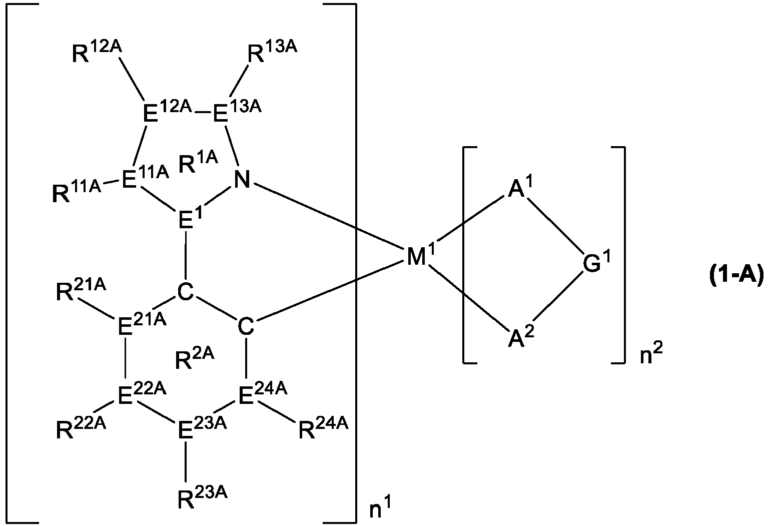 wherein,
M¹, n¹, n², Ring R^{1A}, E¹, E^{11A}, E^{12A}, E^{13A}, R^{11A}, R^{12A}, R^{13A} and A¹-G¹-A² represent the same meaning as described above,
Ring R^{2A} represents a benzene ring, a pyridine ring or a diazabenzene ring,
E^{21A}, E^{22A}, E^{23A} and E^{24A} each independently represent a nitrogen atom or a carbon atom, and when a plurality of E^{21A}, E^{22A}, E^{23A} and E^{24A} are present, they may be the same or different at each occurrence, and when E^{21A} is a nitrogen atom, R^{21A} is absent, when E^{22A} is a nitrogen atom, R^{22A} is absent, when E^{23A} is a nitrogen atom, R^{23A} is absent, when E^{24A} is a nitrogen atom, R^{24A} is absent,
R^{21A}, R^{22A}, R^{23A} and R^{24A} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent, and when a plurality of R^{21A}, R^{22A}, R^{23A} and R^{24A} are present, they may be the same or different at each occurrence, and, R^{21A} and R^{22A}, R^{22A} and R^{23A}, R^{23A} and R^{24A}, and R^{11A} and R^{21A} each may be combined together to form a ring together with the atoms to which they are attached.

8. The composition according to Claim 7, wherein the phosphorescent compound represented by said formula (1-A) is a phosphorescent compound represented by the formula (1-A1), a phosphorescent compound represented by the formula (1-A2) or a phosphorescent compound represented by the formula (1-A3), 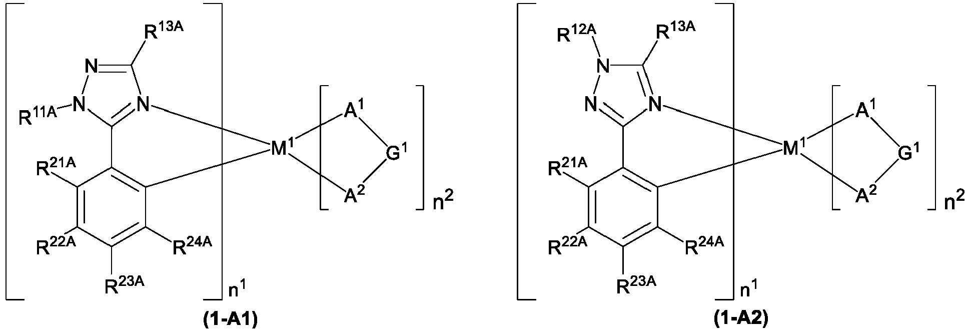 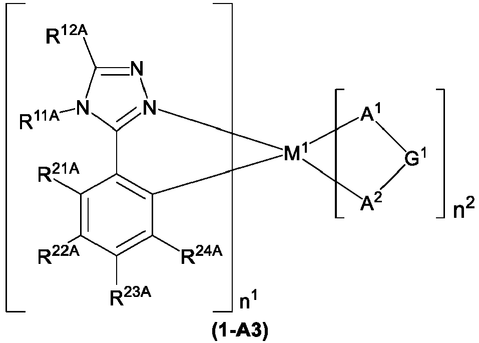 wherein, M¹, n¹, n², R^{11A}, R^{12A}, R^{13A}, R^{21A}, R^{22A}, R^{23A}, R^{24A} and A¹-G¹-A² represent the same meaning as described above.

9. A light emitting device comprising the composition according to any one of Claims 1 to 8.

## Patentansprüche

1. Zusammensetzung, die Folgendes umfasst:
eine Verbindung, die durch die Formel (C-1) dargestellt wird, und
eine phosphoreszierende Verbindung, die durch die Formel (1) dargestellt wird, 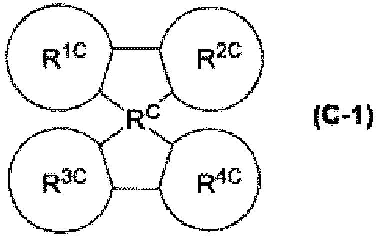 wobei,
Ring R^{1C}, Ring R^{2C}, Ring R^{3C} und Ring R^{4C} jeweils unabhängig voneinander einen aromatischen Kohlenwasserstoffring oder einen aromatischen Heteroring darstellen, und die vorgenannten Ringe optional einen Substituenten aufweisen, wenn mehrere der Substituenten vorhanden sind, sie zusammen mit den Atomen, an die sie gebunden sind, zu einem Ring kombiniert werden können, und wobei mindestens einer von Ring R^{1C}, Ring R^{2C}, Ring R^{3C} und Ring R^{4C} einen Substituenten aufweist, der eine Arylgruppe, die optional einen Substituenten aufweist, oder eine einwertige heterocyclische Gruppe, die optional einen Substituenten aufweist, ist,
R^{C} ein Kohlenstoffatom, ein Siliziumatom oder ein Germaniumatom darstellt, 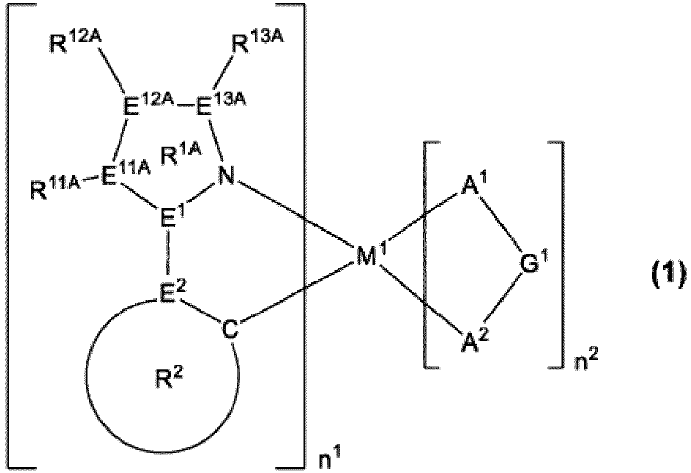 wobei
M¹ ein Rhodiumatom, ein Palladiumatom, ein Iridiumatom oder ein Platinatom darstellt,
n¹ eine ganze Zahl von 1 oder mehr darstellt, n² eine ganze Zahl von 0 oder mehr darstellt, und n¹ + n² 3 ist, wenn M¹ ein Rhodiumatom oder ein Iridiumatom ist, während n¹ + n² gleich 2 ist, wenn M¹ ein Palladiumatom oder ein Platinatom ist,
Ring R^{1A} einen Triazolring darstellt,
Ring R² einen aromatischen Kohlenwasserstoffring oder einen aromatischen Heteroring darstellt, und die vorgenannten Ringe optional einen Substituenten aufweisen, und wenn mehrere der Substituenten vorhanden sind, sie zusammen mit den Atomen, an die sie gebunden sind, zu einem Ring kombiniert werden können, und wenn mehrere Ringe R² vorhanden sind, sie gleich oder verschieden sein können,
E¹, E², E^{11A}, E^{12A} und E^{13A} jeweils unabhängig voneinander ein Stickstoffatom oder ein Kohlenstoffatom darstellen, und wenn mehrere E¹, E², E^{11A}, E^{12A} und E^{13A} vorhanden sind, sie bei jedem Auftreten gleich oder verschieden sein können, und mindestens eines von E¹ und E² ein Kohlenstoffatom ist,
R^{11A}, R^{12A} und R^{13A} jeweils unabhängig voneinander Folgendes darstellen: eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, wobei die vorgenannten Gruppen optional einen Substituenten aufweisen, und wenn mehrere R^{11A}, R^{12A} und R^{13A} vorhanden sind, sie bei jedem Auftreten gleich oder verschieden sein können,
R^{11A} und R^{12A} zusammen mit den Atomen, an die sie gebunden sind, zu einem Ring kombiniert werden können, R^{12A} und R^{13A} zusammen mit den Atomen, an die sie gebunden sind, zu einem Ring kombiniert werden können, und der Substituent, dessen Ring R² optional aufweist, und R^{11A} zusammen mit den Atomen, an die sie gebunden sind, zu einem Ring kombiniert werden können,
wenn E^{11A} ein Stickstoffatom ist, R^{11A} vorhanden oder abwesend sein kann, wenn E^{12A} ein Stickstoffatom ist, R^{12A} vorhanden oder abwesend sein kann, und wenn E^{13A} ein Stickstoffatom ist, R^{13A} vorhanden oder abwesend sein kann,
A¹-G¹-A² einen anionischen zweizähnigen Liganden darstellt, A¹ und A² jeweils unabhängig voneinander ein Kohlenstoffatom, ein Sauerstoffatom oder ein Stickstoffatom darstellen, und diese Atome ein ringbildendes Atom sein können, G¹ eine Einfachbindung oder eine Atomgruppe darstellt, die zusammen mit A¹ und A² einen zweizähnigen Liganden bildet, und wenn mehrere A¹-G¹-A² vorhanden sind, sie gleich oder verschieden sein können.

2. Zusammensetzung nach Anspruch 1, wobei mindestens einer der Ringe R^{1C}, R^{2C}, R^{3C} und R^{4C} eine durch die Formel (D-1) dargestellte Gruppe aufweist, 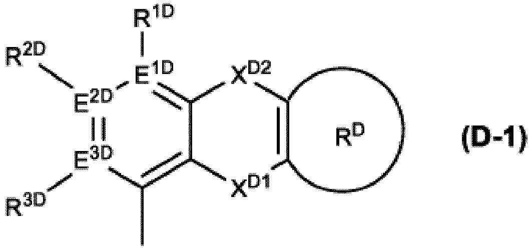 wobei,
Ring R^{D} einen aromatischen Kohlenwasserstoffring oder einen aromatischen Heteroring darstellt, und die vorgenannten Ringe optional einen Substituenten aufweisen, und wenn mehrere der Substituenten vorhanden sind, sie zusammen mit den Atomen, an die sie gebunden sind, zu einem Ring kombiniert werden können,
X^{D1} und X^{D2} jeweils unabhängig voneinander eine Einfachbindung, ein Sauerstoffatom, ein Schwefelatom, eine durch -N(R^{XD1})- oder eine durch -C(R^{XD2})₂-dargestellte Gruppe darstellen, R^{XD1} und R^{XD2} jeweils unabhängig voneinander Folgendes darstellen: ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, und die vorgenannten Gruppen optional einen Substituenten aufweisen, und mehrere R^{XD2} gleich oder verschieden sein können und zusammen mit dem Kohlenstoffatom, an das sie gebunden sind, einen Ring bilden können,
E^{1D}, E^{2D} und E^{3D} jeweils unabhängig voneinander ein Stickstoffatom oder ein Kohlenstoffatom darstellen,
R^{1D}, R^{2D} und R^{3D} jeweils unabhängig voneinander Folgendes darstellen: ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, und die vorgenannten Gruppen optional einen Substituenten aufweisen,
wenn E^{1D} ein Stickstoffatom ist, R^{1D} abwesend ist, wenn E^{2D} ein Stickstoffatom ist, R^{2D} abwesend ist, und wenn E^{3D} ein Stickstoffatom ist, R^{3D} abwesend ist,
R^{1D} und R^{2D} zusammen mit den Kohlenstoffatomen, an die sie gebunden sind, zu einem Ring kombiniert werden können, R^{2D} und R^{3D} zusammen mit den Kohlenstoffatomen, an die sie gebunden sind, zu einem Ring kombiniert werden können, R^{1D} und R^{XD1} zusammen mit den Atomen, an die sie gebunden sind, zu einem Ring kombiniert werden können, R^{1D} und R^{XD2} zusammen mit den Kohlenstoffatomen, an die sie gebunden sind, zu einem Ring kombiniert werden können, der Substituent, dessen Ring R^{D} optional aufweist, und R^{XD1} zusammen mit den Atomen, an die sie gebunden sind, zu einem Ring kombiniert werden können, und der Substituent, dessen Ring R^{D} optional aufweist, und R^{XD2} zusammen mit den Kohlenstoffatomen, an die sie gebunden sind, zu einem Ring kombiniert werden können.

3. Zusammensetzung nach Anspruch 2, wobei die durch die Formel (D-1) dargestellte Gruppe eine durch die Formel (D-2) dargestellte Gruppe ist, 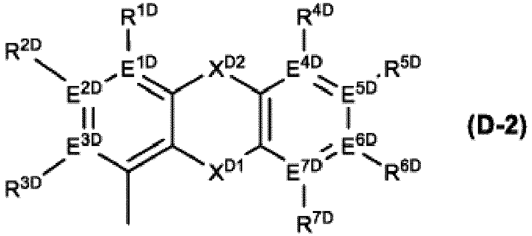 wobei
X^{D1}, X^{D2}, E^{1D}, E^{2D}, E^{3D}, R^{1D}, R^{2D} und R^{3D} die gleiche Bedeutung wie oben beschrieben haben,
E^{4D}, E^{5D}, E^{6D} und E^{7D} jeweils unabhängig voneinander ein Stickstoffatom oder ein Kohlenstoffatom darstellen,
R^{4D}, R^{5D}, R^{6D} und R^{7D} jeweils unabhängig voneinander das Folgende darstellen: ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, und die vorgenannten Gruppen optional einen Substituenten aufweisen,
wenn E^{4D} ein Stickstoffatom ist, R^{4D} abwesend ist, wenn E^{5D} ein Stickstoffatom ist, R^{5D} abwesend ist, wenn E^{6D} ein Stickstoffatom ist, R^{6D} abwesend ist, und wenn E^{7D} ein Stickstoffatom ist, R^{7D} abwesend ist, und
R^{4D} und R^{5D}, R^{5D} und R^{6D}, R^{6D} und R^{7D}, R^{4D} und R^{XD1}, R^{4D} und R^{XD2}, R^{7D} und R^{XD1} sowie R^{7D} und R^{XD2} jeweils zusammen mit den Atomen, an die sie gebunden sind, zu einem Ring kombiniert werden können.

4. Zusammensetzung nach einem der Ansprüche 1 bis 3, wobei die durch die Formel (C-1) dargestellte Verbindung eine durch die Formel (C-2) dargestellte Verbindung ist, 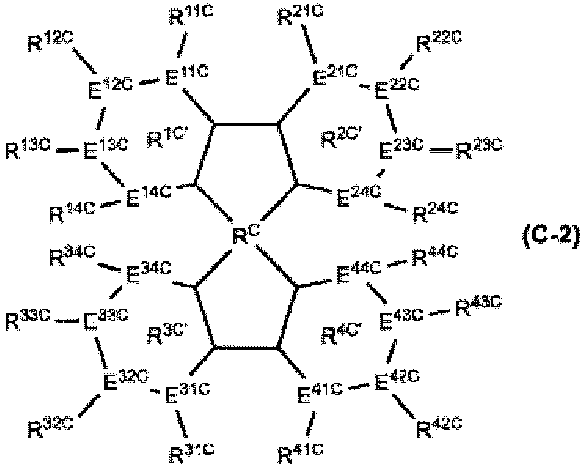 wobei,
R^{C} die gleiche Bedeutung hat wie oben beschrieben,
E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} und E^{44C} jeweils unabhängig voneinander ein Stickstoffatom oder ein Kohlenstoffatom darstellen,
Ring R^{1C'}, Ring R^{2C'}, Ring R^{3C'} und Ring R^{4C'} jeweils unabhängig voneinander einen Benzolring, einen Pyridinring oder einen Diazabenzenring darstellen,
R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} und R^{44C} jeweils unabhängig voneinander Folgendes darstellen: ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, und die vorgenannten R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} und R^{44C} eine Arylgruppe ist, die optional einen Substituenten aufweist, oder eine einwertige heterocyclische Gruppe ist, die optional einen Substituenten aufweist, wobei
wenn E^{11C} ein Stickstoffatom ist, R^{11C} abwesend ist, wenn E^{12C} ein Stickstoffatom ist, R^{12C} abwesend ist, wenn E^{13C} ein Stickstoffatom ist, R^{13C} abwesend ist, wenn E^{14C} ein Stickstoffatom ist, R^{14C} abwesend ist, wenn E^{21C} ein Stickstoffatom ist, R^{21C} abwesend ist, wenn E^{22C} ein Stickstoffatom ist, R^{22C} abwesend ist, wenn E^{23C} ein Stickstoffatom ist, R^{23C} abwesend ist, wenn E^{24C} ein Stickstoffatom ist, R^{24C} abwesend ist, wenn E^{31C} ein Stickstoffatom ist, R^{31C} abwesend ist, wenn E^{32C} ein Stickstoffatom ist, R^{32C} abwesend ist, wenn E^{33C} ein Stickstoffatom ist, R^{33C} abwesend ist, wenn E^{34C} ein Stickstoffatom ist, R^{34C} abwesend ist, wenn E^{41C} ein Stickstoffatom ist, R^{41C} abwesend ist, wenn E^{42C} ein Stickstoffatom ist, R^{42C} abwesend ist, wenn E^{43C} ein Stickstoffatom ist, R^{43C} abwesend ist, und wenn E^{44C} ein Stickstoffatom ist, R^{44C} abwesend ist, und
R^{11C} und R^{12C}, R^{12C} und R^{13C}, R^{13C} und R^{14C}, R^{14C} und R^{34C}, R^{34C} und R^{33C}, R^{33C} und R^{32C}, R^{32C} und R^{31C}, R^{31C} und R^{41C}, R^{41C} und R^{42C}, R^{42C} und R^{43C}, R^{43C} und R^{44C}, R^{44C} und R^{24C}, R^{24C} und R^{23C}, R^{23C} und R^{22C}, R^{22C} und R^{21C}, und R^{21C} und R^{11C} jeweils zusammen mit den Kohlenstoffatomen, an die sie gebunden sind, zu einem Ring kombiniert werden können.

5. Zusammensetzung nach Anspruch 4, wobei die durch die Formel (C-2) dargestellte Verbindung eine durch die Formel (C-3) dargestellte Verbindung ist, 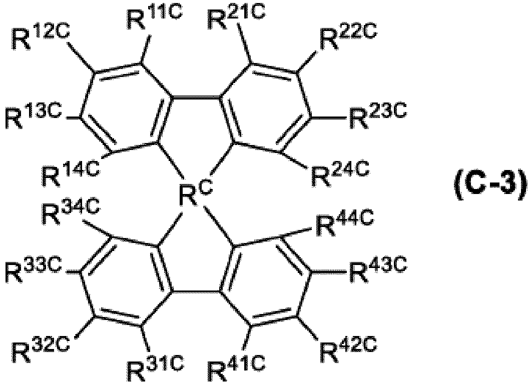 wobei R^{C}, R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} und R^{44C} die gleiche Bedeutung wie oben beschrieben haben.

6. Zusammensetzung nach Anspruch 4 oder 5, wobei mindestens eines von R^{11C}, R^{12C}, R^{14C}, R^{21C}, R^{22C}, R^{24C}, R^{31C}, R^{32C}, R^{34C}, R^{41C}, R^{42C} und R^{44C} eine Gruppe ist, die durch die Formel (D-1) dargestellt wird.

7. Zusammensetzung nach einem der Ansprüche 1 bis 6, wobei die phosphoreszierende Verbindung, dargestellt durch die Formel (1), eine phosphoreszierende Verbindung ist, dargestellt durch die Formel (1-A), 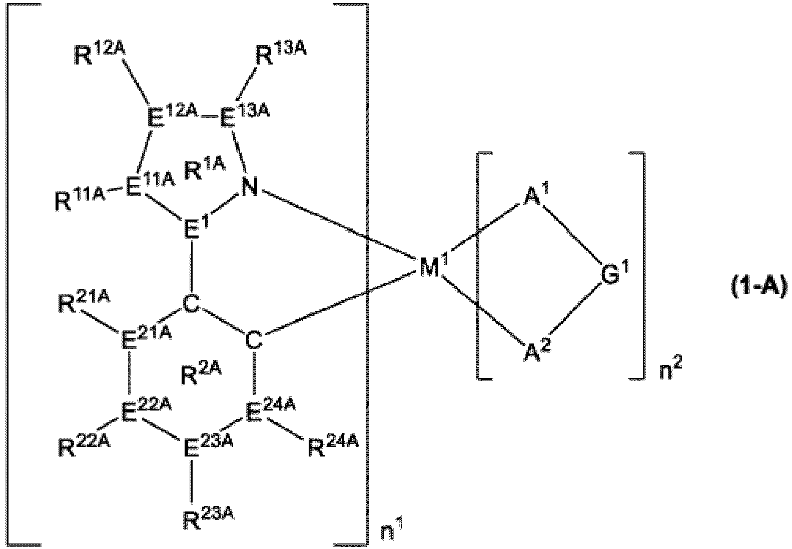 wobei,
M¹, n¹, n², Ring R^{1A}, E¹, E^{11A}, E^{12A}, E^{13A}, R^{11A}, R^{12A}, R^{13A} und A¹-G¹-A² die gleiche Bedeutung wie oben beschrieben haben,
Ring R^{2A} einen Benzolring, einen Pyridinring oder einen Diazabenzolring darstellt,
E^{21A}, E^{22A}, E^{23A} und E^{24A} jeweils unabhängig voneinander ein Stickstoffatom oder ein Kohlenstoffatom darstellen, und wenn mehrere E^{21A}, E^{22A}, E^{23A} und E^{24A} vorhanden sind, sie bei jedem Auftreten gleich oder verschieden sein können, und wenn E^{21A} ein Stickstoffatom ist, R^{21A} abwesend ist, wenn E^{22A} ein Stickstoffatom ist, R^{22A} abwesend ist, wenn E^{23A} ein Stickstoffatom ist, R^{23A} abwesend ist, wenn E^{24A} ein Stickstoffatom ist, R^{24A} abwesend ist,
R^{21A}, R^{22A}, R^{23A} und R^{24A} jeweils unabhängig voneinander Folgendes darstellen: ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom, und die vorgenannten Gruppen optional einen Substituenten aufweisen, und wenn mehrere R^{21A}, R^{22A}, R^{23A} und R^{24A} vorhanden sind, sie bei jedem Auftreten gleich oder verschieden sein können, und R^{21A} und R^{22A}, R^{22A} und R^{23A}, R^{23A} und R^{24A} sowie R^{11A} und R^{21A} jeweils zusammen mit den Atomen, an die sie gebunden sind, zu einem Ring kombiniert werden können.

8. Zusammensetzung nach Anspruch 7, wobei die phosphoreszierende Verbindung, die durch die Formel (1-A) dargestellt wird, eine phosphoreszierende Verbindung, die durch die Formel (1-A1) dargestellt wird, eine phosphoreszierende Verbindung, die durch die Formel (1-A2) dargestellt wird, oder eine phosphoreszierende Verbindung, die durch die Formel (1-A3) dargestellt wird, ist, 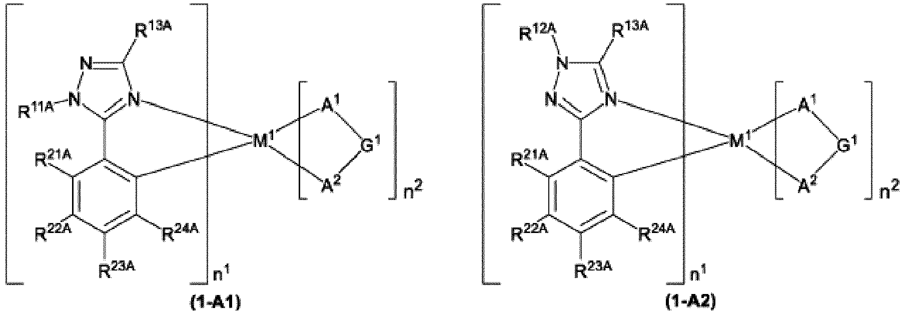 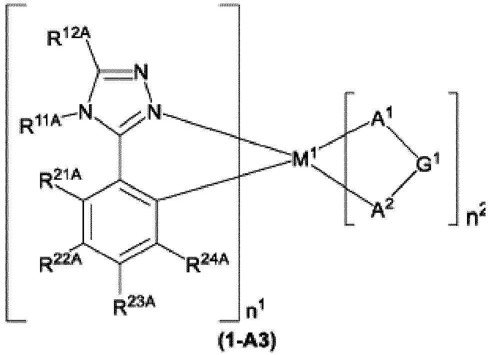 wobei M¹, n¹, n², R^{11A}, R^{12A}, R^{13A}, R^{21A}, R^{22A}, R^{23A}, R^{24A} und A¹-G¹-A² die gleiche Bedeutung wie oben beschrieben haben.

9. Lichtemittierende Vorrichtung, die die Zusammensetzung nach einem der Ansprüche 1 bis 8 umfasst.

## Revendications

1. Composition comprenant
un composé représenté par la formule (C-1) et
un composé phosphorescent représenté par la formule (1), 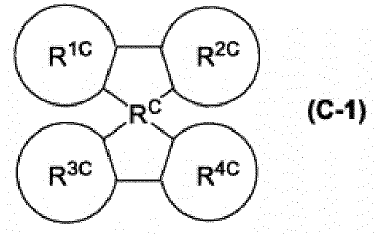 dans laquelle,
le cycle R^{1C}, le cycle R^{2C}, le cycle R^{3C} et le cycle R^{4C} représentent chacun indépendamment un cycle hydrocarboné aromatique ou un cycle hétéro aromatique, et les cycles précédents ont éventuellement un substituant, lorsqu'une pluralité de substituants sont présents, ils peuvent être combinés ensemble pour forment un cycle avec les atomes auxquels ils sont attachés, et dans laquelle au moins l'un parmi le cycle R^{1C}, le cycle R^{2C}, le cycle R^{3C} et le cycle R^{4C} a un substituant qui est un groupe aryle ayant éventuellement un substituant ou un groupe hétérocyclique monovalent ayant éventuellement un substituant,
R^{C} représente un atome de carbone, un atome de silicium ou un atome de germanium, 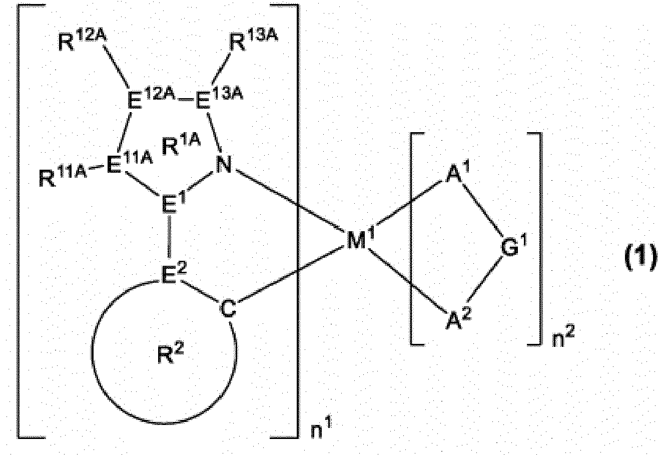 dans laquelle,
M¹ représente un atome de rhodium, un atome de palladium, un atome d'iridium ou un atome de platine,
n¹ représente un nombre entier de 1 ou plus, n² représente un nombre entier de 0 ou plus, et n¹+n² vaut 3 lorsque M¹ est un atome de rhodium ou un atome d'iridium, tandis que n¹+n² vaut 2 lorsque M¹ est un atome de palladium ou un atome de platine,
le cycle R^{1A} représente un cycle triazole,
le cycle R² représente un cycle hydrocarboné aromatique ou un cycle hétéro aromatique, et les cycles précédents ont éventuellement un substituant, et lorsqu'une pluralité de substituants sont présents, ils peuvent être combinés ensemble pour former un cycle avec les atomes auxquels ils sont attachés, et lorsqu'une pluralité de cycles R² sont présents, ils peuvent être identiques ou différents,
E¹, E², E^{11A}, E^{12A} et E^{13A} représentent chacun indépendamment un atome d'azote ou un atome de carbone, et lorsqu'une pluralité de E¹, E², E^{11A}, E^{12A} et E^{13A} sont présents, ils peuvent être identiques ou différents à chaque occurrence, et au moins un parmi E¹ et E² est un atome de carbone,
R^{11A}, R^{12A} et R^{13A} représentent chacun indépendamment un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant, et lorsque une pluralité de R^{11A}, R^{12A} et R^{13A} sont présents, ils peuvent être identiques ou différents à chaque occurrence,
R^{11A} et R^{12A} peuvent être combinés ensemble pour former un cycle avec les atomes auxquels ils sont attachés, R^{12A} et R^{13A} peuvent être combinés ensemble pour former un cycle avec les atomes auxquels ils sont attachés, et le substituant que le cycle R² a éventuellement et R^{11A} peuvent être combinés ensemble pour former un cycle avec les atomes auxquels ils sont attachés,
lorsque E^{11A} est un atome d'azote, R^{11A} peut être présent ou absent, lorsque E^{12A} est un atome d'azote, R^{12A} peut être présent ou absent, et lorsque E^{13A} est un atome d'azote, R^{13A} peut être présent ou absent,
A¹-G¹-A² représente un ligand bidenté anionique, A¹ et A² représentent chacun indépendamment un carbone un atome, un atome d'oxygène ou un atome d'azote, et ces atomes peuvent être un atome constitutif du cycle, G¹ représente une liaison simple ou un groupe atomique constituant un ligand bidenté avec A¹ et A², et lorsqu'une pluralité de A¹-G¹-A² sont présents, ils peuvent être identiques ou différents.

2. Composition selon la revendication 1, dans laquelle au moins l'un dudit cycle R^{1C}, dudit cycle R^{2C}, dudit cycle R^{3C} et dudit cycle R^{4C} a un groupe représenté par la formule (D-1), 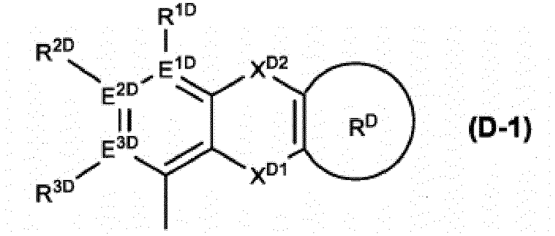 dans laquelle,
le cycle R^{D} représente un cycle hydrocarboné aromatique ou un cycle hétéro aromatique, et les cycles précédents ont éventuellement un substituant, et lorsqu'une pluralité de substituants sont présents, ils peuvent être combinés ensemble pour former un cycle avec les atomes auxquels ils sont attachés,
X^{D1} et X^{D2} représentent chacun indépendamment une liaison simple, un atome d'oxygène, un atome de soufre, un groupe représenté par -N(R^{XD1})- ou un groupe représenté par -C(R^{XD2})₂-, R^{XD1} et R^{XD2} représentent chacun indépendamment un atome d'hydrogène, un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant, et une pluralité de R^{XD2} peuvent être identiques ou différents et peuvent être combinés ensemble pour former un cycle avec l'atome de carbone auquel ils sont attachés,
E^{1D}, E^{2D} et E^{3D} représentent chacun indépendamment un atome d'azote ou un atome de carbone,
R^{1D}, R^{2D} et R^{3D} représentent chacun indépendamment un atome d'hydrogène, un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant,
lorsque E^{1D} est un atome d'azote, R^{1D} est absent, lorsque E^{2D} est un atome d'azote, R^{2D} est absent, et lorsque E^{3D} est un atome d'azote, R^{3D} est absent,
R^{1D} et R^{2D} peuvent être combinés ensemble pour former un cycle avec les atomes de carbone auxquels ils sont attachés, R^{2D} et R^{3D} peuvent être combinés ensemble pour former un cycle avec les atomes de carbone auxquels ils sont attachés, R^{1D} et R^{XD1} peuvent être combinés ensemble pour former un cycle avec les atomes auxquels ils sont attachés, R^{1D} et R^{XD2} peuvent être combinés ensemble pour former un cycle avec les atomes de carbone auxquels ils sont attachés, le substituant que le cycle R^{D} a éventuellement et R^{XD1} peuvent être combiné ensemble pour former un cycle avec les atomes auxquels ils sont attachés, et le substituant que le cycle ^{RD} a éventuellement et R^{XD2} peuvent être combinés ensemble pour former un cycle avec les atomes de carbone auxquels ils sont attachés.

3. Composition selon la revendication 2, dans laquelle le groupe représenté par ladite formule (D-1) est un groupe représenté par la formule (D-2), 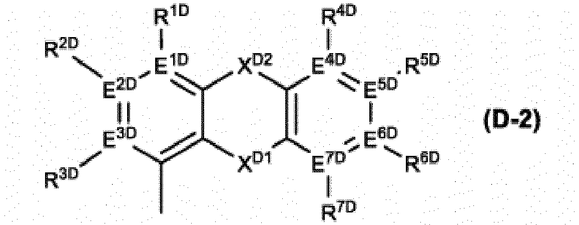 dans laquelle,
X^{D1}, X^{D2}, E^{1D}, E^{2D}, E^{3D}, R^{1D}, R^{2D} et R^{3D} ont la même signification que celle décrite ci-dessus,
E^{4D}, E^{5D}, E^{6D} et E^{7D} représentent chacun indépendamment un atome d'azote ou un atome de carbone,
R^{4D}, R^{5D}, R^{6D} et R^{7D} représentent chacun indépendamment un atome d'hydrogène, un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant,
lorsque E^{4D} est un atome d'azote, R^{4D} est absent, lorsque E^{5D} est un atome d'azote, R^{5D} est absent, lorsque E^{6D} est un atome d'azote, R^{6D} est absent, et lorsque E^{7D} est un atome d'azote, R^{7D} est absent, et
R^{4D} et R^{5D}, R^{5D} et R^{6D}, R^{6D} et R^{7D}, R^{4D} et R^{XD1}, R^{4D} et R^{XD2}, R^{7D} et R^{XD1}, et R^{7D} et R^{XD2} peuvent chacun être combinés ensemble pour former un cycle avec les atomes auxquels ils sont attachés.

4. Composition selon l'une quelconque des revendications 1 à 3, dans laquelle le composé représenté par ladite formule (C-1) est un composé représenté par la formule (C-2), 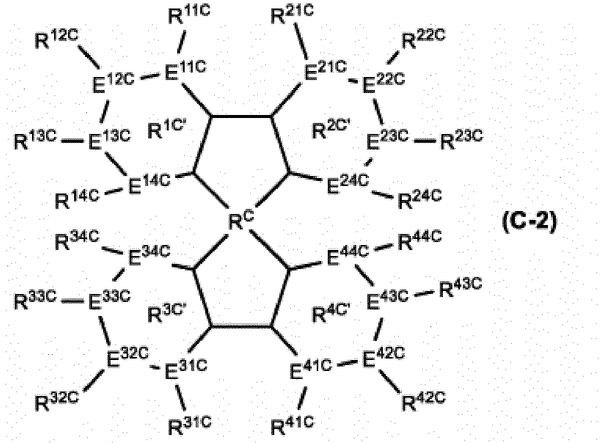 dans laquelle
R^{C} représente la même signification que celle décrite ci-dessus,
E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} et E^{44C} représentent chacun indépendamment un atome d'azote ou un atome de carbone,
le cycle R^{1C'}, le cycle R^{2C'}, le cycle R^{3C'} et le cycle ^{R4C'} représentent chacun indépendamment un cycle benzène, un cycle pyridine ou un cycle diazabenzène,
R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} et R^{44C} représentent chacun indépendamment un atome d'hydrogène, un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant, et dans laquelle au moins l'un parmi R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} et R^{44C} est un groupe aryle ayant éventuellement un substituant ou un groupe hétérocyclique monovalent ayant éventuellement un substituant,
lorsque E^{11C} est un atome d'azote, R^{11C} est absent, lorsque E^{12C} est un atome d'azote, R^{12C} est absent, lorsque E^{13C} est un atome d'azote, R^{13C} est absent, lorsque E^{14C} est un atome d'azote, R^{14C} est absent, lorsque E^{21C} est un atome d'azote, R^{21C} est absent, lorsque E^{22C} est un atome d'azote, R^{22C} est absent, lorsque E^{23C} est un atome d'azote, R^{23C} est absent, lorsque E^{24C} est un atome d'azote, R^{24C} est absent, lorsque E^{31C} est un atome d'azote, R^{31C} est absent, lorsque E^{32C} est un atome d'azote, R^{32C} est absent, lorsque E^{33C} est un atome d'azote, R^{33C} est absent, lorsque E^{34C} est un atome d'azote, R^{34C} est absent, lorsque E^{41C} est un atome d'azote, R^{41C} est absent, lorsque E^{42C} est un atome d'azote, R^{42C} est absent, lorsque E^{43C} est un atome d'azote, R^{43C} est absent, et lorsque E^{44C} est un atome d'azote, R^{44C} est absent, et
R^{11C} et R^{12C}, R^{12C} et R^{13C}, R^{13C} et R^{14C}, R^{14C} et R^{34C}, R^{34C} et R^{33C}, R^{33C} et R^{32C}, R^{32C} et R^{31C}, R^{31C} et R^{41C}, R^{41C} et R^{42C}, R^{42C} et R^{43C}, R^{43C} et R^{44C}, R^{44C} et R^{24C}, R^{24C} et R^{23C}, R^{23C} et R^{22C}, R^{22C} et R^{21C}, et R^{21C} et R^{11C} peuvent chacun être combinés ensemble pour former un cycle avec les atomes de carbone auxquels ils sont attachés.

5. Composition selon la revendication 4, dans laquelle le composé représenté par ladite formule (C-2) est un composé représenté par la formule (C-3), 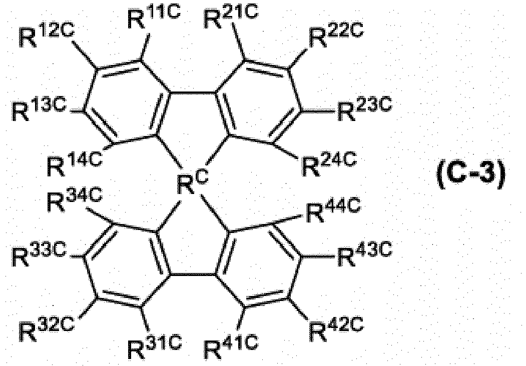 dans laquelle R^{C}, R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} et R^{44C} représentent la même signification que celle décrite ci-dessus.

6. Composition selon la revendication 4 ou 5, dans laquelle au moins l'un parmi ledit R^{11C}, ledit R^{12C}, ledit R^{14C}, ledit R^{21C}, ledit R^{22C}, ledit R^{24C}, ledit R^{31C}, ledit R^{32C}, ledit R^{34C}, ledit R^{41C}, ledit R^{42C} et ledit R^{44C} est un groupe représenté par ladite formule (D-1).

7. Composition selon l'une quelconque des revendications 1 à 6, dans laquelle le composé phosphorescent représenté par ladite formule (1) est un composé phosphorescent représenté par la formule (1-A), 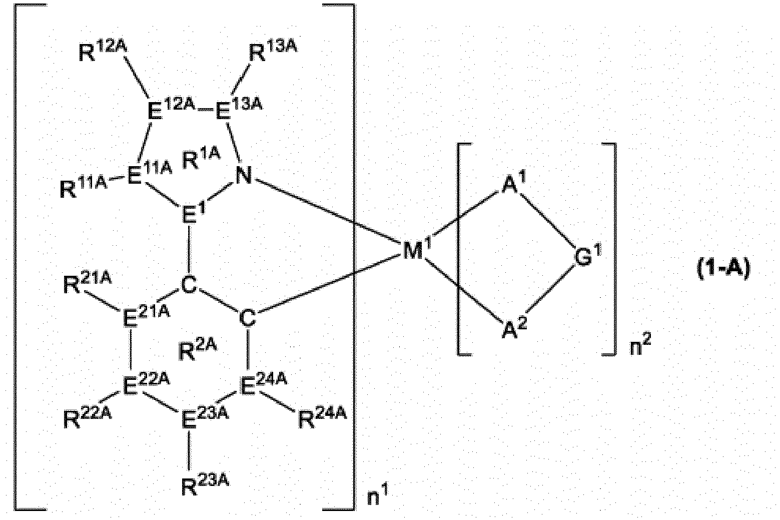 dans laquelle,
M¹, n¹, n², le cycle R^{1A}, E¹, E^{11A}, E^{12A}, E^{13A}, R^{11A}, R^{12A}, R^{13A} et A¹-G¹-A² représentent la même signification que celle décrite ci-dessus,
le cycle R^{2A} représente un cycle benzène, un cycle pyridine ou un cycle diazabenzène,
E^{21A}, E^{22A}, E^{23A} et E^{24A} représentent chacun indépendamment un atome d'azote ou un atome de carbone, et lorsqu'une pluralité de E^{21A}, E^{22A}, E^{23A} et E^{24A} sont présents, ils peuvent être identiques ou différents à chaque occurrence, et lorsque E^{21A} est un atome d'azote, R^{21A} est absent, lorsque E^{22A} est un atome d'azote, R^{22A} est absent, lorsque E^{23A} est un atome d'azote, R^{23A} est absent, lorsque E^{24A} est un atome d'azote, R^{24A} est absent,
R^{21A}, R^{22A}, R^{23A} et R^{24A} représentent chacun indépendamment un atome d'hydrogène, un groupe alkyle, un groupe cycloalkyle, un groupe alcoxy, un groupe cycloalcoxy, un groupe aryle, un groupe aryloxy, un groupe hétérocyclique monovalent, un groupe amino substitué ou un atome d'halogène, et les groupes précédents ont éventuellement un substituant, et lorsqu'une pluralité de R^{21A}, R^{22A}, R^{23A} et R^{24A} sont présents, ils peuvent être identiques ou différents à chaque occurrence, et, R^{21A} et R^{22A}, R^{22A} et R^{23A}, R^{23A} et R^{24A}, et R^{11A} et R^{21A} peuvent chacun être combinés ensemble pour former un cycle avec les atomes auxquels ils sont attachés.

8. Composition selon la revendication 7, dans laquelle le composé phosphorescent représenté par ladite formule (1-A) est un composé phosphorescent représenté par la formule (1-A1), un composé phosphorescent représenté par la formule (1-A2) ou un composé phosphorescent représenté par la formule (1-A3), 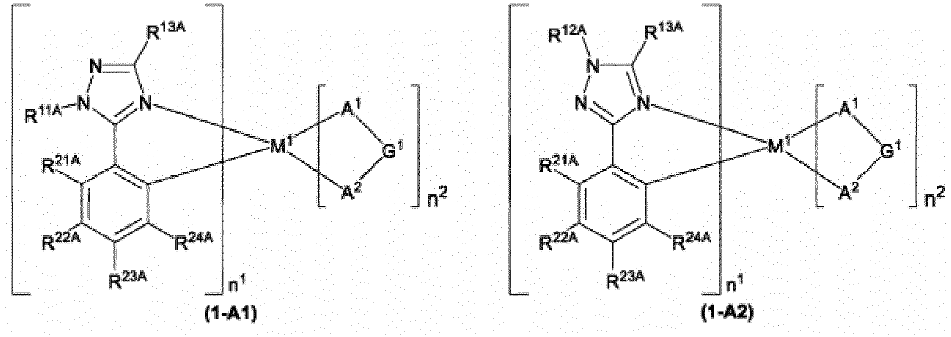 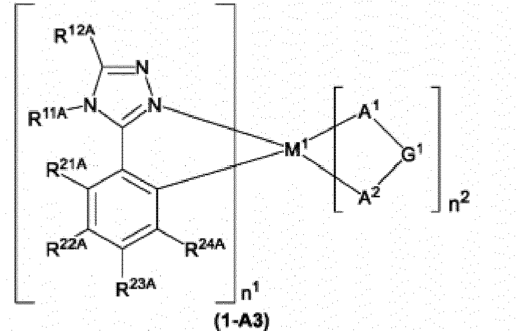 dans laquelle M¹, n¹, n², R^{11A}, R^{12A}, R^{13A}, R^{21A}, R^{22A}, R^{23A}, R^{24A} et A¹-G¹-A² représentent la même signification que celle décrite ci-dessus.

9. Dispositif électroluminescent comprenant la composition selon l'une quelconque des revendications 1 à 8.
